(19) Europäisches Patentamt / European Patent Office / Office européen des brevets

(11) **EP 4 560 302 B1**

(12) **EUROPÄISCHE PATENTSCHRIFT**

(45) Veröffentlichungstag und Bekanntmachung des Hinweises auf die Patenterteilung:
**20.05.2026 Patentblatt 2026/21**

(21) Anmeldenummer: **24212102.8**

(22) Anmeldetag: **11.11.2024**

(51) Internationale Patentklassifikation (IPC):
*G01N 27/16* (2006.01)    *G01N 33/00* (2006.01)

(52) Gemeinsame Patentklassifikation (CPC):
**G01N 33/0006; G01N 27/16**

(54) **GASMESSVORRICHTUNG UND GASMESSVERFAHREN FÜR EIN ZIELGAS MIT VERBESSERTER KOMPENSATION EINER UMGEBUNGSBEDINGUNG**

GAS MEASUREMENT DEVICE AND GAS MEASUREMENT METHOD FOR TARGET GAS WITH IMPROVED COMPENSATION OF AMBIENT CONDITION

DISPOSITIF DE MESURE DE GAZ ET PROCÉDÉ DE MESURE DE GAZ POUR UN GAZ CIBLE AVEC COMPENSATION AMÉLIORÉE D'UNE CONDITION ENVIRONNEMENTALE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **23.11.2023 DE 102023132371**

(43) Veröffentlichungstag der Anmeldung:
**28.05.2025 Patentblatt 2025/22**

(73) Patentinhaber: **Dräger Safety AG & Co. KGaA
23560 Lübeck (DE)**

(72) Erfinder:
- **Osswald, Jürgen
  23558 Lübeck (DE)**
- **Pöthig, Tom
  23558 Lübeck (DE)**
- **Balhorn, Sandra
  23558 Lübeck (DE)**

(56) Entgegenhaltungen:
DE-A1- 102022 102 969     US-A1- 2018 052 124
US-A1- 2018 335 412

**Beschreibung**

Die Erfindung betrifft eine Gasmessvorrichtung und ein Gasmessverfahren, welche die Konzentration eines Zielgases zu messen vermögen und in unterschiedlichen Modi betrieben werden können, wobei in jedem Modus der Einfluss jeweils einer nicht direkt gemessenen Umgebungsbedingung auf eine Detektionsgröße gut kompensiert wird und wobei es vom Modus abhängt, welche Umgebungsbedingung kompensiert wird.

In DE 10 2022 102 969 A1 wird eine Gasmessvorrichtung für brennbare Gase beschrieben. Die Gasmessvorrichtung umfasst einen Detektor, einen Kompensator und eine Auswerteeinheit. Die Gasmessvorrichtung lässt sich in zwei Modi betreiben, nämlich in einem Messmodus und in einem Überwachungsmodus.

In einer bevorzugten Ausgestaltung wendet die Erfindung ein aus dem Stand der Technik bekanntes Prinzip an, das auch unter dem Namen "Wärmetönungs-Sensor" bekannt geworden ist. Das Zielgas ist in dieser Ausgestaltung brennbar, lässt sich also oxidieren. Ein Detektor wird erhitzt und erhitzt eine Gasprobe in einer Messkammer, die Erhitzung der Gasprobe führt zu einer Oxidation von brennbarem Zielgas in der Gasprobe, durch die Oxidation wird Wärmeenergie freigesetzt, die freigesetzte Wärmeenergie erhitzt den Detektor weiter, und eine Detektor-Detektionsgröße, die mit der Temperatur des Detektors korreliert, wird gemessen. Die weitere Erhitzung und damit die Detektor-Detektionsgröße korrelieren mit der gesuchten Konzentration von brennbarem Zielgas.

Die weitere Erhitzung des Detektors hängt nicht nur von der Zielgas-Konzentration, sondern auch von Umgebungsbedingungen ab. Aus dem Stand der Technik ist weiterhin bekannt, einen Kompensator einzusetzen, der weniger oder überhaupt kein Zielgas oxidiert, aber auf Umgebungsbedingungen idealerweise genauso reagiert wie der Detektor. Eine Detektionsgröße des Kompensators wird gemessen und verwendet, um den Einfluss von Umgebungsbedingungen auf die Detektor-Detektionsgröße zu kompensieren.

Die Erfindung lässt sich auch für eine Gasmessvorrichtung anwenden, die ein anderes Prinzip anwendet, um die Konzentration eines Zielgases zu messen, insbesondere ein optisches Messverfahren mit einer Strahlungsquelle und einem Photodetektor (Absorptionsspektroskopie) oder ein akustisches Messverfahren mit einer Strahlungsquelle oder Schallquelle und einem Akustiksensor oder ein elektrochemisches Messverfahren.

Der Erfindung liegt die Aufgabe zugrunde, eine Gasmessvorrichtung und ein Gasmessverfahren bereitzustellen, welche die Konzentration eines Zielgases zu messen vermögen und besser als bekannte Gasmessvorrichtungen und Gasmessverfahren den Einfluss einer Umgebungsbedingung auf die Messergebnisse zu kompensieren vermögen, ohne dass notwendigerweise ein Sensor für diese Umgebungsbedingungen benötigt wird.

Die Aufgabe wird durch eine Gasmessvorrichtung mit den Merkmalen des Anspruchs 1 und durch ein Gasmessverfahren mit den Merkmalen des Anspruchs 12 gelöst. Vorteilhafte Ausgestaltungen der erfindungsgemäßen Gasmessvorrichtung sind, soweit sinnvoll, auch vorteilhafte Ausgestaltungen des erfindungsgemäßen Gasmessverfahrens und umgekehrt.

Die erfindungsgemäße Gasmessvorrichtung vermag die Konzentration mindestens eines Zielgases in einem räumlichen Bereich zu messen. In einer Anwendung ist das Zielgas ein brennbares Zielgas, in einer anderen Anwendung ein sonstiges für Menschen schädliches Zielgas. Das Zielgas kann auch ein für Menschen lebensnotwendiges Gas sein, beispielsweise Sauerstoff, oder Kohlendioxid oder ein Narkosemittel. In der Regel liefert die Gasmessvorrichtung nur mindestens einen Schätzwert (Näherungswert) für die tatsächliche Zielgas-Konzentration, wobei der Schätzwert vom tatsächlichen Wert abweichen kann. Das erfindungsgemäße Gasmessverfahren wird automatisch und unter Verwendung einer erfindungsgemäßen Gasmessvorrichtung durchgeführt.

Eine Gasprobe fließt aus dem zu überwachenden Bereich in das Innere der Gasmessvorrichtung und erreicht dort sowohl einen Detektor als auch einen Kompensator der Gasmessvorrichtung. In einer Realisierungsform saugt eine Fluidfördereinheit der Gasmessvorrichtung die Gasprobe an, in einer anderen Ausgestaltung diffundiert die Gasprobe in das Innere.

Der Detektor weist eine messbare Detektor-Detektionsgröße auf, z.B. die elektrische Spannung oder Stromstärke. Diese Detektor-Detektionsgröße korreliert mit der Konzentration des Zielgases in einer Gasprobe. Ein Detektor-Detektionsgrößen-Sensor der Gasmessvorrichtung vermag die Detektor-Detektionsgröße zu messen.

Der Kompensator weist eine messbare Kompensator-Detektionsgröße auf. Die Kompensator-Detektionsgröße korreliert weniger als die Detektor-Detektionsgröße oder sogar überhaupt nicht mit der gesuchten Zielgas-Konzentration. Ein Kompensator-Detektionsgrößen-Sensor der Gasmessvorrichtung vermag die Kompensator-Detektionsgröße zu messen. Möglich ist, dass ein Detektionsgrößen-Sensor eine Gesamt-Detektionsgröße zu messen vermag, die sowohl von der Detektor-Detektionsgröße als auch von der Kompensator-Detektionsgröße abhängt.

Anmerkung: Die Formulierung, dass ein Sensor eine physikalische Größe misst, bedeutet folgendes: Der Sensor misst direkt die physikalische Größe oder eine andere physikalische Größe, die mit der zu messenden Größe korreliert und daher ein Maß für die zu messende Größe ist. Im vorliegenden Fall ist eine Detektionsgröße beispielsweise die Temperatur eines stromleitenden Bauteils, und der Sensor misst die elektrische Spannung und / oder die elektrische Stromstärke.

Die Gasprobe stammt aus einem zu überwachenden räumlichen Bereich. Umgebungsbedingungen, insbesondere die Umgebungstemperatur, in diesem räumlichen Bereich wirken auf die Gasprobe ein. In der Regel wird die Detektor-Detektionsgröße daher zwangsläufig von mindestens einer Umgebungsbedingung beeinflusst, insbesondere von der Umgebungstemperatur. Die Kompensator-Detektionsgröße wird ebenfalls von dieser Umgebungsbedingung beeinflusst. Die Kompensator-Detektionsgröße korreliert aber weniger als die Detektor-Detektionsgröße mit der Zielgas-Konzentration und hängt in einer Ausgestaltung nicht nennenswert von der Zielgas-Konzentration ab. Idealerweise hängen beide Detektionsgrößen - optional nach einer Korrektur um jeweils einen Nullwert - in gleicher Weise von allen Umgebungsbedingungen ab. In der Praxis ist diese ideale Situation in der Regel nicht zu erreichen.

Eine signalverarbeitende Auswerteeinheit vermag automatisch die Konzentration des Zielgases in der Gasprobe zu ermitteln. Für diese Ermittlung verwendet die Auswerteeinheit die gemessene Detektor-Detektionsgröße, also ein Signal des Detektor-Detektionsgrößen-Sensors, und die gemessene Kompensator-Detektionsgröße, also ein Signal des Kompensator-Detektionsgrößen-Sensors, und - falls vorhanden - ein Signal eines optionalen Sensors für eine Umgebungsbedingung. Möglich ist, dass die Auswerteeinheit das jeweilige Signal von mindestens zwei Umgebungsbedingungen-Sensoren verwendet. Der Schritt, dass die Auswerteeinheit die Zielgas-Konzentration ermittelt, lässt sich auch als ein Vorgang bezeichnen, dass die Auswerteeinheit eine Schätzung für die tatsächliche Zielgas-Konzentration berechnet. Durch diesen Schritt wird also wenigstens näherungsweise die tatsächliche Zielgas-Konzentration gemessen.

Die ermittelte Zielgas-Konzentration hängt also sowohl von der Detektor-Detektionsgröße als auch von der Kompensator-Detektionsgröße ab. Die Kompensator-Detektionsgröße ermöglicht es, bis zu einem gewissen Grad den Einfluss der Umgebungstemperatur und den Einfluss mindestens einer weiteren Umgebungsbedingung auf die Detektor-Detektionsgröße rechnerisch zu kompensieren.

In einer bevorzugten Realisierungsform wird eine Gesamt-Detektionsgröße durch Anwendung eines funktionalen Zusammenhangs berechnet, der bevorzugt ein gewichtetes Mittel der gemessenen Detektor-Detektionsgröße und der gemessenen Kompensator-Detektionsgröße umfasst. Die Zielgas-Konzentration wird abhängig von der Gesamt-Detektionsgröße ermittelt.

In der Regel liefert die erfindungsgemäße Gasmessvorrichtung als Messergebnis nicht die tatsächliche Zielgas-Konzentration, sondern einen Schätzwert für die Zielgas-Konzentration. Im Folgenden wird vom "Messergebnis der Gasmessvorrichtung" oder auch von der "ermittelten Zielgas-Konzentration" gesprochen, wobei die ermittelte Zielgas-Konzentration in der Regel von der tatsächlichen Zielgas-Konzentration abweicht. Die ermittelte Zielgas-Konzentration soll relativ gering von der tatsächlichen Zielgas-Konzentration abweichen. Wie dies erfindungsgemäß erreicht wird, wird nachfolgend beschrieben.

Die Detektor-Detektionsgröße und damit das Signal des Detektor-Detektionsgrößen-Sensors hängen einerseits von der gesuchten Zielgas-Konzentration und andererseits von drei Umgebungsbedingungen ab, nämlich von der Umgebungstemperatur, der Umgebungsfeuchte und dem Umgebungsdruck. Die erfindungsgemäße Gasmessvorrichtung kann einen Sensor für eine Umgebungsbedingung umfassen. In der Regel hängen die Kompensator-Detektionsgröße und damit das Signal des Kompensator-Detektionsgrößen-Sensors ebenfalls von diesen drei Umgebungsbedingungen ab. Die Erfindung zeigt einen Weg auf, wie der jeweilige Einfluss dieser drei Umgebungsbedingungen berücksichtigt wird, ohne dass die Gasmessvorrichtung notwendigerweise für jede Umgebungsbedingung jeweils einen Sensor aufzuweisen braucht.

Die Gasmessvorrichtung lässt sich in mindestens einem von mindestens zwei verschiedenen Modi betreiben. Der eine dieser Modi ist ein druckkompensierender Modus, der oder ein anderer dieser Modi ein feuchtekompensierender Modus. Während eines Einsatzes wird die Gasmessvorrichtung in mindestens einem Modus betrieben und zu jedem Zeitpunkt in genau einem Modus. Möglich ist, dass sie während eines Einsatzes oder bei zwei aufeinanderfolgenden Einsätzen in zwei unterschiedlichen Modi betrieben wird.

Im druckkompensierenden Modus ist der Einfluss des Umgebungsdrucks auf ein Ermittlungs-Ergebnis der Auswerteeinheit wie folgt kompensiert:

- Die Randbedingung wird eingehalten, dass der Einfluss der Umgebungsfeuchte auf das Ermittlungs-Ergebnis unterhalb einer vorgegebenen oberen Feuchteeinfluss-Schranke bleibt. Mit anderen Worten: Bei jedem Wert für die Umgebungsfeuchte, der beim Einsatz der Gasmessvorrichtung auftreten kann, weicht die ermittelte von der tatsächlichen Zielgas-Konzentration um höchstens die obere Feuchteeinfluss-Schranke ab. Diese obere Schranke wird beispielsweise in % UEG (untere Explosionsgrenze) angegeben.
- Der Einfluss des Umgebungsdrucks auf das Ermittlungs-Ergebnis wird kompensiert, und zwar unter Einhaltung der gerade genannten Randbedingung.

Entsprechend wird im feuchtekompensierenden Modus der Einfluss der Umgebungsfeuchte auf das Ermittlungs-Ergebnis wie folgt kompensiert:

- Die Randbedingung wird eingehalten, dass der Einfluss des Umgebungsdrucks auf das Ermittlungs-Ergebnis unterhalb einer vorgegebenen oberen Druckeinfluss-Schranke bleibt.

- Der Einfluss der Umgebungsfeuchte auf das Ermittlungs-Ergebnis wird unter Einhaltung dieser Randbedingung kompensiert.

Das Merkmal, dass eine Umgebungsbedingung kompensiert wird, kann in der Regel nicht vollständig erzielt werden. Mit der Formulierung, dass eine Umgebungsbedingung kompensiert wird, ist folgendes gemeint: Die Umgebungsbedingung wird wenigstens bis zu einem gewissen Grad kompensiert. Die Formulierung, dass eine Umgebungsbedingung kompensiert wird, bedeutet bevorzugt folgendes: Vor einem produktiven Einsatz wird die Gasmessvorrichtung kalibriert. Für diese Kalibrierung wird eine Stichprobe mit mehreren Stichprobenelementen verwendet. Jedes Stichprobenelement umfasst das Zielgas mit einer bekannten Zielgas-Konzentration. Die Gasmessvorrichtung misst die jeweilige Zielgas-Konzentration jedes Stichprobenelements unter bekannten Umgebungsbedingungen. Während der Kalibrierung wird die Gasmessvorrichtung so kalibriert, dass folgendes Ergebnis erzielt wird: Bei Anwendung auf die Stichprobe ist der Einfluss des Umgebungsdrucks (druckkompensierender Modus) bzw. der Einfluss der Umgebungsfeuchte (feuchtekompensierender Modus) bestmöglich kompensiert. Die Formulierung, das eine Randbedingung eingehalten wird, bedeutet, dass diese Randbedingungen eingehalten wird, wenn die Gasmessvorrichtung auf die Stichprobe angewendet wird.

Bevorzugt wird die Gasmessvorrichtung so kalibriert, dass der Einfluss des Umgebungsdrucks (druckkompensierender Modus) bzw. der Einfluss der Umgebungsfeuchte (feuchtekompensierender Modus) unter Einhaltung der Randbedingung bestmöglich kompensiert wird.

Während eines produktiven Einsatzes vermag die Gasmessvorrichtung nicht notwendigerweise eine Umgebungsbedingung optimal zu kompensieren, insbesondere nicht notwendigerweise optimal bei jeder möglichen Kombination von Umgebungsbedingungen. Im produktiven Einsatz wird auch nicht notwendigerweise eine Randbedingung bei jeder möglichen Kombination von Umgebungsbedingungen eingehalten.

Im druckkompensierenden Modus hängt die ermittelte Zielgas-Konzentration auf eine erste Weise von der Detektor-Detektionsgröße und von der Kompensator-Detektionsgröße ab, im feuchtekompensierenden Modus auf eine zweite Weise. Diese beiden Weisen unterscheiden sich voneinander. Beispielsweise hängt die ermittelte Zielgas-Konzentration von einem gewichteten Mittel der beiden Detektionsgrößen ab, wobei mindestens ein Gewichtsfaktor für den einen Modus anders ist als für den anderen Modus.

Bereits erwähnt wurde eine Ausgestaltung, in der die Auswerteeinheit die Zielgas-Konzentration abhängig von einer Gesamt-Detektionsgröße ermittelt, wobei die Gesamt-Detektionsgröße durch Anwendung eines funktionalen Zusammenhangs auf die beiden einzelnen Detektionsgrößen berechnet wird und wobei der funktionale Zusammenhang ein gewichtetes Mittel der Detektor-Detektionsgröße und der Kompensator-Detektionsgröße umfasst. Im druckkompensierenden Modus hängt dieser funktionale Zusammenhang anders von der Detektor-Detektionsgröße und / oder von der Kompensator-Detektionsgröße ab als im feuchtekompensierenden Modus. Beispielsweise unterscheiden sich der Gewichtsfaktor, mit dem die Detektor-Detektionsgröße in den funktionalen Zusammenhang einfließt, und / oder der Gewichtsfaktor der Kompensator-Detektionsgröße von Modus zu Modus.

Erfindungsgemäß lässt sich die Gasmessvorrichtung in mindestens einem von mindestens zwei verschiedenen Modi betreiben. In einer Ausgestaltung lässt sie sich in mindestens einem von vier verschiedenen Modi betreiben, nämlich zusätzlich in einem druckoptimierten und / oder in einem feuchteoptimierten Modus. Im druckoptimierten Modus wird der Einfluss des Umgebungsdrucks auf das Ermittlungs-Ergebnis der Auswerteeinheit bestmöglich kompensiert, also ohne eine Randbedingung betreffend eine andere Umgebungsbedingung einzuhalten. Entsprechend wird im feuchteoptimierten Modus der Einfluss der Umgebungsfeuchte auf das Ermittlungs-Ergebnis bestmöglich kompensiert, also ohne Einhaltung einer Randbedingung betreffend eine andere Umgebungsbedingung.

Die mindestens zwei unterschiedlichen Modi ermöglichen es, die Gasmessvorrichtung an eine bestimmte Einsatzbedingung sowie an bestimmte Anforderungen an die Genauigkeit des Ermittlungs-Ergebnisses anzupassen. Beispielsweise wird erwartet, dass der Umgebungsdruck bei einem Einsatz stark schwankt, während die Umgebungsfeuchte annähernd gleichbleibt. Diese Einsatzbedingung tritt beispielsweise dann auf, wenn die Zielgas-Konzentration in einem Rohr gemessen wird, wobei ein Gasgemisch durch das Rohr fließt. Der Druck des Gasgemischs im Rohr kann stark schwanken. Daher wird bevorzugt bei dieser Anwendung die Gasmessvorrichtung im druckkompensierenden oder sogar im optionalen druckoptimierten Modus betrieben.

Falls hingegen erwartet wird, dass die Umgebungsfeuchte stark schwankt, während der Umgebungsdruck annähernd gleichbleibt, wird bevorzugt die Gasmessvorrichtung im feuchtekompensierenden oder sogar im optionalen feuchteoptimierten Modus betrieben. Diese Einsatzbedingung tritt beispielsweise dann auf, wenn die Zielgas-Konzentration in einem vollständig oder wenigstens weitgehend umschlossenen Raum, beispielsweise in einer Messkammer oder einem Behälter, gemessen wird.

Die Erfindung erspart in vielen Fällen die Notwendigkeit, einen Kompromiss einzugehen, damit die Gasmessvorrichtung unverändert und ohne Anpassung für jede mögliche Einsatzbedingung verwendet werden kann. Dieser Kompromiss kann dazu führen, dass sie bei manchen Einsatzbedingungen kein ausreichend gutes Messergebnis liefert.

Die Erfindung lässt sich in Kombination mit mindestens einem Sensor für eine Umgebungsbedingung verwenden. Die Erfindung spart aber die Notwendigkeit ein, dass die Gasmessvorrichtung für jede Umgebungsbedingung, die einen Einfluss auf das Ermittlungs-Ergebnis nimmt oder nehmen könnte, jeweils einen ausreichend zuverlässigen Sensor umfasst, also sowohl einen Temperatur-Sensor als auch einen Feuchte-Sensor als auch einen Druck-Sensor. Die Erfindung erspart auch die Notwendigkeit, ein Signal mit einer Information über eine Umgebungsbedingung von einem räumlich entfernten Sensor empfangen und verarbeiten zu müssen. Die Umgebungsbedingungen an der Messposition dieses räumlich entfernten Sensors kann deutlich von der Umgebungsbedingung an der Messposition der erfindungsgemäßen Gasmessvorrichtung differieren.

In einer Ausgestaltung lässt die Gasmessvorrichtung sich wahlweise in jedem der mindestens zwei, optional der vier verschiedenen Modi betreiben. Bevorzugt wird die Einstellung, in welchem Modus die Gasmessvorrichtung betrieben wird, ausschließlich durch Anpassung der Auswerteeinheit vorgenommen, also in der Regel durch Anpassung der Software. Beispielsweise wird mindestens eine Berechnungsvorschrift, die die Auswerteeinheit anwendet, um die geschätzte Zielgas-Konzentrationen durch die Auswertung von Signalen zu ermitteln, entsprechend an den jeweiligen Modus angepasst. Die Hardware kann hingegen in vielen Fällen die gleiche bleiben, und zwar für jeden möglichen Modus, in dem die Gasmessvorrichtung eingesetzt wird oder eingesetzt werden kann. Einerseits erleichtert dieses Merkmal es, die Erfindung auf einer bereits vorhandenen Gasmessvorrichtung zu realisieren. Andererseits ermöglicht dieses Merkmal es, mehrere baugleiche Gasmessvorrichtungen herzustellen und dann jedes dieser baugleichen Geräte für den jeweiligen Modus einzustellen. Dieses Vorgehen ist in vielen Fällen zuverlässiger, insbesondere aufgrund einer möglichen Serienfertigung, als die Verwendung von Gasmessvorrichtungen mit unterschiedlichen Hardware-Bestandteilen für die verschiedenen Modi.

Möglich ist, dass eine erfindungsgemäße Gasmessvorrichtung vorab an einen Modus angepasst wird, also bei einer Kalibrierung und / oder Justierung der Gasmessvorrichtung. Möglich ist, dass die Gasmessvorrichtung sich dann nur in diesem Modus verwenden lässt. In einer alternativen Ausgestaltung umfasst die Gasmessvorrichtung hingegen zusätzlich eine Auswahleinheit. Ein Benutzer oder eine übergeordnete Steuerung wählt mit Hilfe dieser Auswahleinheit einen der mindestens zwei verschiedenen möglichen Modi aus. Die Gasmessvorrichtung wird dann in diesem Modus betrieben. Der Benutzer oder die Steuerung kann später einen mithilfe der Auswahleinheit anderen Modus auswählen. Diese Ausgestaltung ermöglicht es, dieselbe Gasmessvorrichtung nacheinander für unterschiedliche Einsatzbedingungen zu verwenden, ohne einen Bestandteil der Gasmessvorrichtung austauschen zu müssen.

Möglich ist auch, dass die Gasmessvorrichtung sich automatisch von einem Modus in einen anderen Modus umschaltet und nacheinander in mindestens zwei verschiedenen Modi, bevorzugt jedem möglichen Modus, jeweils einen Schätzwert für die Zielgas-Konzentration misst.

Wie bereits erwähnt, hängen die Detektor-Detektionsgröße und die Kompensator-Detektionsgröße nicht nur von der Zielgas-Konzentration ab, sondern zusätzlich von Umgebungsbedingungen, insbesondere von der Umgebungstemperatur, der Umgebungsfeuchte und dem Umgebungsdruck. In einer Ausgestaltung umfasst die Gasmessvorrichtung zusätzlich mindestens einen Sensor für eine Umgebungsbedingung, insbesondere einen Temperatur-Sensor. Der Temperatur-Sensor der Gasmessvorrichtung vermag eine Temperatur in der Umgebung der Gasmessvorrichtung zu messen. Die Auswerteeinheit der Gasmessvorrichtung verwendet zusätzlich jeweils ein Signal von dem oder jedem Sensor für eine Umgebungsbedingung, beispielsweise ein Signal, welches eine Information über die gemessene Umgebungstemperatur umfasst. Möglich ist, dass die Gasmessvorrichtung für mindestens zwei Umgebungsbedingungen jeweils einen Sensor umfasst und die Auswerteeinheit zwei Signale von diesen beiden Sensoren verwendet, um die Zielgas-Konzentration zu ermitteln. Dank der Erfindung ist es aber nicht erforderlich, dass die Gasmessvorrichtung für jede relevante Umgebungsbedingung jeweils ein Sensor umfasst

In einer bevorzugten Ausgestaltung umfasst die Gasmessvorrichtung einen Temperatur-Sensor, aber weder einen Sensor für die Umgebungsfeuchte noch einen Sensor für den Umgebungsdruck. Diese Ausgestaltung ist in vielen Anwendungen insbesondere aus folgendem Grund von Vorteil: In der Regel kommen sowohl ein Sensor für die Umgebungsfeuchte als auch ein Sensor für den Umgebungsdruck in einen chemischen und mechanischen Kontakt mit der Umgebung und altern daher relativ schnell, zumindest dann, wenn keine geeigneten und häufig relativ teuren Gegenmaßnahmen getroffen werden. Ein Temperatur-Sensor hingegen kann von der Umgebung chemisch und mechanisch getrennt werden. In der Regel ist ein thermischer Kontakt ausreichend.

In einer Ausgestaltung lässt sich ein Sensor für eine Umgebungsbedingung wahlweise aktivieren oder deaktivieren, beispielsweise manuell von einem Benutzer oder auch automatisch von einem Steuergerät der Gasmessvorrichtung, wobei dieser Sensor zur Gasmessvorrichtung gehört. Beispielsweise deaktivieren das Steuergerät oder auch ein Benutzer einen Sensor für eine Umgebungsbedingung, falls festgestellt oder entschieden wurde, dass dieser Sensor defekt ist. Dass ein Sensor für eine Umgebungsbedingung defekt ist, lässt sich insbesondere an einem Messwert des Sensors erkennen, der außerhalb eines in der Praxis auftretenden Wertebereichs für die Umgebungsbedingung liegt.

Oder ein Benutzer oder das Steuergerät deaktivieren einen Sensor für eine Umgebungsbedingung, wenn die Gasmessvorrichtung in einer Umgebung eingesetzt werden soll, die für diesen Sensor schädlich ist oder schädlich sein kann oder wenn dieser Sensor viel elektrische Energie verbraucht oder in dieser Umgebung keinen zuverlässigen Messwert für die Umgebungsbedingung zu liefern vermag. Beispielsweise umfasst die Gasmessvorrichtung einen Temperatur-Sensor, der dauerhaft aktiv ist, sowie einen Drucksensor und / oder einen Feuchtesensor, der wahlweise aktiviert oder deaktiviert ist.

In einer Fortbildung dieser Ausgestaltung umfasst die Gasmessvorrichtung jeweils einen Sensor für jede relevante Umgebungsbedingung, also insbesondere jeweils einen Sensor für die Umgebungstemperatur, für die Umgebungsfeuchte und den Umgebungsdruck. Jeder dieser Sensoren lässt sich aktivieren und deaktivieren, und zwar bevorzugt unabhängig von jedem anderen Sensor. Bevorzugt kann ein Benutzer jeden Sensor für eine Umgebungsbedingung wahlweise aktivieren oder deaktivieren. Die Ausgestaltung, dass die Gasmessvorrichtung jeweils einen Sensor für jede relevante Umgebungsbedingung umfasst, erleichtert es, mehrere erfindungsgemäße Gasmessvorrichtungen herzustellen. Diese Gasmessvorrichtungen weisen alle die gleiche Hardware auf und umfassen insbesondere die Sensoren für die Umgebungsbedingungen. Um eine bestimmte Gasmessvorrichtung anzupassen, reicht es aus, einzelne Sensoren zu aktivieren oder zu deaktivieren. Nicht erforderlich ist, Gasmessvorrichtungen mit unterschiedlicher Hardware herzustellen.

In einer Ausgestaltung lässt sich die Gasmessvorrichtung wahlweise in einem Modus-Auswahl-Zustand betreiben, in dem mindestens einer der zwei bis vier erfindungsgemäßen oder optionalen Modi ausgewählt und angewendet werden kann, oder in einem Standard-Zustand, in dem die Auswerteeinheit die Zielgas-Konzentration unabhängig von einem Modus oder in einem Default-Modus ermittelt oder in dem die Gasmessvorrichtung die Zielgas-Konzentration nacheinander in jedem Modus misst, sich also automatisch von einem Modus in einen anderen Modus umschaltet.

Die Ausgestaltung mit diesen beiden Zuständen lässt sich mit der Ausgestaltung kombinieren, bei der mindestens ein Sensor für eine Umgebung wahlweise aktiviert oder deaktiviert ist. Eine mögliche Anwendung dieser Kombination ist die folgende: Die Gasmessvorrichtung umfasst einen Druck-Sensor, der wahlweise aktiviert oder deaktiviert ist. Bei aktiviertem Druck-Sensor verwendet die Auswerteeinheit das Signal des Druck-Sensors, um den Einfluss des Umgebungsdrucks auf die Ermittlung der Zielgas-Konzentration zu berücksichtigen. Bei deaktiviertem Druck-Sensor wird die Gasmessvorrichtung wenigstens zeitweise im druckkompensierenden Modus betrieben. Das Entsprechende gilt für einen Feuchte-Sensor und dem feuchtekompensierenden Modus. Im Standard-Zustand vermag die Gasmessvorrichtung beispielsweise jede für das Messergebnis relevante Umgebungsbedingungen zu messen. Der entsprechende Sensor ist also vorhanden und aktiviert.

In einer Realisierungsform ist die Gasmessvorrichtung als ein sogenannter Wärmetönungs-Sensor ausgestaltet. Der Detektor umfasst ein erhitzbares Detektor-Segment, und die Gasmessvorrichtung vermag das Detektor-Segment zu erhitzen. Insbesondere vermag die Gasmessvorrichtung eine elektrische Spannung an das Detektor-Segment anzulegen, und der dann fließende Strom erhitzt das Detektor-Segment. Das erhitzte Detektor-Segment oxidiert brennbares Zielgas, welches als Bestandteil der Gasprobe den Detektor erreicht hat - natürlich nur dann, wenn diese Gasprobe ausreichend viel brennbares Zielgas umfasst. Die Oxidierung des Zielgases setzt Wärmeenergie frei, und die freigesetzte Wärmeenergie vergrößert die Temperatur des Detektor-Segments. Die Temperatur des Detektor-Segments korreliert also mit der Zielgas-Konzentration.

Der Kompensator umfasst ein erhitzbares Kompensator-Segment. Die Gasmessvorrichtung vermag das Kompensator-Segment zu erhitzen. In einer ersten Alternative vermag das erhitzte Kompensator-Segment pro Zeiteinheit weniger brennbares Zielgas zu oxidieren als das erhitzte Detektor-Segment, idealerweise überhaupt kein brennbares Zielgas. In einer anderen Ausgestaltung ist die Gasmessvorrichtung wie folgt ausgestaltet: Pro Zeiteinheit erreicht eine geringere Menge der Gasprobe den Kompensator als den Detektor. Diese beiden Alternativen können miteinander kombiniert werden.

Erfindungsgemäß umfasst die Gasmessvorrichtung einen Detektor-Detektionsgrößen-Sensor und zusätzlich einen Kompensator-Detektionsgrößen-Sensor. Falls die Erfindung auf einen Wärmetönungs-Sensor angewendet wird, so ist die Gasmessvorrichtung wie folgt ausgestaltet: Der Detektor-Detektionsgrößen-Sensor misst die Temperatur des Detektor-Segments. Der Kompensator-Detektionsgrößen-Sensor misst die Temperatur des Kompensator-Segments. Die Auswerteeinheit ermittelt die Zielgas-Konzentration abhängig von

- einem Signal des Detektor-Detektionsgrößen-Sensors,
- einem Signal des Kompensator-Detektionsgrößen-Sensors,
- optional einem Signal eines Temperatur-Sensors und
- optional einem Signal eines Feuchte-Sensors und / oder einem Signal eines Druck-Sensors.

In einer Realisierungsform wird eine sogenannte Brückenspannung gemessen, die sowohl von der Spannung, die am Detektor anliegt, als auch von der Spannung, die am Kompensator anlegt, abhängt, insbesondere in einer Wheatstone'schen Messbrücke. Die Auswerteeinheit ermittelt die Zielgas-Konzentration abhängig von der Brückenspannung und optional vom Signal des Temperatur-Sensors.

Idealerweise reagiert der Kompensator auf Umgebungsbedingungen genauso wie der Detektor, wird aber weniger oder sogar überhaupt nicht von einem brennbaren Zielgas beeinflusst. Diese ideale Situation lässt sich in der Praxis in der Regel nicht realisieren.

Die Ausgestaltung als Wärmetönungs-Sensor erspart die Notwendigkeit vorzugeben, welche Zielgase in dem zu überwachenden Bereich auftreten können und detektiert werden sollen. Vielmehr vermag ein Wärmetönungs-Sensor in der Regel jedes brennbare Zielgas zu detektieren, vorausgesetzt die Zielgas-Konzentration ist ausreichend groß. In der Regel vermag ein Wärmetönungs-Sensor wenigstens näherungsweise die summierten Konzentrationen aller brennbaren Zielgase zu ermitteln.

Die Gasmessvorrichtung kann auch anders als gerade beschrieben ausgestaltet sein. Beispielsweise umfasst die Gasmessvorrichtung eine Strahlungsquelle oder Schallquelle, welche elektromagnetische Strahlung bzw. Schall zu emittieren vermag, und als Detektor einen Empfänger, der abhängig von der Intensität von auftreffender elektromagnetischer Strahlung oder Schall ein Signal zu erzeugen vermag. Die emittierte Strahlung oder der emittierte Schall durchdringen eine Messkammer, in der sich eine zu untersuchende Gasprobe befindet. Ein zu detektierendes Zielgas absorbiert in einem bestimmten Wellenlängen-Bereich einen Teil der Strahlung oder des Schalls und reduziert daher die Intensität der auftreffenden Strahlung oder beeinflusst die Geschwindigkeit des auftreffenden Schalls. Die gemessene Intensität fungiert als die Detektor-Detektionsgröße. Der Detektor-Detektionsgrößen-Sensor misst die Intensität der auftreffenden Strahlung oder des Schalls. Als Kompensator fungiert beispielsweise ein Referenzempfänger, als Kompensator-Detektionsgröße die Intensität der Strahlung, die auf den Referenzempfänger auftrifft. Beispielsweise mithilfe von geeigneten Wellenlängen-Filtern oder -Spiegeln wird sichergestellt, dass ein zu detektierendes Zielgas zwar die Intensität der Strahlung, die auf den Detektor auftrifft, reduziert, aber nicht die Intensität der Strahlung, die auf den Kompensator auftrifft. Eine derartige Gasmessvorrichtung wird oft als infrarotoptische (photo-elektrische) Gasmessvorrichtung bezeichnet und verbraucht in vielen Fällen weniger elektrische Energie als ein Wärmetönungs-Sensor.

Die Erfindung lässt sich z.B. auch in Kombination mit einem photo-akustischen (infrarot-akustischen) oder elektrochemischen Sensor verwenden.

Nachfolgend wird eine bevorzugte Realisierungsform einer infrarot-optischen Gasmessvorrichtung beschrieben. Die Strahlungsquelle emittiert elektromagnetische Strahlung. Der Detektor ist ein Photodetektor, der abhängig von der Intensität von auftreffender elektromagnetischer Strahlung ein Signal erzeugt und nachfolgend als Zielgas-Photodetektor bezeichnet wird. Das Signal des Zielgas-Photodetektors hängt nicht nur von der Zielgas-Konzentration ab, sondern zusätzlich von mindestens einer Umgebungsbedingung. Beispielsweise absorbieren sowohl das Zielgas als auch Wassertröpfchen und / oder Partikel in der Umgebung und damit in der Gasprobe elektromagnetische Strahlung.

Typischerweise absorbiert das zu detektierende Zielgas elektromagnetische Strahlung in einem bestimmten Wellenlängen-Bereich. Bevorzugt ist zwischen der Strahlungsquelle und dem Photodetektor daher ein Wellenlängen-Filter angeordnet, der ausschließlich oder wenigstens überwiegend nur Strahlung in dem Wellenlängen-Bereich passieren lässt, in dem das Zielgas die elektromagnetische Strahlung abschwächt.

Der Wellenlängen-Bereich des Zielgases kann aber mit einem Wellenlängen-Bereich, in dem Wassertröpfchen und / oder Partikel Strahlung abschwächen, überlappen. Außerdem kann ein Signal des Zielgas-Photodetektors auch von der Umgebungsfeuchte und / oder dem Umgebungsdruck beeinflusst werden. In einer bevorzugten Realisierungsform umfasst die Gasmessvorrichtung daher zusätzlich einen Referenz-Photodetektor, der als der Kompensator-Detektionsgrößen-Sensor fungiert. Ein weiterer Wellenlängen-Filter lässt elektromagnetische Strahlung in dem Wellenlängen-Bereich passieren, in dem Wassertröpfchen und / oder Partikel Strahlung abschwächen. Die Auswerteeinheit ermittelt die Zielgas-Konzentration abhängig von einem Signal des Zielgas-Photodetektors und einem Signal des Referenz-Photodetektors. Möglich ist auch, anstelle zweier Wellenlängen-Filter zwei verschiedene Strahlungsquellen vorzusehen, die elektromagnetische Strahlung in unterschiedlichen Wellenlängen-Bereichen emittieren.

Bereits dargelegt wurde die bevorzugte Ausgestaltung, dass die Gasmessvorrichtung sich ausschließlich durch Anpassung oder Veränderung der Auswerteeinheit an den jeweiligen Modus anpassen lässt. In einer Ausgestaltung wird ein Software-Programm angepasst, welches die Auswerteeinheit anwendet, um die Zielgas-Konzentration zu ermitteln.

In einer Ausgestaltung besitzt die Auswerteeinheit dauerhaft oder wenigstens zeitweise Lesezugriff auf ein rechnerauswertbares Modell. Dieses Modell ist beispielsweise in einem Datenspeicher der Gasmessvorrichtung abgespeichert oder ist ein Bestandteil eines Programms, welches die Auswerteeinheit anwendet und ausführt. Das Modell umfasst für den oder für jeden Modus, in dem die Gasmessvorrichtung sich betreiben lässt, jeweils einen funktionalen Zusammenhang. Der funktionale Zusammenhang für einen Modus beschreibt einen Zusammenhang zwischen

- der Zielgas-Konzentration einerseits und
- jeder Detektionsgröße, also dem jeweiligen Signal jedes Detektionsgrößen-Sensors,

    bevorzugt der Umgebungstemperatur, also dem Signal des Temperatur-Sensors, sowie

    optional der Umgebungsfeuchte und/oder des Umgebungsdrucks, also dem Signal des Feuchte-Sensors und / oder dem Signal des Druck-Sensors

    andererseits.

Beispielsweise treten im funktionalen Modell das Signal des Detektor-Detektionsgrößen-Sensors und das Signal des Kompensator-Detektionsgrößen-Sensors auf, außerdem bevorzugt das Signal des Temperatur-Sensors sowie optional das Signal des Feuchte-Sensors und / oder das Signal des Druck-Sensors.

Zu einem Zeitpunkt wird die Gasmessvorrichtung in einem Modus betrieben. Um in diesem Modus die Zielgas-Konzentration zu ermitteln, wendet die Auswerteeinheit den funktionalen Zusammenhang, der für diesen Modus gültig ist, auf das jeweilige Signal jedes Detektionsgrößen-Sensors und bevorzugt das Signal des Temperatur-Sensors an. Dadurch liefert die Auswerteeinheit als Messergebnis eine ermittelte Zielgas-Konzentration.

Weiterhin betrifft die Erfindung eine Kalibriervorrichtung und ein Kalibrierverfahren, durch welche sich eine Gasmessvorrichtung gemäß der gerade beschriebenen Ausgestaltung, also mit dem funktionalen Modell, kalibrieren lassen.

Die Kalibriervorrichtung vermag eine Vorgabe zu erfassen, die bevorzugt von einem Benutzer stammt. Die erfasste Vorgabe spezifiziert mindestens einen Modus, in dem die zu kalibrierende Gasmessvorrichtung betreibbar sein soll. Möglich ist, dass die Vorgabe mindestens zwei verschiedene Modi spezifiziert.

Die Kalibriervorrichtung vermag automatisch ein rechnerauswertbares Modell zu generieren. Das generierte Modell lässt sich von der Auswerteeinheit der Gasmessvorrichtung anwenden. Für den oder jeden Modus, der in der erfassten Vorgabe spezifiziert ist, umfasst das generierte Modell jeweils einen funktionalen Zusammenhang. Der funktionale Zusammenhang für einen Modus beschreibt in rechnerauswertbare Form einen Zusammenhang zwischen

- der Zielgas-Konzentration einerseits und
- dem jeweiligen Signal jedes Detektionsgrößen-Sensors und bevorzugt dem Signal des Temperatur-Sensors und optional einem Signal eines weiteren Sensors für eine Umgebungsbedingung andererseits.

Um das Modell zu generieren, verwendet die Kalibriervorrichtung eine vorgegebene Stichprobe und wendet eine Menge von vorgegebenen möglichen Zusammenhängen an. In einer Realisierungsform weist jeder vorgegebene mögliche funktionale Zusammenhang jeweils mindestens einen Modell-Parameter auf, und die Kalibriervorrichtung ermittelt jeweils einen Wert für jeden Modell-Parameter. Bei einem Einsatz der Gasmessvorrichtung ist in jeden möglichen funktionalen Zusammenhang für den oder jeden Parameter ein Parameterwert eingesetzt. Dadurch wird aus dem möglichen Zusammenhang ein tatsächlich angewendeter Zusammenhang. Möglich ist auch, dass ein anderes lernendes Verfahren auf die Stichprobe angewendet wird und beispielsweise ein neuronales Netz trainiert wird.

Die verwendete Stichprobe wird vorab empirisch ermittelt und umfasst mehrere Stichprobenelemente. Jedes Stichprobenelement umfasst eine Kennzeichnung einer Umgebungsbedingungen-Zielgas-Kombination und eine Signalwerte-Kombination. Die Umgebungsbedingungen-Zielgas-Kombination ist eine Kombination

- einer Umgebungstemperatur,
- eines Umgebungsdrucks,
- einer Umgebungsfeuchte und
- einer tatsächlichen Zielgas-Konzentration.

Diese vier Größen beeinflussen die oder jede Detektionsgröße, und zwar in der Regel unabhängig voneinander. Die Signalwerte-Kombination enthält für das jeweilige Signal jedes Detektionsgrößen-Sensors und optional für jedes Signal eines Temperatur-Sensors für eine Umgebungsbedingung jeweils einen Wert, der bei der Umgebungsbedingungen-Zielgas-Kombination des Stichprobenelements gemessen ist. Die tatsächliche Zielgas-Konzentration des Stichprobenelements wird vorgegeben oder mit einem anderen Gerät gemessen und führt zu den Signalwerten der Detektionsgrößen-Sensoren.

Die Kalibriervorrichtung ist dazu ausgestaltet, für jeden spezifizierten Modus und für jedes Stichprobenelement jeweils die folgenden Schritte durchzuführen:

- Jeder mögliche funktionale Zusammenhang wird auf die Signalwerte-Kombination des Stichprobenelements angewendet. Dadurch wird ein resultierender Wert für die Zielgas-Konzentration berechnet.
- Der berechnete Wert für die Zielgas-Konzentration wird mit dem tatsächlichen Wert der Zielgas-Konzentration in diesem Stichprobenelement verglichen.

Weiterhin ist die Kalibriervorrichtung dazu ausgestaltet, für jeden spezifizierten Modus jeweils die folgenden Schritte durchzuführen:

- Einer der vorgegebenen möglichen funktionalen Zusammenhänge wird als derjenige funktionale Zusammenhang ausgewählt, der tatsächlich für diesen Modus verwendet wird. Um einen möglichen funktionalen Zusammenhang auszuwählen, werden die Vergleichsergebnisse verwendet, also die Ergebnisse des Vergleichs zwischen der berechneten und der tatsächlichen Zielgas-Konzentration in den Stichprobenelementen. Beispielsweise wird derjenige funktionale Zusammenhang verwendet, bei dem die berechneten Werte für die Zielgas-Konzentration am wenigsten von den entsprechenden tatsächlichen Werten abweichen.

- Bewirkt wird, dass der ausgewählte funktionale Zusammenhang von der Auswerteeinheit als derjenige funktionale Zusammenhang verwendet wird, der tatsächlich in diesem Modus angewendet wird.

Diese Ausgestaltung ermöglicht es, die Kalibrierung empirisch durchzuführen. Nicht erforderlich ist es, ein vollständiges analytisches Modell, welches die Auswerteeinheit anwendet, vorzugeben.

Eine weitere Fortbildung der Ausgestaltung mit dem funktionalen Modell betrifft eine Anordnung mit einer ersten und einer zweiten erfindungsgemäßen Gasmessvorrichtung. Die erste Gasmessvorrichtung lässt sich im druckkompensierenden Modus betreiben, die zweite Gasmessvorrichtung im feuchtekompensierenden Modus. Die Auswerteeinheit der ersten Gasmessvorrichtung hat Lesezugriff auf ein erstes rechnerauswertbares Modell, die Auswerteeinheit der zweiten Gasmessvorrichtung Lesezugriff auf ein zweites rechnerauswertbares Modell. Das erste rechnerauswertbare Modell beschreibt eine Abhängigkeit der Zielgas-Konzentration von der oder jede Detektionsgröße und bevorzugt von der Umgebungstemperatur, und die Gasmessvorrichtung erfüllt bei der Verwendung des ersten Modells die Anforderungen an einen Betrieb im druckkompensierenden Modus. Das zweite rechnerauswertbare Modell beschreibt ebenfalls eine Abhängigkeit der Zielgas-Konzentration von der oder jede Detektionsgröße und bevorzugt von der Umgebungstemperatur und erfüllt die Anforderungen an einen Betrieb im feuchtekompensierenden Modus.

Die oder eine erfindungsgemäße Gasmessvorrichtung kann als ein mobiles Gerät ausgestaltet sein, wobei ein Benutzer dieses Gerät mit sich führt, während der Benutzer sich in einem Bereich aufhält. Das Gerät informiert den Benutzer über die Konzentration mindestens eines Zielgases in dem räumlichen Bereich. Bevorzugt hat das mobile Gerät eine eigene Spannungsversorgungseinheit und eine eigene Ausgabeeinheit. Die oder eine erfindungsgemäße Gasmessvorrichtung kann auch als ein stationäres Gerät ausgestaltet sein, welches an einen bestimmten Ort im räumlichen Bereich installiert ist und bevorzugt Nachrichten mit gemessenen Zielgas-Konzentrationen an einen räumlich entfernten Empfänger übermittelt. Der räumlich entfernte Empfänger gibt Nachrichten in mindestens einer von einem Menschen wahrnehmbaren Form aus.

Im Folgenden wird die Erfindung anhand eines Ausführungsbeispiels beschrieben. Hierbei zeigt

Figur 1     schematisch eine beispielhafte Ausgestaltung der Gasmessvorrichtung;

Figur 2     eine beispielhafte Ausgestaltung des Detektors als Pellistor;

Figur 3     eine beispielhafte abweichende Ausgestaltung des Detektors als flaches Bauteil;

Figur 4     den Detektor von Figur 3 in einer Draufsicht;

Figur 5     eine Randbedingung für die Abhängigkeit von der Umgebungstemperatur;

Figur 6     eine Randbedingung für die Abhängigkeit vom Umgebungsdruck;

Figur 7     eine Randbedingung für die Abhängigkeit von der Umgebungsfeuchte;

Figur 8     beispielhaft zwei Messkurven in Abhängigkeit von einem Verstärkungsfaktor für den Kompensator;

Figur 9     eine Kalibriervorrichtung, welche zwei Gasmessvorrichtungen an zwei verschiedene Modi anpasst.

Die erfindungsgemäße Gasmessvorrichtung und das erfindungsgemäße Gasmessverfahren vermögen im Ausführungsbeispiel einen räumlichen Bereich auf das Vorhandensein mindestens eines brennbaren Zielgases zu überwachen und / oder die Konzentration eines brennbaren Zielgases in diesem Bereich wenigstens näherungsweise zu ermitteln. In einer Anwendung vermag die Gasmessvorrichtung bei Vorhandensein von mehreren brennbaren Zielgasen eine Summe der Zielgas-Konzentrationen zu messen. Die Gasmessvorrichtung nutzt ein aus dem Stand der Technik bekanntes Verfahren, um ein Gasgemisch in dem räumlichen Bereich zu analysieren.

Im Ausführungsbeispiel ist die Gasmessvorrichtung als ein stationäres Gerät ausgestaltet, das während eines Einsatzes an einer bestimmten Stelle in dem zu überwachenden Bereich angeordnet ist. Möglich ist, dass in diesem Bereich mehrere stationäre Gasmessvorrichtungen angeordnet sind. Die oder jede Gasmessvorrichtung steht jeweils wenigstens zeitweise in jeweils einer Datenverbindung mit einem räumlich entfernten Empfänger und übermittelt jeweils mindestens ein Signal an diesen Empfänger. Das übermittelte Signal umfasst eine Information über die gemessene Zielgas-Konzentration. Bevorzugt ist die Datenverbindung eine drahtlose Datenverbindung, wird also mit Funkwellen realisiert. Möglich ist auch eine kabelgebundene Datenverbindung.

Im Inneren eines Gehäuses der Gasmessvorrichtung befindet sich ein Detektor. Durch eine Öffnung des Gehäuses diffundiert eine Gasprobe aus dem zu überwachenden Bereich in das Innere des Gehäuses oder wird in das Innere gefördert, z.B. von einer Pumpe oder einer sonstigen Fluidführungseinheit angesaugt.

Im Ausführungsbeispiel ist die Gasmessvorrichtung als ein Wärmetönungs-Sensor ausgestaltet. Dessen Prinzip wurde eingangs beschrieben. Die Erfindung lässt sich auch für eine Gasmessvorrichtung anwenden, die einen infrarot-optischen oder photo-akustischen oder elektrochemischen Sensor umfasst.

Der Detektor des Wärmetönungs-Sensors umfasst einen elektrisch leitenden Draht mit einem heizenden Segment. Das heizende Detektor-Segment ist beispielsweise eine Spule, die ein Segment des Drahts bildet. Das elektrisch leitende Material ist beispielsweise Platin oder Rhodium oder Wolfram oder eine Legierung unter Verwendung von mindestens einem dieser Metalle. An diesen Draht wird eine elektrische Spannung U angelegt, sodass elektrischer Strom durch den Draht fließt. Der fließende Strom erhitzt das heizende Detektor-Segment, und das erhitzte heizende Detektor-Segment gibt Wärmeenergie ab.

Die abgegebene Wärmeenergie bewirkt, dass im Inneren des Gehäuses mindestens ein brennbares Zielgas oxidiert wird - natürlich nur dann, wenn der räumliche Bereich und damit die Gasprobe im Inneren brennbares Zielgas in ausreichender Menge enthalten.

In einer Anwendung ist Methan ($CH_4$) ein zu detektierendes brennbares Zielgas. Durch eine ausreichende Zufuhr von Wärmeenergie reagiert Methan mit Sauerstoff, und es entstehen Wasser und Kohlendioxid. Aus $CH_4$ und $2\,O_2$ wird also $2\,H_2O$ und $CO_2$.

Bei der Oxidation des Zielgases wird im Inneren des Gehäuses Wärmeenergie freigesetzt. Diese Wärmeenergie wirkt auf den Detektor ein und vergrößert die Temperatur des von Strom durchflossenen und erhitzten Drahts. Diese Temperatur-Erhöhung korreliert mit der freigesetzten Wärmeenergie und damit mit der Konzentration des Zielgases im Inneren des Gehäuses.

Die Temperatur-Änderung verändert eine messbare Eigenschaft des Detektors, welche mit der Detektor-Temperatur korreliert, beispielsweise den elektrischen Widerstand R des vom Strom durchflossenen Drahts des Detektors. Bei vielen elektrisch leitenden Materialien ist der elektrische Widerstand bekanntlich umso höher, je höher die Temperatur des leitenden Materials ist. Die Gasmessvorrichtung misst mindestens eine messbare Größe, welche von der Eigenschaft und damit von der Detektor-Temperatur beeinflusst wird und welche im Folgenden als "Detektionsgröße" bezeichnet wird. Die Detektionsgröße ist beispielsweise direkt die Temperatur oder eine Größe, die mit dem elektrischen Widerstand R des Drahts korreliert, beispielsweise die am Detektor anliegende elektrische Spannung U oder die Stromstärke I oder die vom Detektor-Draht aufgenommene elektrische Leistung P. Falls eine weitere messbare Größe, die ebenfalls von dem elektrischen Widerstand R abhängt, durch eine Regelung konstant gehalten wird, so korreliert die gemessene Detektionsgröße U oder I oder P mit der gesuchten Konzentration des Zielgases. Falls beispielsweise die Stromstärke I des durch den Detektor fließenden Stroms konstant gehalten wird, so korreliert die am Detektor anliegende elektrische Spannung U mit dem elektrischen Widerstand R des Drahts, der Widerstand R korreliert mit der Temperatur des Drahts, die Temperatur des Drahts korreliert mit der Zielgas-Konzentration, und damit korreliert die gemessene elektrische Spannung U mit der gesuchten Zielgas-Konzentration - bei Vorhandensein von mehreren Zielgasen mit der Kombination (Summe) der Zielgas-Konzentrationen.

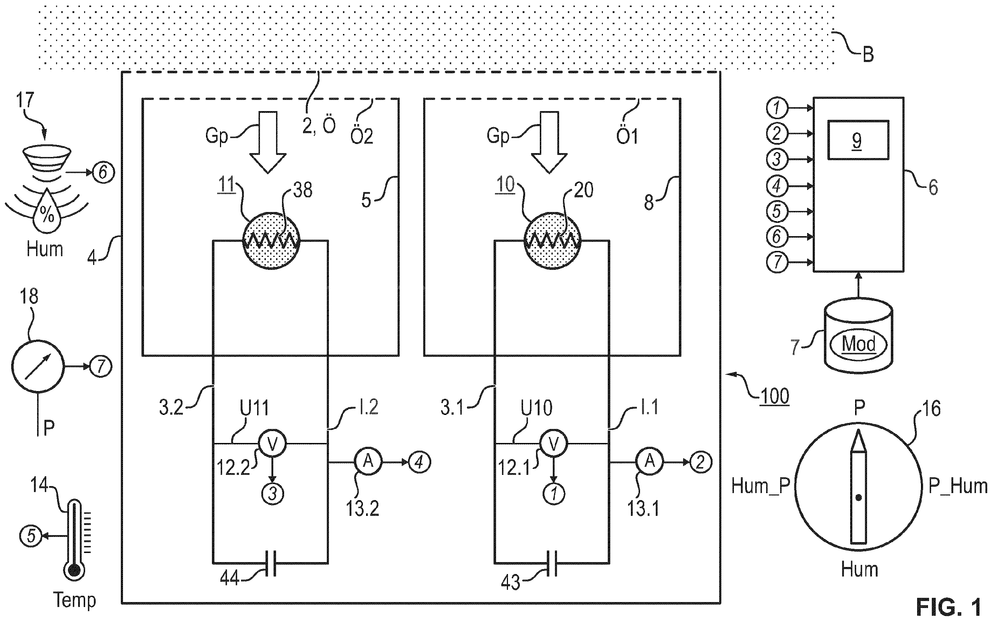

Figur 1 zeigt eine beispielhafte Ausgestaltung einer erfindungsgemäßen Gasmessvorrichtung 100, die einen räumlichen Bereich B auf das Vorhandensein mindestens eines brennbaren Zielgases zu überwachen vermag. In dieser Ausgestaltung ist ein Detektor 10 in einer Detektorkammer 8 angeordnet. Ein weiter unten beschriebener Kompensator 11 ist in einer Kompensatorkammer 5 angeordnet. Die beiden Kammern 8, 5 sind in einem Gehäuse 4 angeordnet. Die Detektorkammer 8 und damit der Detektor 10 stehen über eine Öffnung Ö1 in einer Fluidverbindung mit dem zu überwachenden Bereich B. Die Kompensatorkammer 5 und damit der Kompensator 11 stehen über eine Öffnung Ö2 in einer Fluidverbindung mit dem Bereich B. Dank der Öffnungen Ö1, Ö2 kann eine Gasprobe Gp aus dem Bereich B in das Innere des Gehäuses 4 und dort zu den beiden Kammern 8, 5 gelangen.

Ein optionaler Flammschutz 2, beispielsweise ein metallisches Gitter, vor den Öffnungen Ö1, Ö2 reduziert das Risiko, dass Flammen aus einer Kammer 8, 5 nach außen schlagen. Optional trennt eine nicht gezeigte thermische Barriere im Inneren der Gasmessvorrichtung 100 den Detektor 10 thermisch vom Kompensator 11.

Die am Detektor 10 anliegende elektrische Spannung U10 bewirkt, dass ein elektrischer Strom I fließt. Der fließende Strom I erhitzt das heizende Detektor-Segment 20 auf eine Arbeitstemperatur, die oft zwischen 400 °C und 500 °C liegt. Diese Arbeitstemperatur allein reicht aber in der Regel nicht aus, um ein brennbares Zielgas in der Detektorkammer 8 zu oxidieren. Eine höhere Arbeitstemperatur ist oft unerwünscht, weil diese zu einem unkontrollierten Verbrennen oder einer Zersetzung oder gar zu einem Explodieren von brennbarem Zielgas führen könnte, was oft unerwünscht ist, und außerdem mehr elektrische Energie verbraucht.

Um trotz einer Arbeitstemperatur unterhalb von 500 °C ein brennbares Zielgas oxidieren zu können, umfasst der Detektor 10 ein katalytisches Material, welches in Verbindung mit dem erhitzten heizenden Detektor-Segment 20 das Zielgas oxidiert. Daher wird eine Gasmessvorrichtung mit einem solchen Detektor 10 auch als "katalytischer Sensor" bezeichnet.

In einer häufig verwendeten Realisierung ist das heizende Detektor-Segment 20 von einer elektrischen Isolierung umgeben, beispielsweise von einer Keramikummantelung. Diese elektrische Isolierung isoliert elektrisch das heizende Detektor-Segment 20 und verhindert insbesondere einen unerwünschten Kurzschluss. Die elektrische Isolierung ist thermisch leitend, damit das heizende Detektor-Segment 20 Wärmeenergie in die Umgebung des Detektors 10 abgeben kann und umgekehrt Wärmeenergie im Inneren der Detektorkammer 8 das heizende Detektor-Segment 20 weiter aufheizen kann. Auf diese elektrische Isolierung ist eine Beschichtung aus einem katalytischen Material aufgetragen. Oder ein katalytisches Material ist in die elektrische Isolierung eingebettet. Diese katalytische Beschichtung kommt in Kontakt mit dem Gasgemisch in der Detektorkammer 8 und damit auch mit einem brennbaren Zielgas. Ein derartig aufgebauter Detektor 10 wird oft als "Pellistor" bezeichnet.

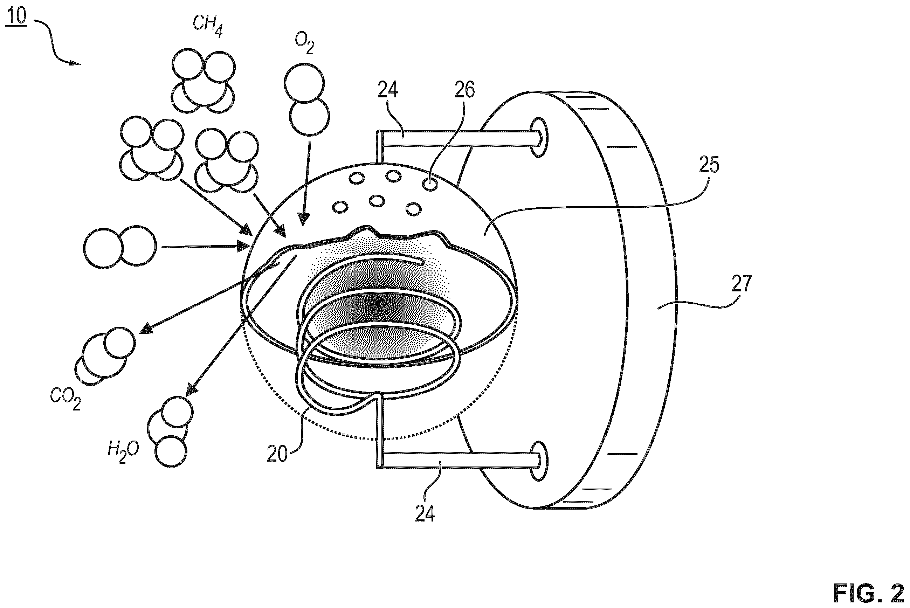

Figur 2 zeigt beispielhaft einen als Pellistor ausgestalteten Detektor 10 sowie schematisch die Umwandlung von Methan ($CH_4$) in $CO_2$ und $H_2O$. Der Detektor 10 umfasst

- einen spiralförmig gewickelten und elektrisch leitenden Draht 20, der als ein heizendes Detektor-Segment fungiert und beispielsweise aus Platin hergestellt ist,
- eine Keramikummantelung 25, welche das heizende Detektor-Segment 20 umgibt und im gezeigten Beispiel die Form einer Vollkugel hat,
- eine katalytische Beschichtung auf der äußeren Oberfläche der Keramikummantelung 25, welche in Figur 2 durch Kreise 26 angedeutet ist,
- eine Montageplatte 27 und
- elektrische Kontaktierungen und mechanische Halterungen 24 für den Draht 20.

Als katalytisches Material wird beispielsweise Platin oder Palladium oder Rhodium oder eine Legierung mit mindestens einem dieser Materialien verwendet. Alternativ oder zusätzlich zu der katalytischen Beschichtung kann auch katalytisches Material 26 in die Keramikummantelung 25 eingebettet sein.

In einer bevorzugten Ausgestaltung hat die Vollkugel des Detektors 10 eine poröse Oberfläche mit einer katalytischen Beschichtung 26. In einer Ausgestaltung wird diese poröse Oberfläche wie folgt hergestellt: Der Detektor 10 mit der porösen Oberfläche, aber ohne die katalytische Beschichtung wird bereitgestellt. Die katalytische Beschichtung 26 wird auf die poröse Oberfläche aufgetragen, und ein Teil des katalytischen Materials dringt in das Innere des Detektors 10 ein. Dank dieser porösen Oberfläche hat der Detektor 10 eine größere Oberfläche verglichen mit einer glatten Oberfläche. Dank dieser größeren Oberfläche vermag der Detektor 10 besser brennbares Zielgas zu oxidieren, insbesondere weil eine größere Menge von Zielgas in Kontakt mit dem katalytischen Material kommt. Ein Gas kann dank der porösen Oberfläche in tiefere Schichten des Detektors 10 gelangen.

Der Kompensator 11 ist in einer Realisierungsform genauso aufgebaut wie der Detektor 10 und umfasst ebenfalls ein heizendes Segment, welches mit dem Bezugszeichen 38 bezeichnet ist. Jedoch kann in einer Realisierungsform pro Zeiteinheit eine geringere Menge von Gas den Kompensator 11 als den Detektor 10 erreichen. In einer anderen Realisierungsform umfasst der Kompensator 11 keine katalytische Beschichtung 26 oder eine katalytische Beschichtung 26, die pro Zeiteinheit weniger Zielgas zu oxidieren vermag als der Detektor 10.

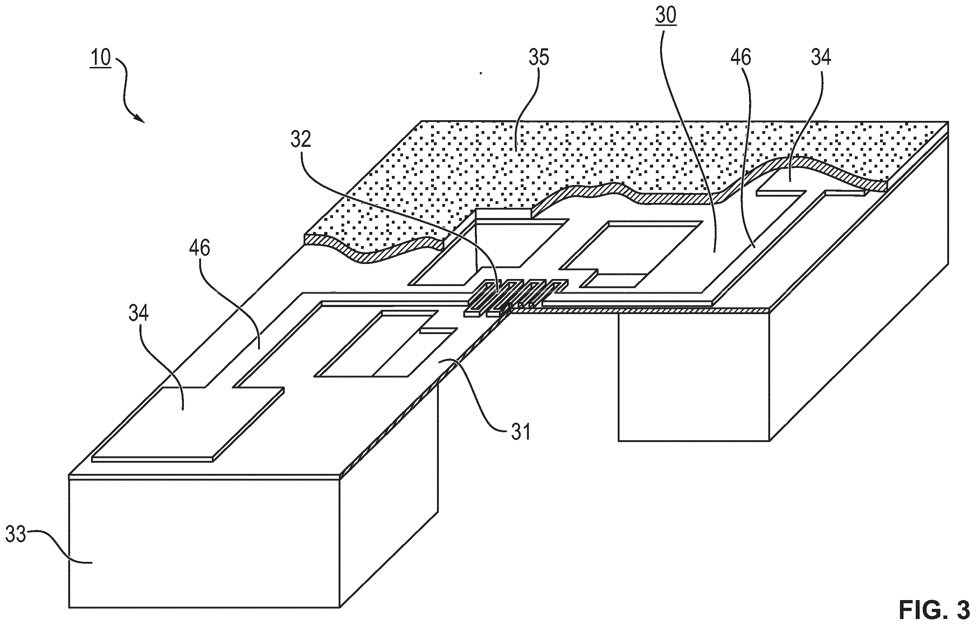

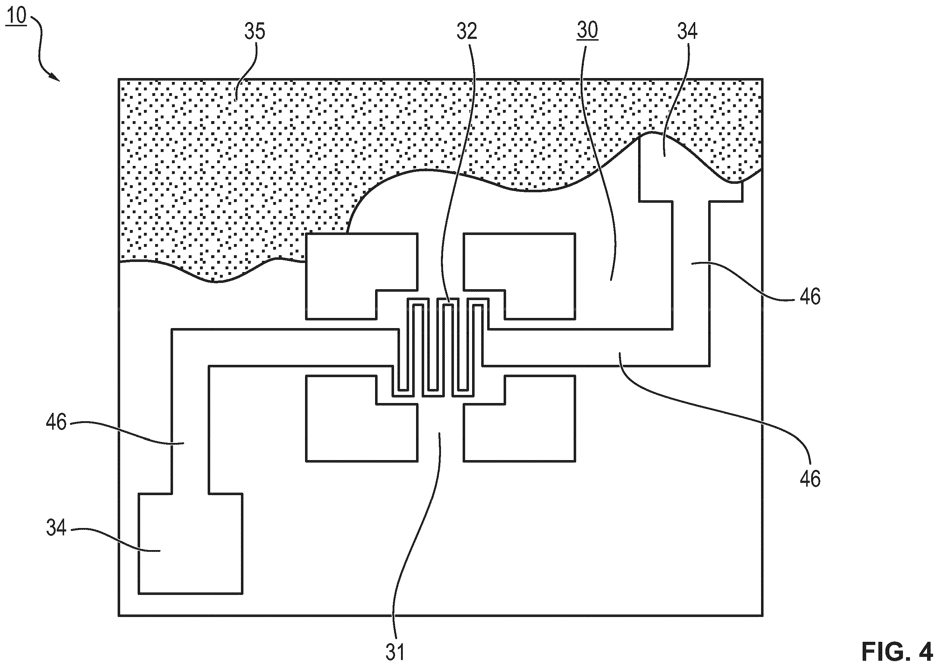

Figur 3 und Figur 4 zeigen eine abweichende Ausgestaltung des Detektors 10 in einer perspektivischen Darstellung (Figur 3) bzw. in einer Draufsicht (Figur 4). Der Detektor 10 umfasst folgende Bestandteile:

- ein elektrisch leitendes Bauteil 30 mit einem heizenden Segment 32 und einer elektrischen Kontaktierung 46, wobei das Bauteil 30 die Form einer Leiterbahn aufweist,
- eine Schutzschicht 35,
- eine Trägerplatte 31, die sich in einer Ebene erstreckt, wobei diese Ebene schräg auf der Zeichenebene von Figur 3 steht und in der Zeichenebene von Figur 4 liegt,
- ein Wafer-Substrat 33, welches die Trägerplatte 31 trägt, und
- elektrische Kontaktstellen 34 für das elektrisch leitende Bauteil 30.

Die Schutzschicht 35 überdeckt wenigstens die Leiterbahn 30, bevorzugt die gesamte Trägerplatte 31, und verhindert, dass die Leiterbahn 30 in direktem Kontakt mit einem Gasgemisch kommt. In einer Realisierungsform ist die Schutzschicht 35 aus Siliziumnitrid hergestellt. Auf die Schutzschicht 35 ist ein katalytisch wirksames Material aufgebracht, und zwar wenigstens in einem Bereich oberhalb des heizenden Segments 32.

Der Kompensator 11 kann wiederum genauso aufgebaut sein wie der Detektor 10 von Figur 3 oder von Figur 4 oder aber weniger oder sogar gar kein katalytisch wirksames Material aufweisen.

Die Temperatur des Detektors 10 und damit auch die oder jede Detektionsgröße wird aber nicht nur von der freigesetzten Wärmeenergie beeinflusst, sondern auch von Umgebungsbedingungen in dem zu überwachenden Bereich B. Die drei wesentlichen Umgebungsbedingungen sind die Umgebungstemperatur Temp, der Umgebungsdruck P und die Umgebungsfeuchte Hum. Insbesondere hängen sowohl der Nullpunkt des Detektors 10 als auch die Erhöhung der Detektor-Temperatur nicht nur von der Zielgas-Konzentration, sondern auch von der Umgebungstemperatur Temp ab. Der Nullpunkt ist derjenige Wert, den die Detektor-Detektionsgröße annimmt, wenn kein Zielgas vorhanden ist. Diese drei Umgebungsbedingungen Temp, P, Hum können auch die Bedingungen im Inneren des Gehäuses 4 und damit auch in der Detektorkammer 8 verändern. Diese Umgebungsbedingungen können nämlich ebenfalls die Detektor-Temperatur und damit eine Detektionsgröße U10 beeinflussen, beispielsweise weil die Wärmeleitfähigkeit in der Umgebung des Detektors 10 verändert wird.

Gewünscht wird, dass die Gasmessvorrichtung 100 trotz variierender Umgebungsbedingungen einerseits zuverlässig ein brennbares Zielgas zu detektieren vermag und andererseits nur wenige Fehlalarme generiert, also nur selten entscheidet, dass ein Zielgas vorhanden ist, obwohl in Wirklichkeit kein Zielgas oberhalb einer Nachweisgrenze aufgetreten ist, was ein fehlerhaftes Ergebnis ist.

Anmerkung: Die drei Umgebungsbedingungen Temperatur, Druck, Feuchte werden mit Temp, P, Hum bezeichnet, Werte dieser drei Umgebungsbedingungen mit temp, p, hum.

Die erfindungsgemäße Gasmessvorrichtung 100 vermag bis zu einem gewissen Grad den Einfluss der drei Umgebungsbedingungen Temp, P, Hum auf die Detektionsgröße rechnerisch zu kompensieren. In der nachfolgenden Beschreibung wird die Stromstärke I.1 durch eine Regelung (closed-loop control) konstant gehalten, und als Detektionsgröße fungiert die am Detektor 10 anliegende elektrische Spannung U10. Wie bereits erwähnt, hängt diese Detektionsgröße U10 von der Temperatur des heizenden Detektor-Segments 20 ab. Diese Temperatur wiederum hängt einerseits von der Zielgas-Konzentration und andererseits von den drei gerade genannten Umgebungsbedingungen ab.

Um den Einfluss von Umgebungsbedingungen zu kompensieren, umfasst die Gasmessvorrichtung 100 zusätzlich zum Detektor 10 den bereits erwähnten Kompensator 11 in der Kompensatorkammer 5, vgl. Figur 1. Der Kompensator 11 umfasst ebenfalls einen Draht mit einem heizenden Kompensator-Segment 38. Auch an den Kompensator 11 wird eine elektrische Spannung U11 angelegt, sodass elektrischer Strom I.2 fließt und das heizende Segment 38 des Kompensators 11 ebenfalls erhitzt wird. Der Kompensator 11 ist ebenfalls den variierenden Umgebungsbedingungen ausgesetzt.

In einer Realisierungsform umfasst der Kompensator 11 ebenfalls einen spiralförmig gewickelten und elektrisch leitenden Draht, der als heizendes Kompensator-Segment fungiert und mit dem Bezugszeichen 38 bezeichnet ist. Der Kompensator 11 umfasst ebenfalls eine Keramikummantelung, eine Montageplatte, elektrische Verbindungen und mechanische Halterungen. In einer Ausgestaltung ist die Keramikummantelung des Kompensators 11 im Gegensatz zum Detektor 10 aber nicht mit einer katalytischen Beschichtung versehen.

In einer anderen Realisierungsform ist der Kompensator 11 genauso aufgebaut wie der Detektor 10, umfasst also ebenfalls eine keramische Beschichtung. Diese keramische Beschichtung ist bei der anderen Realisierungsform ebenfalls katalytisch aktiv. Jedoch ist die Gasmessvorrichtung 100 so ausgestaltet, dass in einer Zeiteinheit weniger Gas aus dem zu überwachenden Bereich B zu dem Kompensator 11 als zu dem Detektor 10 gelangen kann.

Denkbar ist auch, dass das heizende Segment 38 des Kompensators 11 auf eine geringere Temperatur erhitzt wird als das heizende Segment 20 des Detektors 10.

Figur 1 zeigt den Kompensator 11 in der Kompensatorkammer 5. Zu sehen ist, dass der Detektor 10 das heizende Detektor-Segment 20 und der Kompensator 11 das heizendes Kompensator-Segment 38 umfasst. Im gezeigten Beispiel ist der Kompensator 11 ebenfalls als kugelförmiger Pellistor ausgestaltet, umfasst aber im Gegensatz zum Detektor 10 keine katalytisch aktive Beschichtung 26.

In der Realisierungsform, die in Figur 1 gezeigt wird, wird der Detektor 10 von einer Spannungsquelle 43 mit elektrischer Energie versorgt, der Kompensator 11 von einer Spannungsquelle 44. Ein Detektor-Stromkreis 3.1 umfasst den Detektor 10 und die Spannungsquelle 43, ein Kompensator-Stromkreis 3.2 den Kompensator 11 und die Spannungsquelle 44. Weil zwei voneinander unabhängige Stromkreise 3.1 und 3.2 realisiert werden, kann die am Kompensator 11 anliegende elektrische Spannung U11 von der am Detektor 10 anliegenden elektrischen Spannung U10 differieren, und die Stärke des im Kompensator-Stromkreises 3.2 fließenden Stroms kann von der Stärke des im Detektor-Stromkreis 3.1 fließenden Stroms abweichen. Die beiden Spannungsquellen 43, 44 werden bevorzugt mit wieder aufladbaren Batterien (Akkumulatoren) realisiert. Möglich ist, dass dieselbe Spannungsversorgungseinheit sowohl als die erste Spannungsquelle 43 als auch die zweite Spannungsquelle 44 fungiert.

Ein Spannungs-Sensor 12.1 misst die elektrische Spannung U10, die am Detektor 10 anliegt. Ein Stromstärken-Sensor 13.1 misst die Stärke I.1 des elektrischen Stroms, der durch den Stromkreis 3.1 für den Detektor 10 fließt. Ein Spannungs-Sensor 12.2 misst die elektrische Spannung U11, die am Kompensator 11 anliegt. Ein Stromstärken-Sensor 13.2 misst die Stärke I.2 des elektrischen Stroms, der durch den Stromkreis 3.2 für den Kompensator 11 fließt.

Idealerweise wirkt ein brennbares Zielgas nur auf den Detektor 10 ein, die Umgebungsbedingungen hingegen gleichartig auf den Detektor 10 und den Kompensator 11. Wenn diese idealen Bedingungen eingehalten sind, ist die Differenz zwischen der Detektor-Detektionsgröße U10 und der Kompensator-Detektionsgröße U11 - optional jeweils um einen Nullwert korrigiert - ein zuverlässiges Maß für die gesuchte Konzentration des Zielgases, und zwar bei jeder möglichen Kombination von Umgebungsbedingungen.

Diese ideale Bedingung ist in der Praxis aber in der Regel nicht erfüllt. Ein Grund ist, dass der Detektor 10 und der Kompensator 11 bereits aufgrund von bauartbedingten Unterschieden und / oder aufgrund von unvermeidlichen Fertigungstoleranzen verschieden auf Umgebungsbedingungen reagieren. Diese Unterschiede sind insbesondere dann relevant, wenn der Kompensator 11 weniger katalytisch wirksames Material als der Detektor 10 oder sogar überhaupt kein katalytisch wirksames Material aufweist. Ein anderer Grund ist, dass häufig die Oxidation von Zielgasen stärker zu Ablagerungen auf der Oberfläche des Detektors 10 führt als auf der Oberfläche des Kompensators 11. Nachfolgend wird eine erfindungsgemäße Ausgestaltung beschrieben, wie die Gasmessvorrichtung 100 trotz dieser unterschiedlich wirkenden Umgebungsbedingungen in vielen Fällen zuverlässig die Zielgas-Konzentration zu messen vermag.

Gemäß einer bevorzugten Ausgestaltung misst die Gasmessvorrichtung 100 die Umgebungstemperatur, und zwar bevorzugt an einer Messposition an einer äußeren Oberfläche der Gasmessvorrichtung 100. Die Erfindung lässt sich auch realisieren, ohne dass die Gasmessvorrichtung 100 einen Temperatur-Sensor umfasst.

Im gezeigten Ausführungsbeispiel vermag ein Temperatur-Sensor 14 der Gasmessvorrichtung 100 die Umgebungstemperatur Temp zu messen. Im Ausführungsbeispiel liefert der Temperatur-Sensor 14 die Temperaturdifferenz $\Delta$Temp zwischen der aktuellen Umgebungstemperatur und einer vorgegebenen Referenz-Umgebungstemperatur von beispielsweise 20 °C. Der Temperatur-Sensor 14 liefert ein analoges oder ein digitales Signal, das eine Information über die Umgebungstemperatur Temp - im Ausführungsbeispiel mit der Temperaturdifferenz $\Delta$Temp - umfasst. Der Einfluss der Umgebungstemperatur Temp auf die Detektionsgröße wird mit Hilfe eines Signals des Temperatur-Sensors 14 bis zu einem gewissen Grad rechnerisch kompensiert.

Die Gasmessvorrichtung 100 von Figur 1 umfasst in einer Realisierungsform einen Sensor 17 für die Umgebungsfeuchte Hum und einen Sensor 18 für den Umgebungsdruck P. Die Sensoren 17, 18 können in einer Anwendung relativ einfach aufgebaut sein, so dass die Umgebungsfeuchte Hum und / oder der Umgebungsdruck P nur mit einem relativ großen Messfehler gemessen werden. In einer anderen Anwendung lässt sich jeder Sensor 17, 18 aktivieren und deaktivieren. Beispielsweise schaltet ein Benutzer einen Sensor 17 oder 18 aus, wenn die Gasmessvorrichtung 100 in einer Umgebung eingesetzt wird oder werden soll, in welcher der aktivierte Sensor 17 oder 18 beschädigt werden kann, beispielsweise aufgrund eines sehr hohen Drucks oder einer sehr hohen Feuchte oder eines bestimmten Gases in der Umgebung. Möglich ist auch, dass ein Sensor 17, 18 defekt ist und die Gasmessvorrichtung 100 trotzdem eingesetzt werden soll.

Wie der Einfluss des Umgebungsdrucks P und der Umgebungsfeuchte Hum trotzdem wenigstens bis zu einem gewissen Grad kompensiert werden, wird weiter unten beschrieben.

Bevorzugt umfasst die Gasmessvorrichtung 100 zwar einen zuverlässigen Sensor 14 für die Umgebungstemperatur Temp, aber nur einen relativ einfachen und / oder deaktivierbaren oder gar keinen Sensor 18 für den Umgebungsdruck P und einen relativ einfachen und / oder deaktivierbaren oder gar keinen Sensor 17 für die Umgebungsfeuchte Hum. Diese bevorzugte Ausgestaltung hat insbesondere den nachfolgend beschriebenen Vorteil: Ein Temperatur-Sensor 14 lässt sich chemisch von der Umgebung isolieren, und zwar durch Isoliermaterial mit einer guten Wärmeleitfähigkeit. Der Temperatur-Sensor 14 vermag relativ zuverlässig die Umgebungstemperatur Temp zu messen, ist aber nicht direkt den übrigen Umgebungsbedingungen ausgesetzt. Sowohl ein Druck-Sensor als auch ein Feuchte-Sensor müssen hingegen in der Regel in einer Fluidverbindung mit der Umgebung, also mit dem zu überwachenden räumlichen Bereich B stehen, und können daher über einen sehr langen Zeitraum brennbaren Zielgasen und anderen möglicherweise schädlichen Substanzen ausgesetzt sein. Ein Sensor, der in einer Fluidverbindung mit der Umgebung steht und dennoch ausreichend robust und zuverlässig ist, ist oft relativ teuer und / oder schwer und / oder erfordert relativ viel elektrische Energie. Ein Temperatur-Sensor weist diesen Nachteil nicht oder zumindest nur in geringerem Umfang auf.

Ein schematisch gezeigtes signalverarbeitendes Steuergerät (control unit) 6 mit einer Auswerteeinheit 9 empfängt Signale von den Sensoren 12.1, 12.2, 13.1, 13.2, 14, 17, 18 und ermittelt die aktuelle Konzentration eines brennbaren Zielgases im überwachten Bereich B, leitet also einen Schätzwert her. Dieser Schätzwert ist in der Regel zeitlich veränderlich. Falls in dem Bereich B mehrere brennbare Zielgase vorhanden sind, beschreibt im Ausführungsbeispiel der Schätzwert die summierten Konzentrationen dieser brennbaren Zielgase. Das Steuergerät 6 und damit die Auswerteeinheit 9 haben wenigstens zeitweise Lesezugriff auf einen Datenspeicher 7, in dem ein Auswerteprogramm und / oder ein rechnerauswertbares Modell Mod mit mehreren funktionalen Zusammenhänge abgespeichert sind.

Ideal wäre es, wenn die Gasmessvorrichtung 100 nur mit Hilfe des Kompensators 11 und des Temperatur-Sensors 14 den Einfluss aller drei Umgebungsbedingungen, also sowohl den Einfluss der Umgebungstemperatur Temp als auch den Einfluss des Umgebungsdrucks P als auch den Einfluss der Umgebungsfeuchte Hum vollständig kompensieren würde, und zwar für jeden bei einem Einsatz möglichen Wert dieser drei Umgebungsbedingungen. Dies ist in der Praxis aber in der Regel nicht möglich, zumindest dann nicht, wenn der Umgebungsdruck P und / oder die Umgebungsfeuchte Hum im Laufe eines Einsatzes erheblich variieren können und weder ein Druck-Sensor noch ein Feuchte-Sensor vorhanden und aktiviert sind. Ein wesentlicher Grund ist, dass in vielen Fällen der Kompensator 11 und der Detektor 10 auf mindestens eine Umgebungsbedingung unterschiedlich reagieren, insbesondere aufgrund konstruktionsbedingter oder bauartbedingter Unterschiede oder unvermeidlicher Fertigungstoleranzen.

Im Ausführungsbeispiel wird für die drei Umgebungsbedingungen Temperatur Temp, Feuchte Hum und Druck P jeweils ein Wertebereich vorgegeben. Der Wertebereich für die Temperatur reicht von $Temp_{min}$ bis $Temp_{max}$, der Wertebereich für die Feuchte von $Hum_{min}$ bis $Hum_{max}$ und der Wertebereich für den Druck von $P_{min}$ bis $P_{max}$. Die Gasmessvorrichtung 100 erzielt die nachfolgend beschriebene Wirkung, wenn jede dieser drei Umgebungsbedingungen im vorgegebenen Wertebereich liegt.

Die Gasmessvorrichtung 100 des Ausführungsbeispiels lässt sich wahlweise in einem von vier möglichen unterschiedlichen Modi betreiben. In einer Ausgestaltung wird bei der Konfiguration der Gasmessvorrichtung 100 einer dieser vier Modi ausgewählt und mittels entsprechender Software realisiert. Das Modell Mod im Datenspeicher 7 ist dann für diesen ausgewählten Modus gültig. Die Gasmessvorrichtung 100 lässt sich im Betrieb nicht notwendigerweise von einem Modus in einen anderen Modus umschalten.

In einer anderen Ausgestaltung umfasst die Gasmessvorrichtung 100 hingegen einen schematisch gezeigten Schalter 16, mit dem ein Benutzer einen dieser vier möglichen Modi auswählen kann. Der Benutzer betätigt den Schalter 16, um von einem Modus in einen anderen Modus umzuschalten. Dieser Schalter 16 kann z.B. als mechanischer Schalter oder mit Hilfe eines berührungssensitiven Bildschirms (Touchscreen) oder mithilfe von mehreren Tasten realisiert sein. Im Datenspeicher 7 ist denn für jeden Modus jeweils ein Modell abgespeichert.

In einer weiteren Ausgestaltung schaltet die Gasmessvorrichtung 100 sich im laufenden Betrieb automatisch von einem Modus in den anderen Modus um, so dass sie während des Einsatzes in jedem der möglichen Modi betrieben wird. In jedem Modus ermittelt die Gasmessvorrichtung 100 jeweils einen Schätzwert für die Zielgas-Konzentration. In der Regel unterscheiden sich die Schätzwerte von Modus zu Modus. Eine bevorzugte Ausgestaltung, um einen Schätzwert herzuleiten und bevorzugt einem Benutzer anzuzeigen, ist die folgende: Solange ein Schätzwert, der in einem Modus ermittelt wird, in einem vorgegebenen Soll-Konzentrationsbereich liegt, wird dieser Schätzwert ausgegeben. Oder es wird gar keine Mitteilung ausgegeben oder die Mitteilung, dass keine unzulässige Zielgas-Konzentration vorliegt. Falls hingegen mindestens ein Schätzwert außerhalb des vorgegebenen Soll-Konzentrationsbereichs liegt, so wird derjenige Schätzwert ausgegeben, der am weitesten vom vorgegebenen Soll-Konzentrationsbereich entfernt ist, also der größte Schätzwert eines gefährlichen Zielgases oder auch der kleinste Schätzwert eines lebensnotwendigen Zielgases, z.B. Sauerstoff. Bei dieser Ausgestaltung "liegt man auf der sicheren Seite".

Im Ausführungsbeispiel werden folgende vier Modi unterschieden:

P          Der Einfluss des Umgebungsdrucks P wird bestmöglich kompensiert (druckoptimierter Modus).

Hum        Der Einfluss der Umgebungsfeuchte Hum wird bestmöglich kompensiert (feuchteoptimierter Modus).

P_Hum      Der Einfluss des Umgebungsdrucks P wird so gut kompensiert, wie dies bei Einhaltung einer die Umgebungsfeuchte Hum betreffenden Randbedingung möglich ist (druckkompensierender Modus).

Hum_P      der Einfluss der Umgebungsfeuchte wird so gut kompensiert, wie dies bei Einhaltung einer den Umgebungsdruck P betreffenden Randbedingung möglich ist (feuchtekompensierender Modus).

Die Randbedingung, die die Umgebungsfeuchte Hum betrifft, ist vorgegeben und legt beispielsweise fest, dass der Messwert con für die Zielgas-Konzentration Con dann um höchstens x % variiert, wenn die Umgebungsfeuchte Hum im vorgegebenen Feuchtebereich von $Hum_{min}$ bis $Hum_{max}$ bleibt und die tatsächliche Zielgas-Konzentration con konstant bleibt. Bei einem Einsatz liegt die Umgebungsfeuchte Hum stets in diesem Feuchtebereich. Das Entsprechende gilt für die Randbedingung, die den Umgebungsdruck P betrifft, d.h. der Umgebungsdruck liegt im Bereich von $P_{min}$ bis $P_{max}$.

Die Gasmessvorrichtung 100 lässt sich also in einem von vier möglichen Modi betreiben. Dadurch lässt sich die Gasmessvorrichtung 100 an unterschiedliche Einsatzbedingungen anpassen.

Eine mögliche Einsatzbedingung ist die folgende: Die Zielgas-Konzentration in einem Rohr soll gemessen werden, wobei ein Gasgemisch durch dieses Rohr fließt. Bei dieser Einsatzbedingung kann der Umgebungsdruck P stark schwanken, weswegen der Modus P oder der Modus P_Hum sinnvoll sind.

Eine andere mögliche Einsatzbedingung ist die folgende: Die Zielgas-Konzentration in einem umschlossenen Raum soll gemessen werden. Dieser umschlossene Raum steht nur über eine relativ kleine Öffnung mit der Umgebung in einer Fluidverbindung und ist beispielsweise ein Behälter für Fluide. Oder der umschlossene Raum ist eine Prüfkammer, in der unterschiedliche Umgebungsbedingungen und insbesondere verschiedene Feuchten erzeugt werden, um ein Bauteil zu prüfen. Bei dieser anderen Einsatzbedingung kann die Umgebungsfeuchte Hum stark schwanken, weswegen der Modus Hum oder der Modus Hum_P sinnvoll sind.

Die Erfindung ermöglicht es, eine Menge von baugleichen Gasmessvorrichtungen zu produzieren und jede Gasmessvorrichtung 100 dieser Menge an die jeweilige Einsatzbedingung dadurch anzupassen, dass eine der vier möglichen Modi ausgewählt wird. Die Auswahl wird vorab oder im laufenden Betrieb getroffen. Der Betrieb in einem bestimmten Modus erfordert die Implementierung oder auch die Auswahl von Software, während die Hardware unverändert bleibt. Diese Ausgestaltung erhöht in vielen Fällen die Zuverlässigkeit und reduziert die Entwurfs- und Baukosten verglichen mit einer Ausgestaltung, bei der mindestens zwei Mengen von unterschiedlichen Gasmessvorrichtungen hergestellt werden, nämlich jeweils eine Menge für eine Einsatzbedingung.

In der nachfolgenden Darstellung fungiert die am Detektor 10 anliegende elektrische Detektor-Spannung U10 als die Detektionsgröße, die mit der Temperatur des heizenden Segments 20 des Detektors 10 korreliert, vgl. Figur 1. Die Stromstärke I.1 wird konstant gehalten. Entsprechend fungiert die am Kompensator 11 anliegende elektrische Kompensator-Spannung U11 als die Detektionsgröße, die mit der Temperatur des heizenden Segments 38 des Kompensators 11 korreliert. Die Stromstärke I.2 wird konstant gehalten.

Die drei Sensoren 14, 17 und 18 liefern ebenfalls jeweils ein elektrisches Signal - natürlich nur dann, wenn sie tatsächlich vorhanden, intakt und aktiviert sind. Dieses Signal wird mit $U(\Delta Temp)$, $U(Hum)$ bzw. $U(\Delta P)$ bezeichnet. Im Ausführungsbeispiel wird die Annahme verwendet, dass die drei Umgebungsbedingungen Umgebungstemperatur Temp, Umgebungsfeuchte Hum und Umgebungsdruck P jeweils linear auf die Detektionsgrößen wirken. Daher wird vorab für jede dieser drei Umgebungsbedingungen jeweils ein Proportionalitätsfaktor ermittelt, bevorzugt empirisch ermittelt, und beim Einsatz verwendet.

Die Auswerteeinheit 9 berechnet abhängig von den Werten für die fünf Variablen U10, U11, $U(\Delta Temp)$, $U(Hum)$, $U(\Delta P)$ einen Wert für eine Gesamt-Detektionsgröße Det. Hierbei wendet die Auswerteeinheit 9 die Berechnungsvorschrift

$$(1) \qquad Det = F[U10, U11, U(\Delta Temp), U(Hum), U(\Delta P)]$$

an. In einer Ausgestaltung hängt die Funktion F von jeweils mindestens einem Parameter für U10 und U11 ab, optional von mindestens einem weiteren Parameter. In einer bevorzugten Realisierungsform dieser Ausgestaltung hat die Berechnungsvorschrift (1) die Form

$$(2) \qquad Det = U10 - \alpha*U11 - \beta*U(\Delta Temp) - \gamma*U(Hum) - \xi*U(\Delta P) - x0.$$

mit einem Verstärkungsfaktor $\alpha$ für die Kompensator-Spannung U11, einem Verstärkungsfaktor $\beta$ für das Signal $U(\Delta Temp)$ des Temperatur-Sensors 14, einem Verstärkungsfaktor $\gamma$ für das Signal $U(Hum)$ des Feuchte-Sensors 17, einem Verstärkungsfaktor $\xi$ für das Signal $U(\Delta P)$ des Druck-Sensors 18 und einem Nullwert x0. Der Verstärkungsfaktor $\alpha$ kompensiert bis zu einem gewissen Grad bauartbedingte Unterschiede zwischen dem Detektor 10 und dem Kompensator 11 und ist bevorzugt größer als 1,1. Der Verstärkungsfaktor $\beta$ kompensiert bis zu einem gewissen Grad den als linear angenommenen Einfluss der Umgebungstemperatur Temp auf die Gesamt-Detektionsgröße Det. Entsprechend kompensieren die Faktoren y und $\xi$ den ebenfalls als jeweils linear angenommenen Einfluss der Umgebungsfeuchte Hum beziehungsweise des Umgebungsdrucks $\Delta P$.

Falls weder ein Feuchte-Sensor 17 noch ein Druck-Sensor 18 vorhanden ist oder beide Sensoren 17, 18 deaktiviert oder defekt sind, so wird folgende Berechnungsvorschrift angewendet:

$$(3) \qquad Det = U10 - \alpha*U11 - \beta*U(\Delta Temp) - x0.$$

mit einem anderen Faktor x0 als in der Rechenvorschrift (2).

Die Parameter der Funktion F in den Rechenvorschrift (1) bis (3) werden empirisch mit Hilfe einer Stichprobe berechnet. Möglich ist auch, dass die Funktion F die Form eines neuronalen Netzes hat oder durch ein sonstiges lernendes Verfahren erzeugt wird.

Aus dem Wert für die Gesamt-Detektionsgröße Det berechnet die Auswerteeinheit 9 einen Wert für die Zielgas-Konzentration Con und wendet hierbei die Rechenvorschrift

$$(4) \qquad Con_{meas} = F_{Con}(Det)$$

an. Beispielsweise gilt

$$(5) \qquad Con_{meas} = \gamma_{Con}*Det$$

mit einem empirisch bestimmten Faktor $\gamma_{Con}$. Anmerkung: Mit Con wird die tatsächliche Zielgas-Konzentration bezeichnet, mit $Con_{meas}$ die gemessene Größe. Idealerweise gilt $Con_{meas} = Con$.

Bevorzugt wird die Berechnungsvorschrift (4) oder (5) so festgelegt, dass ein Wert von null für die Gesamt-Detektionsgröße Det zu einem Wert von null für die Zielgas-Konzentration Con führt.

Sowohl die Funktion F als auch die Funktion $F_{Con}$ sind in geeigneter rechnerauswertbarer Weise im Datenspeicher 7 abgespeichert und bilden einen Teil des Modells Mod.

Je nach dem angewendeten Modus verwendet die Auswerteeinheit 9 als Funktion F in der Berechnungsvorschrift (1) eine Funktion $F_P$, $F_{P\_Hum}$, $F_{Hum}$ oder $F_{Hum\_P}$. Falls die Berechnungsvorschrift (2) oder (3) angewendet wird, so wird je nach Modus als Verstärkungsfaktor $\alpha$ für die Kompensator-Spannung U11 ein Verstärkungsfaktor $\alpha_P$, $\alpha_{P\_Hum}$, $\alpha_{Hum}$ oder $\alpha_{Hum\_P}$ angewendet. Entsprechend wird als Verstärkungsfaktor $\beta$ für das Signal $U(\Delta Temp)$ des Temperatur-Sensors 14 ein Verstärkungsfaktor $\beta_P$, $\beta_{P\_Hum}$, $\beta_{Hum}$ oder $\beta_{Hum\_P}$ angewendet. In einer Ausgestaltung werden entsprechend vier verschiedene Nullwerte angewendet, in einer anderen Ausgestaltung unabhängig vom Modus stets derselbe Nullwert x0.

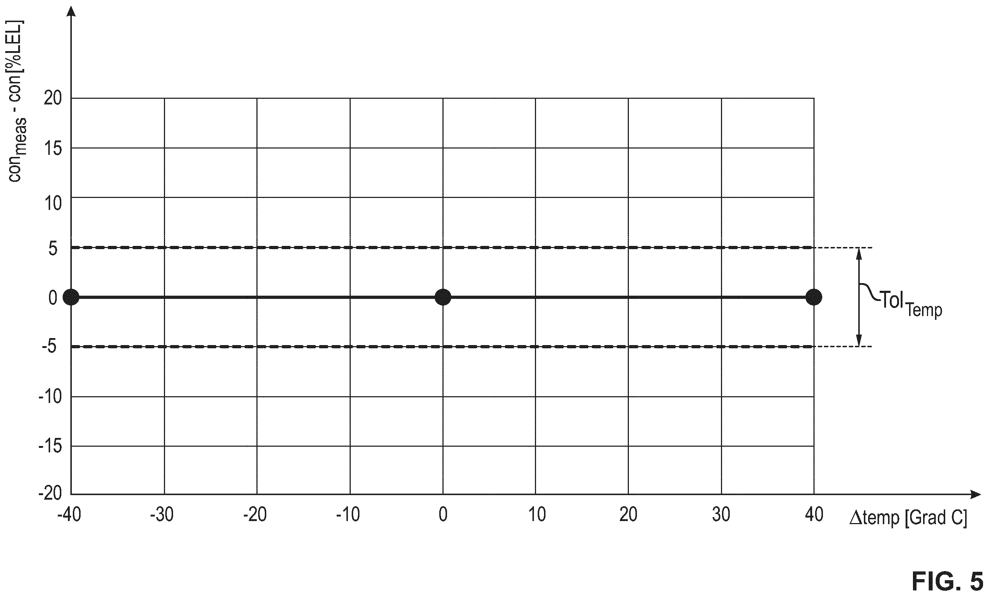

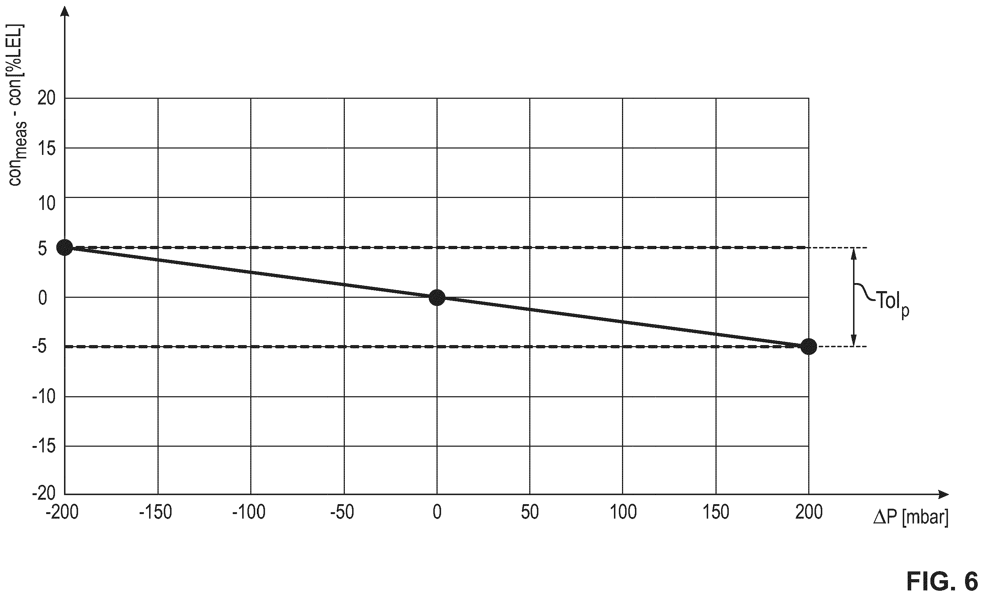

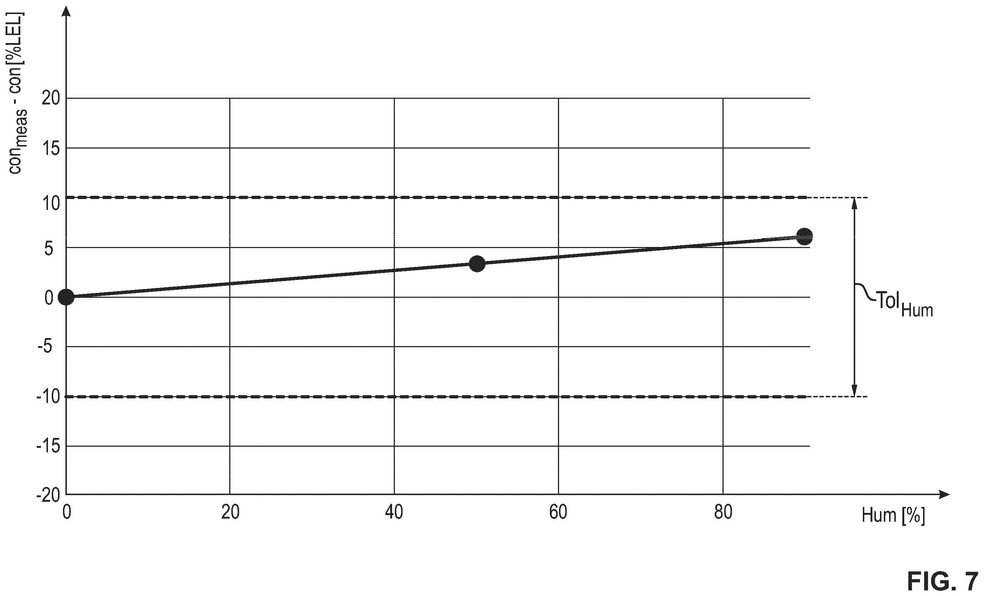

Figur 5 bis Figur 7 veranschaulichen vorgegebene Randbedingungen dafür, wie der Messwert $Con_{meas}$ für die Zielgas-Konzentration Con von der Temperaturdifferenz $\Delta Temp$ (Figur 5), vom Umgebungsdruck P (Figur 6) bzw. von der Umgebungsfeuchte Hum (Figur 7) abhängt. In diesem Beispiel wurden die Berechnungsvorschrift (3) mit den Verstärkungsfaktoren von $\alpha$ = 1,8 und $\beta$= -1,11 sowie die Berechnungsvorschrift (5) angewendet. Auf der x-Achse ist die Abweichung $\Delta Temp$ der Umgebungstemperatur Temp von einer Referenz-Umgebungstemperatur in [Grad C] (Figur 5), die Abweichung $\Delta P$ des Umgebungsdrucks P von einem vorgegebenen Referenzdruck in [mbar] (Figur 6) bzw. die Umgebungsfeuchte Hum in [% rel. Feuchte] (Figur 7) aufgetragen. Auf der y-Achse ist jeweils die Abweichung zwischen dem Messwert $con_{meas}$ und der tatsächlichen Zielgas-Konzentration con in [% UEG] aufgetragen. Dargestellt sind ein Toleranzband $Tol_{Temp}$ für die Abhängigkeit von der Umgebungstemperatur Temp, ein Toleranzband $Tol_P$ für die Abhängigkeit vom Umgebungsdruck P und ein Toleranzband $Tol_{Hum}$ für die Abhängigkeit von der Umgebungsfeuchte Hum.

Nachfolgend wird beispielhaft beschrieben, wie die oben erwähnten Rechenvorschriften empirisch ermittelt werden. Mindestens eine Stichprobe wird generiert. Um die oder eine Stichprobe zu generieren, wird die Gasmessvorrichtung nacheinander unterschiedlichen definierten Testumgebungen ausgesetzt. Jede Testumgebung weist drei Werte temp, hum, p für die drei Umgebungsbedingungen Temperatur, Feuchte, Druck sowie einen Wert für die Zielgas-Konzentration auf. Jedes gewonnene Stichprobenelement umfasst jeweils

- einem resultierenden Signalwert $u(\Delta temp)$ des Temperatursensors 14,
- falls die entsprechenden Sensoren vorhanden sind, jeweils einen resultierenden Signalwert $u(hum)$, $u(\Delta p)$ des Feuchte-Sensors 17 und des Druck-Sensors 18 und
- einen Wert con für die Zielgas-Konzentration Con.

Jede Festlegung einer Berechnungsvorschrift (1) bis (5) führt für jedes Stichprobenelement jeweils zu

- einem resultierenden Signalwert u10 der Detektor-Detektionsgröße U10 und u11 der Kompensator-Detektionsgröße U11 und
- einem resultierenden Signalwert u(det) der Gesamt-Detektionsgröße Det.

Verschiedene nachfolgend beschriebenen Ausgestaltungen reduzieren den Aufwand, um die verwendeten Berechnungsvorschriften zu ermitteln.

Bevorzugt wird für jeden Modus dieselbe Funktion $F_{Con}$ in der Berechnungsvorschrift (4) ermittelt, wofür die Stichprobe verwendet wird. Unter Verwendung der Stichprobe werden weiterhin die Funktionen $F_P$, $F_{P\_Hum}$, $F_{Hum}$ oder $F_{Hum\_P}$ in der Berechnungsvorschrift (1) ermittelt.

Bevorzugt decken in der Stichprobe die Werte für die Umgebungstemperatur Temp den gesamten Wertebereich von $Temp_{min}$ bis $Temp_{max}$ ab. Mindestens dann, wenn die Gasmessvorrichtung 100 im druckkompensierenden oder druckoptimierten Modus verwendet werden soll, decken in der Stichprobe die Werte für den Umgebungsdruck P den gesamten Wertebereich von $P_{min}$ bis $P_{max}$ ab. Entsprechend decken mindestens dann, wenn die Gasmessvorrichtung 100 im feuchtekompensierenden oder feuchteoptimierten Modus verwendet werden soll, in der Stichprobe die Werte für die Umgebungsfeuchte Hum den gesamten Wertebereich von $Hum_{min}$ bis $Hum_{max}$ ab. Bevorzugt decken in der Stichprobe die Werte für die tatsächliche Zielgas-Konzentration den gesamten Wertebereich ab, bei dem die Gasmessvorrichtung 100 eingesetzt werden kann.

In vielen Fällen hängen die Funktionen $F_P$, $F_{P\_Hum}$, $F_{Hum}$ oder $F_{Hum\_P}$ in der Berechnungsvorschrift (1) und die Funktion $F_{Con}$ in der Berechnungsvorschrift (4) monoton von den Argumenten ab. Dies gilt insbesondere für die Funktionen (2), (3) und (5), die jeweils linear von ihren Argumenten abhängen. Daher reicht es in vielen Fällen aus, dass in der Stichprobe für jede Umgebungsbedingung Temp, Hum, P und für die Zielgas-Konzentration Con jeweils die beiden Extremwerte und mindestens ein, bevorzugt mindestens drei dazwischenliegende Werte verwendet werden. Falls beispielsweise ein dazwischenliegender Wert verwendet wird, so hat die Stichprobe $3^4$ = 81 Stichprobenelemente.

In einer Ausgestaltung wird aus der Stichprobe eine Teil-Stichprobe mit mehreren Stichprobenelementen ausgewählt. Diese Teil-Stichprobe umfasst unterschiedliche Werte für die Zielgas-Konzentration. Mithilfe der Teil-Stichprobe wird empirisch die Funktion in der Berechnungsvorschrift (4) ermittelt, beispielsweise der Faktor $y_{Con}$ in der Berechnungsvorschrift (5). Mit den restlichen Stichprobenelementen werden die Funktionen $F_P$, $F_{P\_Hum}$, $F_{Hum}$ und / oder $F_{Hum\_P}$ ermittelt. Die Berechnungsvorschrift (4), die mithilfe der Teil-Stichprobe ermittelt wurde, wird beibehalten. Jedes restliche Stichprobenelement liefert daher einen berechneten Wert $con_{meas}$ für die Zielgas-Konzentration Con.

Nachfolgend wird beispielhaft beschrieben, wie Werte für die Parameter der Berechnungsvorschrift (2) hergeleitet werden. Bei dieser Herleitung wird davon ausgegangen, dass die drei Umgebungsbedingungen Temp, P, Hum und die tatsächliche Zielgas-Konzentration Con unabhängig voneinander auf die Gesamt-Detektionsgröße Det und damit auf die gemessene Zielgas-Konzentration $Con_{meas}$ einwirken. Außerdem wird davon ausgegangen, dass die Umgebungstemperatur Temp annähernd linear auf die Gesamt-Detektionsgröße Det und damit auf die gemessene Zielgas-Konzentration $Con_{meas}$ einwirkt, so dass der Einfluss der Umgebungstemperatur Temp ausreichend genau mit dem Faktor $\beta$ kompensiert werden kann. Diese Annahmen stimmen in vielen Fällen ausreichend genau mit der Realität überein.

Zunächst wird eine sogenannte Nullpunkt-Justierung vorgenommen. Diese Nullpunkt-Justierung wird am Beispiel der Berechnungsvorschrift (2) erläutert. Hierbei wird die Gasmessvorrichtung 100 einer definierten Referenz-Testumgebung $Cond_{Ref}$ ausgesetzt. Die Referenz-Testumgebung $Cond_{Ref}$ weist eine Referenz-Konzentration $con_{Ref}$ von zu detektierendem Zielgas, eine Referenz-Umgebungstemperatur $temp_{Ref}$, einen-Referenz-Umgebungsdruck $p_{Ref}$ und eine Referenz-Umgebungsfeuchte $hum_{Ref}$ auf. Beispielsweise sind $con_{Ref}$ = 0 und $Hum_{Ref}$ = 0 %, die Zielgas-Konzentration Con und die Umgebungsfeuchte Hum nehmen also den jeweils geringstmöglichen Wert an.

In einer Realisierungsform wird der Faktor $\beta_{Ref}$ anfänglich auf 0 gesetzt, d.h. der Einfluss der Umgebungstemperatur Temp und damit das Signal U($\Delta$Temp) werden anfänglich vernachlässigt. Der Faktor $\alpha_{Ref}$ wird anfänglich auf 1 gesetzt, d.h. die Gesamt-Detektionsgröße Det hängt von der Differenz zwischen den beiden Spannungen U10 und U11 ab. Im Laufe der Nullpunkt-Justierung können die Faktoren $\alpha_{Ref}$ und $\beta_{Ref}$ andere Werte einnehmen. Der Nullwert x0 wird stets so eingestellt, dass die Gesamt-Detektionsgröße Det und damit die gemessene Zielgas-Konzentration $Con_{meas}$ für die Referenz-Testumgebung $Cond_{Ref}$, also bei Abwesenheit von brennbarem Zielgas, den Wert 0 annehmen.

Anschließend wird empirisch ein Wert für den Faktor $y_{Con}$ in der Berechnungsvorschrift (5) ermittelt. Die Referenz-Testumgebung $Cond_{Ref}$ wird dergestalt abgewandelt, dass sie nacheinander verschiedene Zielgas-Konzentrationen con(1), ... aufweist. Für jede Zielgas-Konzentration con(1), ... liefert die Gasmessvorrichtung 100 jeweils einen Wert det(1), ... für die Gesamt-Detektionsgröße Det. Hierfür verwendet die Gasmessvorrichtung 100 die gerade ermittelten oder eingestellten Faktoren $\alpha_{Ref}$, $\beta_{Ref}$ und x0. Dieses Vorgehen liefert eine Stichprobe {[con(1), det(1)], ...}. Der Faktor $y_{Con}$ wird unter Verwendung dieser Stichprobe mithilfe einer Regressionsanalyse festgelegt.

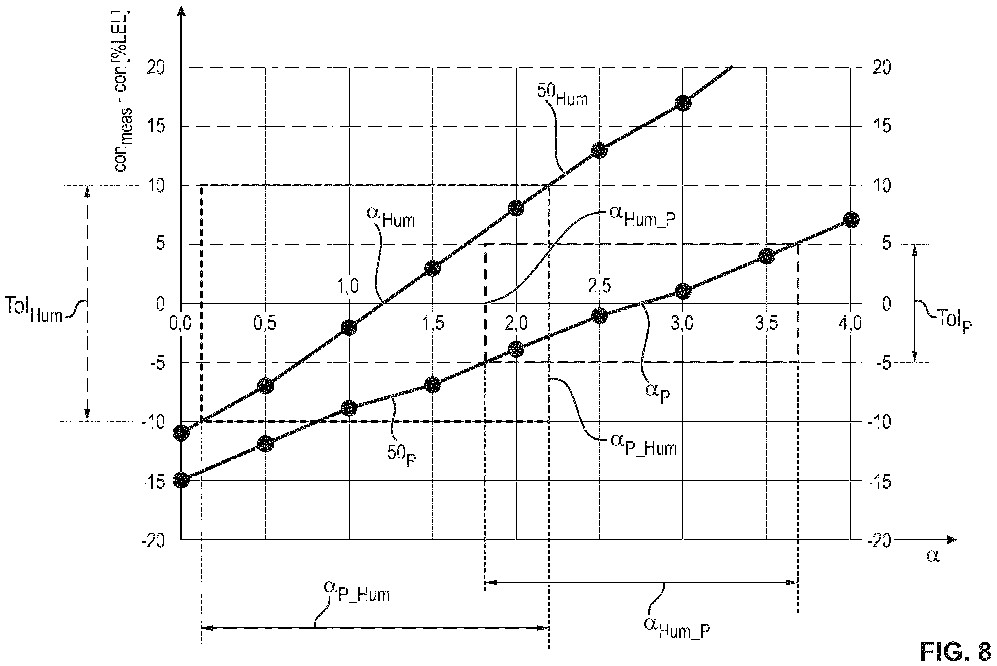

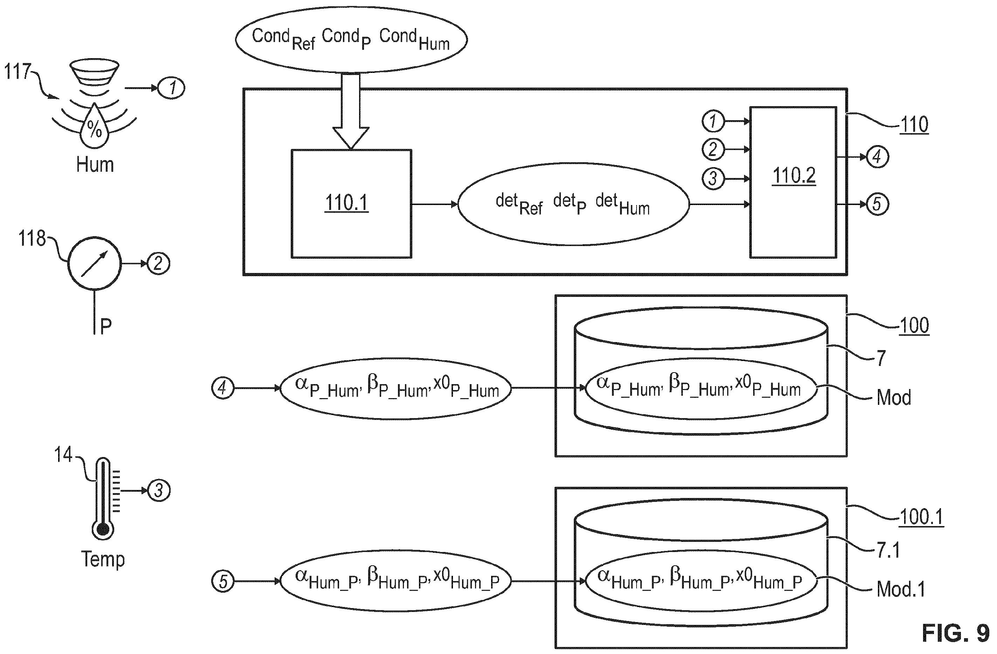

Nunmehr wird mindestens eine gegenüber der Referenz-Testumgebung $Cond_{Ref}$ veränderte Testumgebung hergestellt, indem mindestens eine Umgebungsbedingung verändert wird. Figur 8 und Figur 9 veranschaulichen beispielhaft ein Vorgehen, bei der die Gasmessvorrichtung 100 zwei verschiedenen definierten Testumgebungen $Cond_{Hum}$ und $Cond_P$ ausgesetzt wird. In Figur 9 werden eine Kalibriervorrichtung 110 mit einem ersten Bestandteil 110.1 und einem zweiten Bestandteil 110.2 sowie eine weitere Gasmessvorrichtung 100.1 gezeigt, wobei die Gasmessvorrichtung 100.1 zur Gasmessvorrichtung 100 baugleich ist.

Die Testumgebung $Cond_{Hum}$ weist eine deutlich erhöhte Umgebungsfeuchte Hum auf als die Referenz-Testumgebung $Cond_{Ref}$, während die anderen Umgebungsbedingungen Con, Temp, P gleich sind. Beispielsweise beträgt die relative Umgebungsfeuchte Hum bei der Referenz-Testumgebung $Cond_{Ref}$ 0% und bei der Testumgebung $Cond_{Hum}$ 90%, das ist der größte Wert, bei dem die Gasmessvorrichtung 100 noch eingesetzt werden kann. Die Testumgebung $Cond_P$ weist eine deutlich erhöhten oder auch verringerten Umgebungsdruck P auf als die Referenz-Testumgebung $Cond_{Ref}$, während die anderen Umgebungsbedingungen Con, Temp, Hum gleich sind. Beispielsweise weist die Testumgebung $Cond_P$ einen um 200 mbar erhöhten Umgebungsdruck P auf als die Referenz-Testumgebung $Cond_{Ref}$.

Die Gasmessvorrichtung 100 liefert bei der Testumgebung $Cond_{Hum}$ jeweils einen Wert u10($Cond_{Hum}$), u11($Cond_{Hum}$), u($\Delta$Temp) ($Cond_{Hum}$) für die drei Signale U10, U11, U($\Delta$Temp), bei der Testumgebung $Cond_P$ jeweils einen Wert u10($Cond_P$), u11($Cond_P$), u($\Delta$Temp) ($Cond_P$). Zunächst werden die Werte $\beta_{Ref}$ und $y_{Con}$, die so wie gerade beschrieben ermittelt oder eingestellt worden sind, verwendet. Für den Verstärkungsfaktor $\alpha$ wird ein Wert ermittelt, was nachfolgend beschrieben wird. Der Nullwert x0 wird so eingestellt, dass in der Referenz-Testumgebung $Cond_{Ref}$, also bei Abwesenheit von brennbarem Zielgas, eine Zielgas-Konzentration von Null gemessen wird.

Figur 8 zeigt zwei Messkurven $50_P$, $50_{Hum}$, die mit derselben Gasmessvorrichtung 100 erzeugt wurden. Auf der x-Achse ist der Verstärkungsfaktor $\alpha$ aufgetragen, auf der y-Achse die resultierende Abweichung des Messergebnisses $con_{meas}$ von der tatsächlichen Zielgas-Konzentration con in % LEL (Lower Explosive Level, Untere Explosionsgrenze UEG), wobei diese Abweichung $con_{meas}$ - con vom Verstärkungsfaktor $\alpha$ abhängt. Hierfür wurden die Berechnungsvorschriften (3) und (5) angewendet. Daher können eine positive oder eine negative Abweichung der gemessenen Zielgas-Konzentration $Con_{meas}$ von der tatsächlichen Zielgas-Konzentrationen Con auftreten. Die Messkurve $50_P$ wurde bei der Testumgebung $Cond_P$ gewonnen, die Messkurve $50_{Hum}$ bei der Testumgebung $Cond_{Hum}$.

Zu sehen ist, dass die Messkurve $50_{Hum}$ die x-Achse bei $\alpha_{Hum}$ = 1,2 schneidet. Dies bedeutet: Beim Wert $\alpha_{Hum}$ = 1,2 für den Verstärkungsfaktor $\alpha$ und beim resultierenden Nullwert $_XO_{Hum}$ liefert die Gasmessvorrichtung 100 den richtigen Wert 0 für die Zielgas-Konzentration Con. Dies gilt für die Testumgebung $Cond_{Hum}$. In vielen Fällen ist die Annahme gerechtfertigt, dass der Wert $\alpha_{Hum}$ = 1,2 auch bei einer niedrigeren Umgebungsfeuchte Hum zu einem richtigen Wert für die Zielgas-Konzentration Con führt. Anschließend wird der Wert $\beta$, mit dem der Einfluss der Umgebungstemperatur Temp kompensiert wird, eingestellt, beispielsweise indem die Gasmessverrichtung 100 einer veränderten Umgebungstemperatur ausgesetzt wird und der resultierende Messwert $con_{meas}$ geprüft wird. Dieses Vorgehen liefert einen Satz $\alpha_{Hum}$, $\beta_{Hum}$, $_X O_{Hum}$ von Parameterwerten. Dieser Satz von Parameterwerten wird für den feuchteoptimierten Modus Hum verwendet

In Figur 8 ist anhand der Messkurve $50_P$ zu sehen, dass der feuchteoptimierte Modus Hum bei einem veränderten Druck P zu einem falschen Messwert führt, nämlich zu $con_{meas}$ - con = -8,5 LEL. Dies wird in manchen Anwendungen in Kauf genommen, beispielsweise wenn die Gasmessvorrichtung 100 in einer Prüfkammer mit stark variierender Umgebungsfeuchte Hum eingesetzt wird. Im feuchtekompensierenden Modus Hum_P soll hingegen eine vorgegebene Randbedingung betreffend den Umgebungsdruck P eingehalten werden. Die gemessene Zielgas-Konzentration $Con_{meas}$ soll bei jedem Umgebungsdruck P und daher auch jeder Druckdifferenz $\Delta$P von der tatsächlichen Zielgas-Konzentration Con um maximal 5% LEL differieren. Diese Randbedingung wird in Figur 8 durch ein Toleranzband $Tol_P$ = +- 5% LEL um die x-Achse veranschaulicht. Der Einfluss der Umgebungsfeuchte Hum soll im feuchtekompensierenden Modus Hum_P unter Einhaltung dieser Randbedingung bestmöglich kompensiert werden. Wie man aus Figur 8 ablesen kann, führt die Einhaltung dieser Randbedingung zu einem Wert $\alpha_{Hum\_P}$ = 1,8 LEL. Ein Satz von Parameterwerten $\alpha_{Hum\_P}$, $\beta_{Hum\_P}$, $_X 0_{Hum\_P}$ für den feuchtekompensierenden Modus Hum_P wird hergeleitet.

Die Messkurve $50_P$ schneidet die x-Achse in $\alpha_P$ = 2,8. Dieser Wert wird für den druckoptimierten Modus P verwendet. Ein Satz von Parameterwerten $\alpha_P$, $\beta_P$, $_X O_P$ für den druckoptimierten Modus P wird hergeleitet. Für den druckkompensierenden Modus P_Hum wird die Randbedingung verwendet, dass die gemessene Zielgas-Konzentration $Con_{meas}$ von der tatsächlichen Zielgas-Konzentration Con bei jeder Umgebungsfeuchte Hum um maximal 10% LEL abweichen soll. Diese Randbedingung wird in Figur 8 durch das Toleranzband $Tol_{Hum}$ angedeutet. Diese Randbedingung führt zu einem Wert $\alpha_{P\_Hum}$ = 2,3 für den druckkompensierenden Modus P_Hum. Ein Satz von Parameterwerten $\alpha_{P\_Hum}$, $\beta_{P\_Hum}$, $_X 0_{P\_Hum}$ für den druckkompensierenden Modus P_Hum wird hergeleitet.

Wie bereits dargelegt, ist in vielen Fällen die Annahme gerechtfertigt, dass die Zielgas-Konzentration Con unabhängig von den Umgebungsbedingungen Temp, P, Hum auf die Detektionsgrößen U10, U11, U($\Delta$Temp) einwirkt. Daher wird bevorzugt die Berechnungsvorschrift (5) mit dem bereits ermittelten Faktor $\gamma_{Con}$ verwendet. Möglich ist auch, für jeden Modus jeweils einen Faktor $\gamma_{Con}$ empirisch zu ermitteln.

Figur 9 veranschaulicht beispielhaft, wie zwei Gasmessvorrichtungen 100 und 100.1 mithilfe einer Kalibriervorrichtung 110 vor dem ersten Einsatz kalibriert werden. Die beiden Gasmessvorrichtungen 100 und 100.1 sind baugleich und weisen insbesondere die gleichen Detektoren und Kompensatoren auf. Sie sind beispielsweise so wie mit Bezug auf Figur 1 beschrieben aufgebaut, umfassen aber nicht notwendigerweise einen Schalter 16. Die Gasmessvorrichtung 100 soll im druckkompensierenden Modus P_Hum betrieben werden, die Gasmessvorrichtung 100.1 im feuchtekompensierenden Modus Hum_P. Die Kalibriervorrichtung 110 liefert einen Satz $\alpha_{P\_Hum}$, $\beta_{P\_Hum}$, $_x 0_{P\_Hum}$ von Parameterwerten für den druckkompensierenden Modus P_Hum und einen Satz $\alpha_{Hum\_P}$, $\beta_{Hum\_P}$, $_x 0_{Hum\_P}$ von Parameterwerten für den feuchtekompensierenden Modus Hum_P. Um diese beiden Sätze von Parameterwerten herzuleiten, wird die Gasmessvorrichtung 100 mit den Temperatur-Sensor 14 verwendet. Außerdem werden ein Feuchte-Sensor 117 und ein Druck-Sensor 118 verwendet, um die jeweilige Testumgebung $Cond_{Ref}$, $Cond_P$, $Cond_{Hum}$ herzustellen. Die beiden Sensoren 117 und 118 sind robust und zuverlässig und werden nur für die Kalibrierung verwendet und sind keine Bestandteile der Gasmessvorrichtungen 100, 100.1.

Bei dem gerade beschriebenen Vorgehen werden die Berechnungsvorschriften (3) und (5) verwendet, und drei verschiedene Testumgebungen $Cond_{Ref}$, $Cond_P$, $Cond_{Hum}$ werden verwendet. In vielen Fällen führt dieses Vorgehen zu einer Gasmessvorrichtung 100, 100.1, die in dem jeweiligen Modus ausreichend genau die Zielgas-Konzentration Con zu messen vermag. Nachfolgend wird ein Vorgehen beschrieben, das allgemeiner anwendbar ist. Dieses Vorgehen erfordert mehr Aufwand und Rechenzeit.

Zunächst wird die Kalibrierung für den Modus P beschrieben, also in dem Modus, bei dem der Einfluss des Umgebungsdrucks P bestmöglich kompensiert wird. In der in Figur 1 gezeigten Stellung steht der Schalter 16 auf diesem Modus P.

Nachfolgend wird beispielhaft beschrieben, wie die Funktion F = $F_P$ empirisch bestimmt wird. Eine erste Messwerte-Stichprobe wird ermittelt. Für die erste Messwerte-Stichprobe werden bevorzugt folgende Bedingungen hergestellt: Kein brennbares Zielgas ist vorhanden, d.h. die Zielgas-Konzentration beträgt Null. Die Umgebungsfeuchte Hum nimmt einen gleichbleibenden Wert $hum_0$ an, beispielsweise 0%. Der Umgebungsdruck P nimmt M verschiedene Werte p(1), ..., p(M) aus dem Wertebereich von $P_{min}$ bis $P_{max}$ an, die Umgebungstemperatur N verschiedene Werte temp(1), ..., temp(N) aus dem Wertebereich von $Temp_{min}$ bis $Temp_{max}$. Bevorzugt gilt N < M. Möglich ist, dass N gleich 1 gilt, dass also die Umgebungstemperatur Temp für alle Werte der ersten Messwerte-Stichprobe die gleiche ist. Die M Werte p(1), ..., p(M) für den Umgebungsdruck P und die N Werte temp(1), ..., temp(N) für die Umgebungstemperatur Temp sind so gewählt, dass sie bei einem Einsatz tatsächlich auftreten können.

Für die erste Messwerte-Stichprobe werden insgesamt also M*N verschiedene Bedingungen hergestellt. Jede Bedingung $x_{i,j}$ (i=1, ..., M; j=1, ..., N) legt jeweils eine gleichbleibende Zielgas-Konzentration con(1) [bevorzugt con(1) gleich Null], einen Umgebungsdruck p(i), eine Umgebungstemperatur temp(j) und die Umgebungsfeuchte hum(1) fest. Die Gasmessvorrichtung 100 wird nacheinander diesen M*N verschiedenen Bedingungen $x_{1,1}$, ..., $x_{M,N}$ ausgesetzt, und die drei Detektionsgrößen U10, U11, U($\Delta$Temp) werden gemessen. Jede Bedingung $x_{i,j}$ führt zu jeweils drei Messwerten $u10(x_{i,j})$, $u11(x_{i,j})$, $u(\Delta Temp)(x_{i,j})$ für die drei Variablen U10, U11, U($\Delta$Temp). Die erste Messwerte-Stichprobe besteht also aus M*N Stichprobenelementen, wobei jedes Stichprobenelement die Form

$$(6) \qquad \{[u10(x_{i,j}),\ u11(x_{i,j}),\ u(\Delta Temp)(x_{i,j})];\ [p(i),\ \Delta temp(j),\ hum(1)]\}$$

aufweist.

Wie oben bereits dargelegt, hängt die Funktion F = $F_P$ in der Berechnungsvorschrift (1) von mehreren Parametern Par(1), ..., Par(x) ab, x >= 2. Falls beispielsweise die Berechnungsvorschrift (3) verwendet wird, so sind dies die x = 3 Parameter $\alpha = \alpha_P$, $\beta = \beta_P$, x0 = $x0_P$ oder bei konstantem Nullwert x0 die beiden Parameter $\alpha = \alpha_P$ und $\beta = \beta_P$. Falls jedem Parameter Par(1), ..., Par(x) in der Funktion $F_P$ jeweils ein Wert par(1), ..., par(x) zugewiesen wird und dann die Berechnungsvorschrift (1) auf ein Tripel [u10($x_{i,j}$), u11($x_{i,j}$), u($\Delta$Temp)($x_{i,j}$)] angewendet wird, so liefert die Anwendung einen Wert

$$(7) \qquad det(x_{i,j}) = F_P[u10(x_{i,j}),\ u11(x_{i,j}),\ u(\Delta Temp)(x_{i,j})]$$

für die Gesamt-Detektionsgröße Det. Dies liefert eine erste Detektionsgrößen-Stichprobe mit M*N Stichprobenelementen, wobei jedes Stichprobenelement die Form

$$(8) \qquad \{\ det(x_{i,j});\ [u10(x_{i,j}),\ u11(x_{i,j}),\ u(\Delta Temp)(x_{i,j})]\ \}$$

hat (i=1, ..., M; j=1, ..., N).

Beim Betrieb im Modus P soll der Einfluss des Umgebungsdrucks P so weit wie möglich rechnerisch kompensiert werden. Dies bedeutet: Die Gesamt-Detektionsgröße Det wird so festgelegt, dass sie so wenig wie möglich, idealerweise überhaupt nicht, vom Umgebungsdruck P abhängt. In Kauf genommen wird, dass sie relativ stark von der Umgebungsfeuchte Hum und wenigstens etwas von der Umgebungstemperatur Temp abhängt.

Um die Gesamt-Detektionsgröße Det für den Betrieb im Modus P festzulegen, sind x Werte par(1), ..., par(x) für die x Parameter Par(1), ..., Par(x) der Funktion $F_P$ festzulegen. Jeder Satz par(1), ..., par(x) von Parameterwerten führt zu jeweils einer empirischen Varianz Var = Var[par(1), ..., par(x)] der resultierenden ersten Detektionsgrößen-Stichprobe. Die Parameterwerte par(1), ..., par(x) werden so festgelegt, dass sie zu einer minimalen empirischen Varianz Var in der ersten Detektionsgrößen-Stichprobe führen. Um die Parameterwerte par(1), ..., par(x) festzulegen, wird also eine Zielfunktion numerisch minimiert. Die Variablen dieser Zielfunktionen sind die x Parameter Par(1), ..., Par(x) der Funktion $F_P$. Die Zielfunktion ist ein Maß für die empirische Varianz Var der Gesamt-Detektionsgröße Det = $F_P$[U10, U11, U($\Delta$Temp)].

Dieses Vorgehen wird beispielhaft für die bevorzugte Ausgestaltung erläutert, dass die Berechnungsvorschrift (3) verwendet wird. Jedes Tripel von Werten für die drei Parameter $\alpha_P$, $\beta_P$, $x0_P$ führt zu einem Wert var für die empirische Varianz Var der resultierenden ersten Detektionsgrößen-Stichprobe. Es gilt

$$(9) \qquad det(x_{i,j}) = u10(x_{i,j}) - \alpha_P * u11(x_{i,j}) - \beta_P * u(\Delta Temp)(x_{i,j}) - x0_P$$

Die zu minimierende Zielfunktion ist also das Maß für die empirische Varianz Var der Gesamt-Detektionsgröße Det = U10 - $\alpha_P$*U11 - $\beta_P$*U($\Delta$Temp) - $x0_P$ als Funktion der drei Parameter $\alpha_P$, $\beta_P$, $_x0_P$.

Als Maß für die empirische Varianz Var lässt sich die Differenz zwischen dem größten Wert max {det($x_{i,j}$), wobei i=1, ..., M; j=1, ..., N} und dem kleinsten Wert min{det($x_{i,j}$), wobei i=1, ..., M; j=1, ..., N} für die Gesamt-Detektionsgröße Det verwenden. Möglich ist auch, folgende Rechenvorschrift zu verwenden:

$$(10) \qquad Var = \frac{1}{M*N-1} \sum_{i=1}^{M} \sum_{j=1}^{N} [det(x_{i,j}) - det_{avg}]^2$$

mit

$$(11) \quad det_{avg} = \frac{1}{M*N} \sum_{i=1}^{M} \sum_{j=1}^{N} det(x_{i,j})$$

Das nachfolgend beschriebene zweistufige Verfahren reduziert den Rechenaufwand deutlich. In vielen Fällen führt es zu einem ähnlich guten Ergebnis für den Modus P.

Die Funktion $F_P$ der Rechenvorschrift (1) wird vereinfacht und in zwei Funktionen aufgeteilt, nämlich

$$(12) \quad Det = F_{P,10,11}[U10,U11] - F_{P,Temp}[U(\Delta Temp)].$$

Die beiden Funktionen $F_{P,10,11}$ und $F_{P,Temp}$ hängen von jeweils mindestens einem Parameter ab. Eine Sonderform von (12) ist die Rechenvorschrift (3) mit

$$(13) \quad F_{P,10,11}[U10,U11] = U10 - \alpha_P*U11 - x0_P \text{ und}$$

$$F_{P,Temp}[U(\Delta Temp)] = \beta_P*U(\Delta Temp).$$

Zunächst wird für den oder jeden Parameter der Funktion $F_{P,10,11}$ jeweils ein Wert festgelegt. Bei der Ausgestaltung gemäß der Rechenvorschrift (3) wird jeweils ein Wert für die Parameter $\alpha_P$ und $x0_P$ festgelegt. Hierfür wird eine reduzierte erste Detektionsgrößen-Stichprobe verwendet, in der die Messwerte des Temperatur-Sensors 14 fortgelassen sind. Jedes Stichprobenelement dieser reduzierten Detektionsgrößen-Stichprobe hat also die Form

$$(14) \quad \{ [det(x_{i,j}); [u10(x_{i,j}), u11(x_{i,j})] \}$$

Mithilfe dieser reduzierten ersten Detektionsgrößen-Stichprobe wird der jeweilige Wert für den oder jeden Parameter der Funktion $F_{P,10,11}$ so festgelegt, dass das Maß für die empirische Varianz Var minimiert wird.

Nunmehr ist die Funktion $F_{P,10,11}$ festgelegt. Diese Funktion $F_{P,10,11}$ wird auf die reduzierte Detektionsgrößen-Stichprobe angewendet. Genauer gesagt: Die Funktion $F_{P,10,11}$ wird auf die jeweiligen beiden Werte $u10(x_{i,j})$, $u11(x_{i,j})$ in jedem Stichprobenelement der Form (8), nämlich

$$(8) \quad \{ det(x_{i,j}); [u10(x_{i,j}), u11(x_{i,j}), u(\Delta Temp)(x_{i,j})] \}$$

angewendet. Mit der Bezeichnung $det_{P,10,11}(x_{i,j}) = F_{P,10,11} [u10(x_{i,j}), u11(x_{i,j})]$ wird eine reduzierte zweite Detektionsgrößen-Stichprobe erzeugt, in der jedes Stichprobenelement die Form

$$(15) \quad \{ det(x_{i,j}); [det_{P,10,11}(x_{i,j}), u(\Delta Temp)(x_{i,j})] \}$$

aufweist. Mithilfe dieser Stichprobe wird jeweils ein Wert für den oder jeden Parameter der Funktion $F_{P,Temp}$ festgelegt. Im Falle der Berechnungsvorschrift (3) ist dies ein Wert für den einzigen Parameter $\beta_P$. Der oder jeder Wert wird wieder so festgelegt, dass das Maß für die Varianz Var minimiert wird.

Bei einer weiteren Vereinfachung wird bei typischen Umgebungsbedingungen und abhängig von einem Verstärkungsfaktor $\alpha$ der Nullwert $x0_P$ bestimmt. Die Umgebungsbedingungen sind beispielsweise 20 °C, 1000 mbar und 0% relative Feuchte. Die beiden gerade beschriebenen Detektionsgrößen-Stichproben werden dafür verwendet, um zunächst den Verstärkungsfaktor $\alpha_P$ für die Kompensator-Spannung U11 und anschließend den Verstärkungsfaktor $\beta_P$ für die gemessene Umgebungstemperatur $U(\Delta Temp)$ zu berechnen. Hierbei wird der vorab verwendete Nullwert x0 verwendet, ohne ihn zu verändern.

Verschiedene Varianten dieses Vorgehens sind möglich.

Wie oben beschrieben, wird aus der ersten Messwerte-Stichprobe eine erste Detektionsgrößen-Stichprobe hergeleitet, wobei die erste Messwerte-Stichprobe die Form (6) und die erste Detektionsgrößen-Stichprobe die Form (7) hat und jede Stichprobe jeweils M*N Stichprobenelemente aufweist. Möglich ist auch, aus der ersten Messwerte-Stichprobe eine erste Konzentrations-Stichprobe mit M*N Stichprobenelementen herzuleiten, wobei jedes Stichprobenelement die Form

$$(16) \quad con(x_{i,j}) = F_{Con} \{ F_P[u10(x_{i,j}), u11(x_{i,j}), u(\Delta Temp)(x_{i,j})] \}$$

hat (i=1, ..., M; j=1, ..., N).

In einer Ausgestaltung wird zusätzlich zur ersten eine zweite Messwerte-Stichprobe ermittelt. Genau wie bei der ersten Messwerte-Stichprobe nimmt die Umgebungsfeuchte Hum den gleichbleibenden Wert $hum_0$ an, der Umgebungs-druck P M verschiedene Werte, die Umgebungstemperatur Temp N verschiedene Werte. Im Gegensatz zur ersten Messwerte-Stichprobe ist jedoch brennbares Zielgas in der Umgebung vorhanden. Die Funktion, welche empirisch mit Hilfe der ersten Messwerte-Stichprobe hergeleitet wird, wird mit $F_{P,0}$ bezeichnet. Mit Hilfe der zweiten Messwerte-Stichprobe wird empirisch eine Funktion $F_{P,con}$ hergeleitet. Die angewendete Funktion F wird durch eine geeignete Mittelung aus den beiden Funktionen $F_{P,0}$ und $F_{P,con}$ hergeleitet.

Nunmehr wird die Konfiguration für den Modus P_Hum beschrieben. Der Messwert $con_{meas}$ für die Zielgas-Konzentration $Con_{meas}$ soll abhängig von der Umgebungsfeuchte Hum dann um höchstens x % variieren, wenn die Umgebungsfeuchte Hum in einem vorgegebenen Feuchtebereich bleibt.

Wiederum wird die erste Messwerte-Stichprobe verwendet, also eine Stichprobe mit Stichprobenelementen, wobei jedes Stichprobenelement die Form (6) hat, also

$$(6) \quad \{[u10(x_i), u11(x_i), u(\Delta Temp)(x_i)]; [\Delta temp(i), p(i), hum(1)]\}$$

aufweist. Bei der ersten Messwerte-Stichprobe liegt durchgehend die gleiche Umgebungsfeuchte $hum(x_1)$ vor.

Zusätzlich werden K-1 weitere Messwerte-Stichproben erzeugt, K >=2. Bei jeder zweiten Stichprobe wird durchgehend jeweils ein Wert hum(2), ..., hum(K) für die Umgebungsfeuchte Hum eingestellt, wobei die insgesamt K Werte hum(1), ..., hum(K) alle voneinander verschieden sind. Die erste Stichprobe wurde bei M*N Bedingungen $x_{i,j,1} = x_{i,j}$ (i=1, ..., M*N) erzeugt, wobei die Umgebungsbedingungen con(1), p(i), $\Delta temp(j)$, hum(1) vorlagen (i=1, ..., M; j=1, ..., N). Bei jeder weiteren Stichprobe liegen die M*N Bedingungen $x_{i,j,k}$ (i=1, ..., M; j=1, ..., N; k=2, ..., K) vor.

Insgesamt liegen K Stichproben mit jeweils M*N Stichprobenelementen vor, wobei die Stichprobe Nr. k die Form

$$(17) \quad \{[u10(x_{i,j,k}), u11(x_{i,j,k}), u(\Delta Temp)(x_{i,j,k})]; [p(i), \Delta temp(j), hum(k)]\}$$

aufweist (i=1, ..., M; j=1, ..., N; k=1, ..., K).

Im Modus P_Hum wendet die Auswerteeinheit 9 die Berechnungsvorschrift

$$(18) \quad Det = F_{P\_Hum}[U10, U11, U(\Delta Temp)]$$

an. Bevorzugt hat die Berechnungsvorschrift (18) die Form

$$(19) \quad Det = U10 - \alpha_{P\_Hum}*U11 - \beta_{P\_Hum}*U(\Delta Temp) - x0_{P\_Hum}.$$

Für die x Parameter Par(1), ..., Par(x) der Funktion $F_{P\_Hum}$ sind Werte zu berechnen.

Weiter oben wurde mit Bezug auf die Berechnungsvorschrift (7) die Herleitung einer ersten Detektionsgrößen-Stichprobe beschrieben. Dieses Verfahren wird abgewandelt. Entsprechend werden K Detektionsgrößen-Stichproben hergeleitet. Jede Detektionsgrößen-Stichproben hat jeweils M*N Stichprobenelemente, wobei jedes Stichprobenelement die Form

$$(20) \quad det(x_{i,j,k}) = F_{P\_Hum}[u10(x_{i,j,k}), u11(x_{i,j,k}), u(\Delta Temp)(x_{i,j,k})]$$

aufweist.

Die oben erwähnte Randbedingung, dass der Messwert con für die Zielgas-Konzentration Con abhängig von der Umgebungsfeuchte Hum um höchstens x % variieren soll, führt zu einer Randbedingung für die Gesamt-Detektionsgröße Det. Diese soll in Abhängigkeit von der Umgebungsfeuchte Hum um höchstens y % variieren. Der Faktor y hängt von dem vorgegebenen funktionalen Zusammenhang zwischen der Zielgas-Konzentration Con und der Gesamt-Detektionsgröße Det ab, wobei dieser Zusammenhang durch die Rechenvorschrift (4), insbesondere durch die Rechenvorschrift (5), beschrieben wird. Beispielsweise soll die Gesamt-Detektionsgröße Det bei schwankender Umgebungsfeuchte Hum in einem Toleranzband der Breite y % liegen. Dies führt z.B. zu der Anforderung

$$(21) \quad (1-y)*det_{avg} <= det(x_{i,j,k}) <= (1+y)*det_{avg}$$

für alle i=1, ..., M; j=1, ..., N; k=1, ..., K),

wobei $det_{avg}$ der Mittelwert aller Stichprobenelemente der Detektionsgrößen-Stichprobe(n) ist.

Die Parameterwerte par(1), ..., par(x) für die Funktion $F_{P\_Hum}$ der Rechenvorschrift (18) werden so festgelegt, dass die Randbedingung (21) eingehalten wird.

## **Bezugszeichenliste**

| | |
|---|---|
| 100 | Gasmessvorrichtung, umfasst den Detektor 10, den Kompensator 11, den Temperatur-Sensor 14, das Steuergerät 6 und den Datenspeicher 7 sowie optional den Feuchte-Sensor 17 und den Druck-Sensor 18 |
| 2 | optionaler Flammschutz vor den Öffnungen Ö1, Ö2 |
| 5 | Kompensatorkammer, umgibt den Kompensator 11 |
| 6 | Steuergerät, empfängt Signale von den Sensoren 12.1, 12.2, 13.1, 13.2, 14 |
| 7 | Datenspeicher, in dem das Modell Mod abgespeichert ist |
| 7.1 | Datenspeicher der weiteren Gasmessvorrichtung 100.1, in dem das Modell Mod.1 abgespeichert ist |
| 8 | Detektorkammer, umgibt den Detektor 10 |
| 9 | Auswerteeinheit, gehört zum Steuergerät 6, leitet die Zielgas-Konzentration $Con_{meas}$ her |
| 10 | Detektor, umfasst das heizende Segment 20 |
| 11 | Kompensator, umfasst das heizende Segment 38 |
| 12.1 | Spannungs-Sensor, misst die Spannung U10 |
| 12.2 | Spannungs-Sensor, misst die Spannung U11 |
| 13.1 | Stromstärken-Sensor, misst die Stromstärke I.1 |
| 13.2 | Stromstärken-Sensor, misst die Stromstärke I.2 |
| 14 | Temperatur-Sensor, misst die Differenz $\Delta$Temp zwischen der Umgebungstemperatur und einer Referenztemperatur |
| 16 | Auswahleinheit in Form eines Schalters, mit dem ein Benutzer einen Modus auswählen kann |
| 17 | Feuchte-Sensor der Gasmessvorrichtung 100, misst die Umgebungsfeuchte Hum |
| 18 | Druck-Sensor der Gasmessvorrichtung 100, misst die Druckdifferenz $\Delta$P |
| 20 | heizendes Segment des Detektors 10 |
| 24 | elektrische Kontaktierungen für das heizende Detektor-Segment 20 |
| 25 | Keramikummantelung um das heizende Detektor-Segment 20 |
| 26 | katalytische Beschichtung auf der Keramikummantelung 25 |
| 27 | Montageplatte des Detektors 10 |
| 30 | elektrisch leitendes Bauteil in Form einer Leiterbahn des als flaches Bauteil ausgestalteten Detektors 10 |
| 31 | Trägerplatte für das Bauteil 30 |
| 32 | heizendes Segment, gehört zum Bauteil 30 |
| 33 | Wafer-Substrat, trägt die Trägerplatte 31 |
| 34 | elektrische Kontaktstellen 34 für das Bauteil 30 |
| 35 | Schutzschicht auf dem Bauteil 30 |
| 38 | heizendes Segment des Kompensators 11 |

(fortgesetzt)

| 43, 44 | Spannungsquellen |
|---|---|
| 46 | elektrische Kontaktierung für das Bauteil 30 |
| $50_{Hum}$ | Abhängigkeit des Messfehlers $Con_{meas}$ - Con vom Verstärkungsfaktor $\alpha$ in der Testumgebung $Cond_{Hum}$ |
| $50_P$ | Abhängigkeit des Messfehlers $Con_{meas}$ - Con vom Verstärkungsfaktor $\alpha$ in der Testumgebung $Cond_P$ |
| 100 | Gasmessvorrichtung |
| 100.1 | weitere Gasmessvorrichtung |
| 110 | Kalibriervorrichtung, erzeugt die Modelle Mod und Mod.1, umfasst den Feuchte-Sensor 117 und den Druck-Sensor 118, umfasst die Bestandteile 110.1 und 110.2 |
| 117 | Feuchte-Sensor der Kalibriervorrichtung 110 |
| 118 | Druck-Sensor der Kalibriervorrichtung 110 |
| $\alpha$ | Verstärkungsfaktor für die Kompensator-Spannung U11 in der Gesamt-Detektionsgröße Det |
| $\alpha_{Hum}$ | Verstärkungsfaktor für den feuchteoptimierten Modus |
| $\alpha_{Hum\_P}$ | Verstärkungsfaktor für den feuchtekompensierenden Modus |
| $\alpha_P$ | Verstärkungsfaktor für den druckoptimierten Modus |
| $\alpha_{P\_Hum}$ | Verstärkungsfaktor für den druckkompensierenden Modus |
| $\beta$ | Verstärkungsfaktor für das Signal U($\Delta$Temp) in der Gesamt-Detektionsgröße Det |
| B | räumlicher Bereich, der auf das Vorhandensein eines brennbaren Zielgases überwacht werden soll |
| Con | tatsächliche Zielgas-Konzentration |
| $Con_{meas}$ | von der Gasmessvorrichtung 100 hergeleitete Zielgas-Konzentration |
| $Cond_{Hum}$ | Testumgebung mit einer Umgebungsfeuchte Hum von 90 % relativer Feuchtigkeit |
| $Cond_P$ | Testumgebung mit einem um 200 mbar erhöhten Umgebungsdruck P |
| $Cond_{Ref}$ | Referenz-Testumgebung mit einer Umgebungsfeuchte hum von 0 % relativer Feuchtigkeit und einer Zielgas-Konzentration con von 0 % LEL |
| Det | Gesamt-Detektionsgröße, hängt von U10, U11 und U($\Delta$Temp) ab, optional zusätzlich von U(Hum) und U($\Delta$P) |
| Gp | Gasprobe aus dem Bereich B |
| Hum | Umgebungsfeuchte, zugleich feuchteoptimierter Modus |
| Hum_P | feuchtekompensierender Modus |
| I.1 | Stromstärke des durch den Detektor 10 fließenden Stroms |
| I.2 | Stromstärke des durch den Kompensator 11 fließenden Stroms |
| Mod | rechnerauswertbares Modell mit funktionalen Zusammenhängen zwischen den Detektionsgrößen U10 und U11, dem Signal U($\Delta$Temp) und der Zielgas-Konzentration $Con_{meas}$, wird von der Auswerteeinheit 9 angewendet |
| Mod. 1 | rechnerauswertbares Modell, welches die Auswerteeinheit der weiteren Gasmessvorrichtung 100.1 anwendet, im Datenspeicher 7.1 abgespeichert |
| Ö1 | Öffnung der Detektorkammer 8 |
| Ö2 | Öffnung der Kompensatorkammer 5 |
| P | Umgebungsdruck, zugleich druckoptimierter Modus |
| P_Hum | druckkompensierender Modus |
| $\Delta$P | Differenz zwischen dem Umgebungsdruck P und einem vorgegebenen Referenzdruck, vom Druck-Sensor 18 gemessen |
| Temp | Umgebungstemperatur |

(fortgesetzt)

| $\Delta$Temp | Differenz zwischen der aktuellen Umgebungstemperatur Temp und einer vorgegebenen Referenztemperatur, vom Temperatur-Sensor 14 gemessen |
|---|---|
| U10 | elektrische Spannung, die am Detektor 10 anliegt |
| $Tol_{Hum}$ | Toleranzband für die Abhängigkeit von der Umgebungsfeuchte Hum |
| $Tol_P$ | Toleranzband für die Abhängigkeit vom Umgebungsdruck P |
| $Tol_{Temp}$ | Toleranzband für die Abhängigkeit von der Umgebungstemperatur Temp |
| U11 | elektrische Spannung, die am Kompensator 11 anliegt |
| U($\Delta$Temp) | vom Temperatur-Sensor 14 geliefertes Signal, korreliert mit der Temperaturdifferenz $\Delta$Temp |
| x0 | Konstante in der Gesamt-Detektionsgröße Det |

## Patentansprüche

1. Gasmessvorrichtung (100),

welche dazu ausgestaltet ist, die Konzentration mindestens eines brennbaren Zielgases ($CH_4$) in einem räumlichen Bereich (B) zu messen,
wobei die Gasmessvorrichtung (100)

- einen Detektor (10),
- einen Kompensator (11),
- einen Detektor-Detektionsgrößen-Sensor (12.1),
- einen Kompensator-Detektionsgrößen-Sensor (12.2) und
- eine signalverarbeitende Auswerteeinheit (9)

umfasst,
wobei die Gasmessvorrichtung (100) so ausgestaltet ist, dass wenigstens zeitweise eine Gasprobe (Gp) aus dem räumlichen Bereich (B) den Detektor (10) und den Kompensator (11) erreicht,
wobei der Detektor (10) eine Detektor-Detektionsgröße (U10) aufweist, die mit der Konzentration des Zielgases ($CH_4$) in der Gasprobe (Gp) korreliert,
wobei der Kompensator (11) eine Kompensator-Detektionsgröße (U11) aufweist, die weniger als die Detektor-Detektionsgröße (U10) oder sogar überhaupt nicht mit der Zielgas-Konzentration korreliert,
wobei die Detektor-Detektionsgröße (U10) und die Kompensator-Detektionsgröße (U11) durch mindestens eine auf die Gasprobe (Gp) wirkende Umgebungsbedingung beeinflusst werden oder wenigstens beeinflussbar sind,
wobei der Detektor-Detektionsgrößen-Sensor (12.1) dazu ausgestaltet ist, die Detektor-Detektionsgröße (U10) zu messen,
wobei der Kompensator-Detektionsgrößen-Sensor (12.2) dazu ausgestaltet ist, die Kompensator-Detektionsgröße (U11) zu messen,
wobei die Auswerteeinheit (9) dazu ausgestaltet ist, abhängig von der gemessenen Detektor-Detektionsgröße (U10) und von der gemessenen Kompensator-Detektionsgröße (U11) die Konzentration des Zielgases ($CH_4$) in der Gasprobe (Gp) zu ermitteln, und
wobei die Gasmessvorrichtung (100) während eines Einsatzes in mindestens einem von mindestens zwei verschiedenen Modi betreibbar ist,
**dadurch gekennzeichnet, dass**
ein erster Modus ein druckkompensierender Modus und ein zweiter Modus ein feuchtekompensierender Modus ist,
wobei im druckkompensierenden Modus der Einfluss des Umgebungsdrucks (P) auf ein Ermittlungs-Ergebnis der Auswerteeinheit (9) dergestalt kompensiert ist, dass

- die Randbedingung eingehalten wird, dass der Einfluss der Umgebungsfeuchte (Hum) auf das Ermittlungs-Ergebnis unterhalb einer vorgegebenen oberen Feuchteeinfluss-Schranke bleibt, und
- der Einfluss des Umgebungsdrucks (P) auf das Ermittlungs-Ergebnis unter dieser Randbedingung kompensiert ist,

wobei im feuchtekompensierenden Modus der Einfluss der Umgebungsfeuchte (Hum) auf das Ermittlungs-Ergebnis dergestalt kompensiert ist, dass

- die Randbedingung eingehalten wird, dass der Einfluss des Umgebungsdrucks (P) auf das Ermittlungs-Ergebnis unterhalb einer vorgegebenen oberen Druckeinfluss-Schranke bleibt, und
- der Einfluss der Umgebungsfeuchte (Hum) auf das Ermittlungs-Ergebnis unter dieser Randbedingung kompensiert ist, und

wobei die Auswerteeinheit (9) so ausgestaltet ist, dass im druckkompensierenden Modus die Abhängigkeit der ermittelten Zielgas-Konzentration von der Detektor-Detektionsgröße (U10) und / oder von der Kompensator-Detektionsgröße (U11) anders ist als im feuchtekompensierenden Modus.

2. Gasmessvorrichtung (100) nach Anspruch 1,
   **dadurch gekennzeichnet, dass**

   die Gasmessvorrichtung (100) zusätzlich in einem druckoptimierten Modus und / oder in einem feuchteoptimierten Modus betreibbar ist,
   wobei im druckoptimierten Modus der Einfluss des Umgebungsdrucks (P) auf das Ermittlungs-Ergebnis der Auswerteeinheit (9) ohne Einhaltung einer Randbedingung kompensiert ist,
   wobei im feuchteoptimierten Modus der Einfluss der Umgebungsfeuchte (Hum) auf das Ermittlungs-Ergebnis ohne Einhaltung einer Randbedingung kompensiert ist und
   wobei die Auswerteeinheit (9) so ausgestaltet ist, dass in jedem Modus die Abhängigkeit der ermittelten Zielgas-Konzentration von der Detektor-Detektionsgröße (U10) und / oder von der Kompensator-Detektionsgröße (U11) anders ist als in jedem anderen Modus.

3. Gasmessvorrichtung (100) nach einem der vorhergehenden Ansprüche,
   **dadurch gekennzeichnet, dass**

   die Gasmessvorrichtung (100) wahlweise im druckkompensierenden Modus oder im feuchtekompensierenden Modus betreibbar ist und
   optional zusätzlich wahlweise im druckoptimierten Modus oder im feuchteoptimierten Modus betreibbar ist.

4. Gasmessvorrichtung (100) nach Anspruch 3,
   **dadurch gekennzeichnet, dass**

   die Gasmessvorrichtung (100) eine Auswahleinheit (16) umfasst,
   wobei die Auswahleinheit (16) dazu ausgestaltet ist, eine Vorgabe eines Benutzers oder einer übergeordneten Steuerung zu erfassen,
   wobei die erfasste Vorgabe einen Modus festlegt, in dem die Gasmessvorrichtung (100) betrieben werden soll,
   wobei die Gasmessvorrichtung (100) dazu ausgestaltet ist, abhängig von einer Betätigung der Auswahleinheit (16) im druckkompensierenden Modus oder im feuchtekompensierenden Modus betrieben zu werden, und
   wobei die Gasmessvorrichtung (100) optional zusätzlich dazu ausgestaltet ist, abhängig von einer Betätigung der Auswahleinheit (16) im druckoptimierten Modus oder im feuchteoptimierten Modus betrieben zu werden.

5. Gasmessvorrichtung (100) nach Anspruch 3 oder Anspruch 4,
   **dadurch gekennzeichnet, dass**
   die Gasmessvorrichtung (100) dazu ausgestaltet ist,

   - in mindestens zwei verschiedenen Modi jeweils einen Schätzwert für die Zielgas-Konzentration in derselben Gasprobe (Gp) zu ermitteln und
   - einen Alarm zu erzeugen, wenn mindestens ein ermittelter Schätzwert außerhalb eines vorgegebenen Wertebereichs für die Zielgas-Konzentration liegt.

6. Gasmessvorrichtung (100) nach einem der vorhergehenden Ansprüche,
   **dadurch gekennzeichnet, dass**

   die Gasmessvorrichtung (100) zusätzlich mindestens einen Sensor (14, 17, 18) für eine Umgebungsbedingung umfasst,

wobei der oder jeder Sensor (14, 17, 18) für eine Umgebungsbedingung dazu ausgestaltet ist, die jeweilige Umgebungsbedingung zu messen,

wobei bevorzugt der oder ein Sensor (14) für eine Umgebungsbedingung ein Temperatur-Sensor ist, der dazu ausgestaltet ist, die Umgebungstemperatur zu messen, und

wobei die Auswerteeinheit (9) dazu ausgestaltet ist, die Konzentration des Zielgases ($CH_4$) in der Gasprobe (Gp) zusätzlich abhängig von dem jeweiligen Signal des oder mindestens eines Sensors (14, 17, 18) für eine Umgebungsbedingung zu ermitteln.

7. Gasmessvorrichtung (100) nach Anspruch 6,
**dadurch gekennzeichnet, dass**

der oder mindestens ein Sensor für eine Umgebungsbedingung,
insbesondere ein Sensor (17) für die Umgebungsfeuchte oder ein Sensor (18) für den Umgebungsdruck,
wahlweise aktiviert oder deaktiviert ist,
wobei die Gasmessvorrichtung (100) so ausgestaltet ist, dass
die Auswerteeinheit (9) bei aktiviertem Sensor (17, 18) für eine Umgebungsbedingung die Konzentration des Zielgases ($CH_4$) in der Gasprobe (Gp) zusätzlich abhängig von dem Signal des aktivierten Sensors (17, 18) ermittelt und
die Gasmessvorrichtung (100) mindestens bei deaktiviertem Sensor (17, 18) für eine Umgebungsbedingung im druckkompensierenden Modus und / oder im feuchtekompensierenden Modus betreibbar ist.

8. Gasmessvorrichtung (100) nach einem der vorhergehenden Ansprüche,
**dadurch gekennzeichnet, dass**

der Detektor (10) ein erhitzbares Detektor-Segment (20) umfasst und
der Kompensator (11) ein erhitzbares Kompensator-Segment (38) umfasst,
wobei die Gasmessvorrichtung (100) dazu ausgestaltet ist, das Detektor-Segment (20) so zu erhitzen, dass das erhitzte Detektor-Segment (20) brennbares Zielgas ($CH_4$) in der Gasprobe (Gp) oxidiert und die Oxidation das Detektor-Segment (20) weiter erhitzt,
wobei die Gasmessvorrichtung (100) dazu ausgestaltet ist, das Kompensator-Segment (38) zu erhitzen,
wobei die Gasmessvorrichtung (100)

- in einer ersten Alternative so ausgestaltet ist, dass das erhitzte Kompensator-Segment (38) pro Zeiteinheit weniger brennbares Zielgas als das erhitzte Detektor-Segment (20) oxidiert und
in einer zweiten Alternative so ausgestaltet ist, dass pro Zeiteinheit eine geringere Menge der Gasprobe den Kompensator (11) als den Detektor (10) erreicht, und

wobei der Detektor-Detektionsgrößen-Sensor (12.1) dazu ausgestaltet ist, als Detektor-Detektionsgröße ein Maß (U10) für die Temperatur des Detektor-Segments (20) zu messen, und
wobei der Kompensator-Detektionsgrößen-Sensor (12.2) dazu ausgestaltet ist, als Kompensator-Detektionsgröße ein Maß (U11) für die Temperatur des Kompensator-Segments (38) zu messen.

9. Gasmessvorrichtung (100) nach einem der vorhergehenden Ansprüche,
**dadurch gekennzeichnet, dass**

die Auswerteeinheit (9) wenigstens zeitweise Lesezugriff auf ein rechnerauswertbares Modell (Mod) aufweist,
wobei das Modell (Mod) für den oder jeden Modus, in dem die Gasmessvorrichtung (100) betreibbar ist, jeweils einen funktionalen Zusammenhang umfasst,
wobei der funktionale Zusammenhang für einen Modus einen Zusammenhang zwischen

- der Zielgas-Konzentration einerseits und
- dem jeweiligen Signal jedes Detektionsgrößen-Sensors (12.1, 12.2) und

optional mindestens eines Signals eines Sensors (14, 17, 18) für eine Umgebungsbedingung, andererseits

beschreibt und
wobei die Auswerteeinheit (9) dazu ausgestaltet ist, bei der Ermittlung der Zielgas-Konzentration

den funktionalen Zusammenhang für den Modus, in dem die Gasmessvorrichtung (100) aktuell betrieben wird, auf das jeweilige Signal jedes Detektionsgrößen-Sensors (12.1, 12.2) und optional auf das jeweilige Signal jedes optionalen Sensors (14, 17, 18) für eine Umgebungsbedingung anzuwenden.

10. Anordnung umfassend

- eine erste Gasmessvorrichtung (100) nach einem der vorhergehenden Ansprüche und
- eine zweite Gasmessvorrichtung (100.1) nach einem der vorhergehenden Ansprüche,
wobei die Auswerteeinheit (9) der ersten Gasmessvorrichtung (100) wenigstens zeitweise Lesezugriff auf ein erstes rechnerauswertbares Modell (Mod) hat, welches eine erste Abhängigkeit der Zielgas-Konzentration mindestens von der Detektor-Detektionsgröße (U10) und von der Kompensator-Detektionsgröße (U11) und optional von mindestens einer Umgebungsbedingung für einen Betrieb im druckkompensierenden Modus beschreibt, und
wobei die Auswerteeinheit (9.1) der zweiten Gasmessvorrichtung (100.1) wenigstens zeitweise Lesezugriff auf ein zweites rechnerauswertbares Modell (Mod.1) hat, welches eine zweite Abhängigkeit der Zielgas-Konzentration mindestens von der Detektor-Detektionsgröße (U10) und von der Kompensator-Detektionsgröße (U11) und optional von mindestens einer Umgebungsbedingung für einen Betrieb im feuchtekompensierenden Modus beschreibt.

11. Kalibriervorrichtung (110) zum Kalibrieren einer Gasmessvorrichtung (100) gemäß Anspruch 9,

wobei die Kalibriervorrichtung (110) dazu ausgestaltet ist,
eine Vorgabe zu erfassen, wobei die erfasste Vorgabe mindestens einen Modus spezifiziert, in dem die Gasmessvorrichtung (100) betreibbar sein soll,
ein rechnerauswertbares Modell (Mod) dergestalt zu generieren, dass das generierte Modell (Mod)

- für den oder jeden erfassten Modus jeweils einen funktionalen Zusammenhang umfasst und
- von der Auswerteeinheit (9) der Gasmessvorrichtung (100) anwendbar ist,

wobei die Kalibriervorrichtung (110) dazu ausgestaltet ist, für die Generierung des Modells eine vorgegebene Stichprobe und eine Menge von vorgegebenen möglichen funktionalen Zusammenhängen zu verwenden,
wobei die Stichprobe mehrere Stichprobenelemente umfasst,
wobei jedes Stichprobenelement der Stichprobe

- eine Kennzeichnung einer Umgebungsbedingungen-Zielgas-Kombination,
nämlich eine Kombination einer Umgebungstemperatur, eines Umgebungsdrucks, einer Umgebungsfeuchte und einer tatsächlichen Zielgas-Konzentration und
- eine Signalwerte-Kombination,
die für das jeweilige Signal jedes Detektionsgrößen-Sensors (12.1, 12.2) jeweils einen bei dieser Umgebungsbedingungen-Zielgas-Kombination gemessenen Wert aufweist,

umfasst,
wobei die Kalibriervorrichtung (110) dazu ausgestaltet ist, für jeden Modus, für jeden vorgegebenen möglichen funktionalen Zusammenhang und für jedes Stichprobenelement jeweils die Schritte durchzuführen,

- den funktionalen Zusammenhang auf die Signalwerte-Kombination des Stichprobenelements anzuwenden und dadurch einen Wert für die Zielgas-Konzentration zu berechnen und
- den berechneten Wert für die Zielgas-Konzentration mit dem tatsächlichen Wert der Zielgas-Konzentration in diesem Stichprobenelement zu vergleichen, und

wobei die Kalibriervorrichtung (110) dazu ausgestaltet ist, für jeden Modus

- unter Verwendung der Vergleichsergebnisse einen möglichen funktionalen Zusammenhang auszuwählen und
- zu bewirken, dass der ausgewählte funktionale Zusammenhang als der in diesem Modus anzuwendende funktionale Zusammenhang von der Auswerteeinheit (9) verwendet wird.

12. Gasmessverfahren zum Messen der Konzentration eines brennbaren Zielgases ($CH_4$) in einem räumlichen Bereich (B)

unter Verwendung einer Gasmessvorrichtung (100), die

- einen Detektor (10),
- einen Kompensator (11),
- einen Detektor-Detektionsgrößen-Sensor (12.1) und
- einen Kompensator-Detektionsgrößen-Sensor (12.2)

umfasst,
wobei der Detektor (10) eine Detektor-Detektionsgröße (U10) aufweist, die mit der Konzentration des Zielgases (CH$_4$) in einer Gasprobe (Gp) korreliert,
wobei der Kompensator (11) eine Kompensator-Detektionsgröße (U11) aufweist, die weniger als die Detektor-Detektionsgröße (U10) oder sogar überhaupt nicht mit der Zielgas-Konzentration korreliert,
wobei die Detektor-Detektionsgröße (U10) und die Kompensator-Detektionsgröße (U11) durch mindestens eine auf die Gasprobe (Gp) wirkende Umgebungsbedingung beeinflusst werden oder wenigstens beeinflussbar sind,
wobei das Gasmessverfahren die Schritte umfasst, dass

- bewirkt wird, dass wenigstens zeitweise eine Gasprobe (Gp) aus dem Bereich den Detektor (10) und den Kompensator (11) erreicht,
- der Detektor-Detektionsgrößen-Sensor (12.1) die Detektor-Detektionsgröße (U10) misst,
- der Kompensator-Detektionsgrößen-Sensor (12.2) die Kompensator-Detektionsgröße (U11) misst und
- abhängig von der gemessenen Detektor-Detektionsgröße (U10) und der gemessenen Kompensator-Detektionsgröße (U11) die Konzentration des Zielgases (CH$_4$) in der Gasprobe (Gp) ermittelt wird,

**dadurch gekennzeichnet, dass**
die Gasmessvorrichtung (100) bei der Durchführung des Gasmessverfahrens in einem druckkompensierenden Modus und / oder in einem feuchtekompensierenden Modus betrieben wird,
wobei im druckkompensierenden Modus der Einfluss des Umgebungsdrucks (P) auf ein Ermittlungs-Ergebnis dergestalt kompensiert wird, dass

- die Randbedingung eingehalten wird, dass der Einfluss der Umgebungsfeuchte (Hum) auf das Ermittlungs-Ergebnis unterhalb einer vorgegebenen oberen Feuchteeinfluss-Schranke bleibt, und
- der Einfluss des Umgebungsdrucks (P) auf das Ermittlungs-Ergebnis unter dieser Randbedingung kompensiert wird, und

wobei im feuchtekompensierenden Modus der Einfluss der Umgebungsfeuchte (Hum) auf das Ermittlungs-Ergebnis dergestalt kompensiert wird, dass

- die Randbedingung eingehalten wird, dass der Einfluss des Umgebungsdrucks (P) auf das Ermittlungs-Ergebnis unterhalb einer vorgegebenen oberen Druckeinfluss-Schranke bleibt, und
- der Einfluss der Umgebungsfeuchte (Hum) auf das Ermittlungs-Ergebnis unter dieser Randbedingung kompensiert wird, und

wobei im druckkompensierenden Modus die Abhängigkeit der ermittelten Zielgas-Konzentration von der Detektor-Detektionsgröße (U10) und / oder von der Kompensator-Detektionsgröße (U11) anders ist als im feuchtekompensierenden Modus.

13. Kalibrierverfahren zum Kalibrieren einer Gasmessvorrichtung (100) gemäß Anspruch 9,

wobei eine Stichprobe und eine Menge von vorgegebenen möglichen funktionalen Zusammenhängen vorgegeben werden,
wobei die Stichprobe mehrere Stichprobenelemente umfasst,
wobei jedes Stichprobenelement der Stichprobe

- eine Kennzeichnung einer Umgebungsbedingungen-Zielgas-Kombination,
nämlich eine Kombination einer Umgebungstemperatur, eines Umgebungsdrucks, einer Umgebungsfeuchte und einer tatsächlichen Zielgas-Konzentration, und

- eine Signalwerte-Kombination,

nämlich für das jeweilige Signal jedes Detektionsgrößen-Sensors (12.1, 12.2) jeweils einen bei dieser Umgebungsbedingungen-Zielgas-Kombination gemessenen Wert,
optional zusätzlich mindestens einen Wert eines Sensors (14, 17, 18) für eine Umgebungsbedingung, wobei der Sensor (14, 17, 18) für eine Umgebungsbedingung diesen Wert bei dieser Umgebungsbedingungen-Zielgas-Kombination gemessen hat,

umfasst,
wobei das Kalibrierverfahren die Schritte umfasst, dass

- eine Vorgabe erfasst wird,
wobei die erfasste Vorgabe mindestens einen Modus spezifiziert, in dem die Gasmessvorrichtung (100) betreibbar sein soll, und
- ein rechnerauswertbares Modell (Mod) dergestalt generiert wird,
dass das generierte Modell (Mod) für den oder jeden erfassten Modus jeweils einen funktionalen Zusammenhang umfasst und von der Auswerteeinheit (9) der Gasmessvorrichtung (100) anwendbar ist, und

wobei das Kalibrierverfahren die Schritte umfasst, dass

- für jeden Modus und für jedes Stichprobenelement jeweils jeder vorgegebene mögliche funktionale Zusammenhang auf die Signalwerte-Kombination des Stichprobenelements angewendet wird,

dadurch ein Wert für die Zielgas-Konzentration berechnet wird und
der berechnete Wert für die Zielgas-Konzentration mit dem tatsächlichen Wert der Zielgas-Konzentration in diesem Stichprobenelement verglichen wird,

- für jeden Modus unter Verwendung der Vergleichsergebnisse ein möglicher funktionaler Zusammenhang ausgewählt wird und
bewirkt wird, dass der ausgewählte funktionale Zusammenhang als der in diesem Modus anzuwendende funktionale Zusammenhang von der Auswerteeinheit (9) verwendet wird.

**Claims**

1. Gas measuring device (100),

configured to measure the concentration of at least one flammable target gas ($CH_4$) in a spatial region (B), wherein the gas measuring device (100) comprises

- a detector (10),
- a compensator (11),
- a detector detection-variable sensor (12.1),
- a compensator detection-variable sensor (12.2), and
- a signal-processing evaluation unit (9),

wherein the gas measuring device (100) is configured such that at least temporarily a gas sample (Gp) from the spatial region (B) reaches the detector (10) and the compensator (11),
wherein the detector (10) has a detector detection variable (U10) correlating with the concentration of the target gas ($CH_4$) in the gas sample (Gp),
wherein the compensator (11) has a compensator detection variable (U11) correlating with the target-gas concentration to a lesser extent than the detector detection variable (U10) or not at all,
wherein the detector detection variable (U10) and the compensator detection variable (U11) are influenced or at least are influenceable by at least one ambient condition affecting the gas sample (Gp),
wherein the detector detection-variable sensor (12.1) is configured to measure the detector detection variable (U10),
wherein the compensator detection-variable sensor (12.2) is configured to measure the compensator detection variable (U11),

wherein the evaluation unit (9) is configured to ascertain the concentration of the target gas ($CH_4$) in the gas sample (Gp) depending on the measured detector detection variable (U10) and on the measured compensator detection variable (U11), and

wherein the gas measuring device (100) during use is operable in at least one of at least two different modes, **characterized in that**

a first mode is a pressure-compensating mode and a second mode is a humidity-compensating mode, wherein in the pressure-compensating mode, the influence of the ambient pressure (P) on an ascertaining result of the evaluation unit (9) is compensated for in such a way that

- the boundary condition that the influence of the ambient humidity (Hum) on the ascertaining result remains below a predetermined upper humidity influence limit is satisfied, and
- the influence of the ambient pressure (P) on the ascertaining result is compensated for under this boundary condition,

wherein in the humidity-compensating mode, the influence of the ambient humidity (Hum) on the ascertaining result is compensated for in such a way that

- the boundary condition that the influence of the ambient pressure (P) on the ascertaining result remains below a predetermined upper pressure influence limit is satisfied, and
- the influence of the ambient humidity (Hum) on the ascertaining result is compensated for under this boundary condition, and

wherein the evaluation unit (9) is configured such that in the pressure-compensating mode, the dependence of the ascertained target-gas concentration on the detector detection variable (U10) and/or on the compensator detection variable (U11) is different than in the humidity-compensating mode.

2. Gas measuring device (100) according to claim 1, **characterized in that**

the gas measuring device (100) is additionally operable in a pressure-optimized mode and/or in a humidity-optimized mode,

wherein in the pressure-optimized mode, the influence of the ambient pressure (P) on the ascertaining result of the evaluation unit (9) is compensated for without satisfying a boundary condition,

wherein in the humidity-optimized mode, the influence of the ambient humidity (Hum) on the ascertaining result is compensated for without satisfying a boundary condition, and

wherein the evaluation unit (9) is configured such that in each mode, the dependence of the ascertained target-gas concentration on the detector detection variable (U10) and/or on the compensator detection variable (U11) is different than in each other mode.

3. Gas measuring device (100) according to either of the preceding claims, **characterized in that**

the gas measuring device (100) is operable either in the pressure-compensating mode or in the humidity-compensating mode and

is optionally additionally operable either in the pressure-optimized mode or in the humidity-optimized mode.

4. Gas measuring device (100) according to claim 3, **characterized in that**

the gas measuring device (100) comprises a selection unit (16),

wherein the selection unit (16) is configured to record a specification from a user or a higher-level open-loop controller,

wherein the recorded specification defines a mode in which the gas measuring device (100) is to be operated,

wherein the gas measuring device (100) is configured to be operated in the pressure-compensating mode or in the humidity-compensating mode depending on an actuation of the selection unit (16), and

wherein the gas measuring device (100) is optionally additionally configured to be operated in the pressure-optimized mode or in the humidity-optimized mode depending on the actuation of the selection unit (16).

**5.** Gas measuring device (100) according to claim 3 or claim 4,
**characterized in that**
the gas measuring device (100) is configured

- to ascertain an estimated value for the target-gas concentration in the same gas sample (Gp) in each of at least two different modes, and
- to create an alarm if at least one ascertained estimated value is outside a predetermined value range for the target-gas concentration.

**6.** Gas measuring device (100) according to any of the preceding claims,
**characterized in that**

the gas measuring device (100) additionally comprises at least one sensor (14, 17, 18) for an ambient condition, wherein the or each sensor (14, 17, 18) for an ambient condition is configured to measure the relevant ambient condition,
wherein preferably the or a sensor (14) for an ambient condition is a temperature sensor configured to measure the ambient temperature, and
wherein the evaluation unit (9) is configured to ascertain the concentration of the target gas ($CH_4$) in the gas sample (Gp) additionally depending on the relevant signal from the or at least one sensor (14, 17, 18) for an ambient condition.

**7.** Gas measuring device (100) according to claim 6,
**characterized in that**

the or at least one sensor for an ambient condition,
in particular a sensor (17) for the ambient humidity or a sensor (18) for the ambient pressure,
is either enabled or disabled,
wherein the gas measuring device (100) is configured such that
the evaluation unit (9), when the sensor (17, 18) for an ambient condition is enabled, ascertains the concentration of the target gas ($CH_4$) in the gas sample (Gp) additionally depending on the signal from the enabled sensor (17, 18), and
the gas measuring device (100), at least when the sensor (17, 18) for an ambient condition is disabled, is operable in the pressure-compensating mode and/or in the humidity-compensating mode.

**8.** Gas measuring device (100) according to any of the preceding claims,
**characterized in that**

the detector (10) comprises a heatable detector segment (20), and
the compensator (11) comprises a heatable compensator segment (38),
wherein the gas measuring device (100) is configured to heat the detector segment (20) such that the heated detector segment (20) oxidizes flammable target gas ($CH_4$) in the gas sample (Gp) and the oxidation further heats the detector segment (20),
wherein the gas measuring device (100) is configured to heat the compensator segment (38),
wherein the gas measuring device (100)

- in a first alternative is configured such that the heated compensator segment (38) oxidizes less flammable target gas per unit of time than the heated detector segment (20), and

in a second alternative is configured such that a smaller quantity of the gas sample per unit of time reaches the compensator (11) than reaches the detector (10), and
wherein the detector detection-variable sensor (12.1) is configured to measure an indicator (U10) for the temperature of the detector segment (20) as the detector detection variable, and
wherein the compensator detection-variable sensor (12.2) is configured to measure an indicator (U11) for the temperature of the compensator segment (38) as the compensator detection variable.

**9.** Gas measuring device (100) according to any of the preceding claims,
**characterized in that**

the evaluation unit (9) at least temporarily has read access to a computer-evaluable model (Mod),
wherein the model (Mod) comprises a functional relationship for the or each mode in which the gas measuring device (100) is operable,
wherein the functional relationship for a mode describes a relationship between

- the target-gas concentration and
- the relevant signal from each detection-variable sensor (12.1, 12.2) and optionally at least one signal from a sensor (14, 17, 18) for an ambient condition,

and
wherein the evaluation unit (9) is configured to apply, when ascertaining the target-gas concentration, the functional relationship for the mode in which the gas measuring device (100) is currently being operated to the relevant signal from each detection-variable sensor (12.1, 12.2) and optionally to the relevant signal from each optional sensor (14, 17, 18) for an ambient condition.

10. Arrangement comprising

- a first gas measuring device (100) according to any of the preceding claims and
- a second gas measuring device (100.1) according to any of the preceding claims,

wherein the evaluation unit (9) of the first gas measuring device (100) at least temporarily has read access to a first computer-evaluable model (Mod) describing, for operation in the pressure-compensating mode, a first dependence of the target-gas concentration at least on the detector detection variable (U10) and on the compensator detection variable (U11) and optionally on at least one ambient condition, and
wherein the evaluation unit (9.1) of the second gas measuring device (100.1) at least temporarily has read access to a second computer-evaluable model (Mod.1) describing, for operation in the humidity-compensating mode, a second dependence of the target-gas concentration at least on the detector detection variable (U10) and on the compensator detection variable (U11) and optionally on at least one ambient condition.

11. Calibration device (110) for calibrating a gas measuring device (100) according to claim 9,

wherein the calibration device (110) is configured
to record a specification, wherein the recorded specification specifies at least one mode in which the gas measuring device (100) is to be operable,
to generate a computer-evaluable model (Mod) in such a way that the generated model (Mod)

- comprises a functional relationship for the or each recorded mode, and
- is applicable by the evaluation unit (9) of the gas measuring device (100),

wherein the calibration device (110) is configured to use, for generating the model, a predetermined sample and a set of predetermined possible functional relationships,
wherein the sample comprises a plurality of sample elements,
wherein each sample element of the sample comprises

- a designation of an ambient conditions/target gas combination,

namely a combination of an ambient temperature, an ambient pressure, an ambient humidity, and an actual target-gas concentration, and

- a signal value combination having, for the relevant signal from each detection-variable sensor (12.1, 12.2), a value measured for this ambient conditions/target gas combination,

wherein the calibration device (110) is configured to perform, for each mode, for each predetermined possible functional relationship, and for each sample element, the steps of

- applying the functional relationship to the signal value combination of the sample element and thereby calculating a value for the target-gas concentration, and
- comparing the calculated value for the target-gas concentration to the actual value of the target-gas concentration in this sample element, and

wherein the calibration device (110) is configured, for each mode,

- to select a possible functional relationship using the comparison results, and
- to cause the selected functional relationship to be used by the evaluation unit (9) as the functional relationship to be applied in this mode.

12. Gas measurement method for measuring the concentration of a flammable target gas ($CH_4$) in a spatial region (B)

using a gas measuring device (100) comprising

- a detector (10),
- a compensator (11),
- a detector detection-variable sensor (12.1), and
- a compensator detection-variable sensor (12.2),

wherein the detector (10) has a detector detection variable (U10) correlating with the concentration of the target gas ($CH_4$) in a gas sample (Gp),
wherein the compensator (11) has a compensator detection variable (U11) correlating with the target-gas concentration to a lesser extent than the detector detection variable (U10) or not at all,
wherein the detector detection variable (U10) and the compensator detection variable (U11) are influenced or at least are influenceable by at least one ambient condition affecting the gas sample (Gp),
wherein the gas measurement method comprises the steps of

- causing a gas sample (Gp) from the region, at least temporarily, to reach the detector (10) and the compensator (11),
- the detector detection-variable sensor (12.1) measuring the detector detection variable (U10),
- the compensator detection-variable sensor (12.2) measuring the compensator detection variable (U11), and
- ascertaining the concentration of the target gas ($CH_4$) in the gas sample (Gp) depending on the measured detector detection variable (U10) and the measured compensator detection variable (U11),

**characterized in that**
the gas measuring device (100), when performing the gas measurement method, is operated in a pressure-compensating mode and/or in a humidity-compensating mode,
wherein in the pressure-compensating mode, the influence of the ambient pressure (P) on an ascertaining result is compensated for in such a way that

- the boundary condition that the influence of the ambient humidity (Hum) on the ascertaining result remains below a predetermined upper humidity influence limit is satisfied, and
- the influence of the ambient pressure (P) on the ascertaining result is compensated for under this boundary condition, and

wherein in the humidity-compensating mode, the influence of the ambient humidity (Hum) on the ascertaining result is compensated for in such a way that

- the boundary condition that the influence of the ambient pressure (P) on the ascertaining result remains below a predetermined upper pressure influence limit is satisfied, and
- the influence of the ambient humidity (Hum) on the ascertaining result is compensated for under this boundary condition, and

wherein in the pressure-compensating mode, the dependence of the ascertained target-gas concentration on the detector detection variable (U10) and/or on the compensator detection variable (U11) is different than in the humidity-compensating mode.

13. Calibration method for calibrating a gas measuring device (100) according to claim 9,

wherein a sample and a set of predefined possible functional relationships are predetermined,
wherein the sample comprises a plurality of sample elements,
wherein each sample element of the sample comprises

- a designation of an ambient conditions/target gas combination,

namely a combination of an ambient temperature, an ambient pressure, an ambient humidity, and an actual target-gas concentration, and

- a signal value combination,

namely, for the relevant signal from each detection-variable sensor (12.1, 12.2), a value measured for this ambient conditions/target gas combination,
optionally additionally at least one value from a sensor (14, 17, 18) for an ambient condition, wherein the sensor (14, 17, 18) for an ambient condition has measured this value for this ambient conditions/target gas combination,
wherein the calibration method comprises the steps of

- recording a specification,

wherein the recorded specification specifies at least one mode in which the gas measuring device (100) is to be operable, and

- generating a computer-evaluable model (Mod) in such a way

that the generated model (Mod) comprises a functional relationship for the or each recorded mode and is applicable by the evaluation unit (9) of the gas measuring device (100), and
wherein the calibration method comprises the steps of

- applying, for each mode and for each sample element, each predetermined possible functional relationship to the signal value combination of the sample element,

thereby calculating a value for the target-gas concentration, and
comparing the calculated value for the target-gas concentration to the actual value of the target-gas concentration in this sample element,

- for each mode, selecting a possible functional relationship using the comparison results, and

causing the selected functional relationship to be used by the evaluation unit (9) as the functional relationship to be applied in this mode.

**Revendications**

1. Dispositif de mesure de gaz (100),

lequel est configuré pour mesurer la concentration d'au moins un gaz cible ($CH_4$) combustible dans une zone spatiale (B),
dans lequel le dispositif de mesure de gaz (100) comprend

- un détecteur (10),
- un compensateur (11),
- un capteur de grandeur de détection de détecteur (12.1),
- un capteur de grandeur de détection de compensateur (12.2) et
- une unité d'évaluation (9) traitant des signaux,

dans lequel le dispositif de mesure de gaz (100) est configuré de sorte qu'un échantillon de gaz (Gp) provenant de la zone spatiale (B) atteint au moins temporairement le détecteur (10) et le compensateur (11),
dans lequel le détecteur (10) présente une grandeur de détection de détecteur (U10) qui est en corrélation avec la concentration du gaz cible (CH$_4$) dans l'échantillon de gaz (Gp),

dans lequel le compensateur (11) présente une grandeur de détection de compensateur (U11) qui est moins corrélée à la concentration de gaz cible que la grandeur de détection de détecteur (U10), ou même pas du tout corrélée,

dans lequel la grandeur de détection de détecteur (U10) et la grandeur de détection de compensateur (U11) sont influencées ou peuvent au moins être influencées par au moins une condition ambiante agissant sur l'échantillon de gaz (Gp),

dans lequel le capteur de grandeur de détection de détecteur (12.1) est configuré pour mesurer la grandeur de détection de détecteur (U10),

dans lequel le capteur de grandeur de détection de compensateur (12.2) est configuré pour mesurer la grandeur de détection de compensateur (U11),

dans lequel l'unité d'évaluation (9) est configurée pour déterminer la concentration du gaz cible (CH$_4$) dans l'échantillon de gaz (Gp) en fonction de la grandeur de détection de détecteur (U10) mesurée et de la grandeur de détection de compensateur (U11) mesurée, et

dans lequel le dispositif de mesure de gaz (100) peut fonctionner dans au moins l'un parmi au moins deux modes différents pendant une utilisation,

**caractérisé en ce que**

un premier mode est un mode de compensation de la pression et un second mode est un mode de compensation de l'humidité,

dans lequel, dans le mode de compensation de la pression, l'influence de la pression ambiante (P) sur un résultat de détermination de l'unité d'évaluation (9) est compensée de telle sorte que

- la condition limite est respectée, selon laquelle l'influence de l'humidité ambiante (Hum) sur le résultat de détermination reste en dessous d'une limite d'influence de l'humidité supérieure prédéfinie, et
- l'influence de la pression ambiante (P) sur le résultat de détermination est compensée selon ladite condition limite,

dans lequel, dans le mode de compensation de l'humidité, l'influence de l'humidité ambiante (Hum) sur le résultat de détermination est compensée de telle sorte que

- la condition limite est respectée, selon laquelle l'influence de la pression ambiante (P) sur le résultat de détermination reste en dessous d'une limite d'influence de la pression supérieure prédéfinie, et
- l'influence de l'humidité ambiante (Hum) sur le résultat de détermination est compensée selon ladite condition limite, et

dans lequel l'unité d'évaluation (9) est configurée de sorte que, dans le mode de compensation de la pression, la dépendance de la concentration de gaz cible déterminée par rapport à la grandeur de détection de détecteur (U10) et/ou à la grandeur de détection de compensateur (U11) est différente de celle dans le mode de compensation de l'humidité.

2. Dispositif de mesure de gaz (100) selon la revendication 1,
   **caractérisé en ce que**

   le dispositif de mesure de gaz (100) peut en outre fonctionner dans un mode d'optimisation de la pression et/ou dans un mode d'optimisation de l'humidité,

   dans lequel, dans le mode d'optimisation de la pression, l'influence de la pression ambiante (P) sur le résultat de détermination de l'unité d'évaluation (9) est compensée sans respecter une condition limite,

   dans lequel, dans le mode d'optimisation de l'humidité, l'influence de l'humidité ambiante (Hum) sur le résultat de détermination est compensée sans respecter une condition limite et

   dans lequel l'unité d'évaluation (9) est configurée de sorte que, dans chaque mode, la dépendance de la concentration de gaz cible déterminée par rapport à la grandeur de détection de détecteur (U10) et/ou à la grandeur de détection de compensateur (U11) est différente de celle dans chaque autre mode.

3. Dispositif de mesure de gaz (100) selon l'une des revendications précédentes,
   **caractérisé en ce que**

   le dispositif de mesure de gaz (100) peut fonctionner au choix dans le mode de compensation de la pression ou dans le mode de compensation de l'humidité et

peut fonctionner éventuellement en outre, au choix, dans le mode d'optimisation de la pression ou dans le mode d'optimisation de l'humidité.

4. Dispositif de mesure de gaz (100) selon la revendication 3,
   **caractérisé en ce que**

le dispositif de mesure de gaz (100) comprend une unité de sélection (16),
dans lequel l'unité de sélection (16) est configurée pour détecter une consigne d'un utilisateur ou d'une commande supérieure,
dans lequel la consigne détectée spécifie un mode dans lequel le dispositif de mesure de gaz (100) doit fonctionner,
dans lequel le dispositif de mesure de gaz (100) est configuré pour fonctionner dans le mode de compensation de la pression ou dans le mode de compensation de l'humidité en fonction d'une action de l'unité de sélection (16), et
dans lequel le dispositif de mesure de gaz (100) est éventuellement en outre configuré pour fonctionner dans le mode d'optimisation de la pression ou dans le mode d'optimisation de l'humidité en fonction d'une action de l'unité de sélection (16).

5. Dispositif de mesure de gaz (100) selon la revendication 3 ou la revendication 4,
   **caractérisé en ce que**
   le dispositif de mesure de gaz (100) est configuré pour

- déterminer, dans au moins deux modes différents, respectivement une valeur estimative de la concentration de gaz cible dans le même échantillon de gaz (Gp) et
- générer une alarme lorsqu'au moins une valeur estimative déterminée se situe en dehors d'une plage de valeurs prédéfinie pour la concentration de gaz cible.

6. Dispositif de mesure de gaz (100) selon l'une des revendications précédentes,
   **caractérisé en ce que**

le dispositif de mesure de gaz (100) comprend en outre au moins un capteur (14, 17, 18) pour une condition ambiante,
dans lequel le ou chaque capteur (14, 17, 18) pour une condition ambiante est configuré pour mesurer la condition ambiante respective,
dans lequel, de préférence, le ou un capteur (14) pour une condition ambiante est un capteur de température configuré pour mesurer la température ambiante, et
dans lequel l'unité d'évaluation (9) est configurée pour déterminer la concentration du gaz cible ($CH_4$) dans l'échantillon de gaz (Gp) en outre en fonction du signal respectif du capteur ou d'au moins un capteur (14, 17, 18) pour une condition ambiante.

7. Dispositif de mesure de gaz (100) selon la revendication 6,
   **caractérisé en ce que**

le ou au moins un capteur pour une condition ambiante,
en particulier un capteur (17) pour l'humidité ambiante ou un capteur (18) pour la pression ambiante,
est activé ou désactivé au choix,
dans lequel le dispositif de mesure de gaz (100) est configuré de sorte que
l'unité d'évaluation (9), lorsque le capteur (17, 18) pour une condition ambiante est activé, détermine la concentration du gaz cible ($CH_4$) dans l'échantillon de gaz (Gp) en outre en fonction du signal du capteur (17, 18) activé et
le dispositif de mesure de gaz (100) peut fonctionner dans le mode de compensation de la pression et/ou dans le mode de compensation de l'humidité au moins lorsque le capteur (17, 18) pour une condition ambiante est désactivé.

8. Dispositif de mesure de gaz (100) selon l'une des revendications précédentes,
   **caractérisé en ce que**

le détecteur (10) comprend un segment de détecteur (20) pouvant être chauffé et
le compensateur (11) comprend un segment de compensateur (38) pouvant être chauffé,

dans lequel le dispositif de mesure de gaz (100) est configuré pour chauffer le segment de détecteur (20) de sorte que le segment de détecteur (20) chauffé oxyde le gaz cible ($CH_4$) combustible dans l'échantillon de gaz (Gp) et l'oxydation chauffe davantage le segment de détecteur (20),

dans lequel le dispositif de mesure de gaz (100) est configuré pour chauffer le segment de compensateur (38), dans lequel le dispositif de mesure de gaz (100)

- dans une première alternative, est configuré de sorte que le segment de compensateur (38) chauffé oxyde moins de gaz cible combustible par unité de temps que le segment de détecteur (20) chauffé et

dans une seconde alternative, est configuré de sorte que, par unité de temps, une quantité de l'échantillon de gaz atteignant le compensateur (11) est plus faible que celle atteignant le détecteur (10), et

dans lequel le capteur de grandeur de détection de détecteur (12.1) est configuré pour mesurer, en tant que grandeur de détection de détecteur, une mesure (U10) pour la température du segment de détecteur (20), et dans lequel le capteur de grandeur de détection de compensateur (12.2) est configuré pour mesurer, en tant que grandeur de détection de compensateur, une mesure (U11) pour la température du segment de compensateur (38).

9. Dispositif de mesure de gaz (100) selon l'une des revendications précédentes, **caractérisé en ce que**

l'unité d'évaluation (9) présente au moins temporairement un accès en lecture à un modèle (Mod) exploitable par ordinateur,

dans lequel le modèle (Mod) comprend respectivement une relation fonctionnelle pour le ou chaque mode dans lequel le dispositif de mesure de gaz (100) peut fonctionner,

dans lequel la relation fonctionnelle pour un mode décrit une relation entre

- la concentration de gaz cible d'une part et
- le signal respectif de chaque capteur de grandeur de détection (12.1, 12.2) et

éventuellement, au moins un signal d'un capteur (14, 17, 18) pour une condition ambiante, d'autre part
et

dans lequel l'unité d'évaluation (9) est configurée pour, lors de la détermination de la concentration de gaz cible, appliquer la relation fonctionnelle pour le mode dans lequel le dispositif de mesure de gaz (100) fonctionne actuellement,

au signal respectif de chaque capteur de grandeur de détection (12.1, 12.2) et, éventuellement, au signal respectif de chaque capteur (14, 17, 18) optionnel pour une condition ambiante.

10. Agencement comprenant

- un premier dispositif de mesure de gaz (100) selon l'une des revendications précédentes et
- un second dispositif de mesure de gaz (100.1) selon l'une des revendications précédentes,

dans lequel l'unité d'évaluation (9) du premier dispositif de mesure de gaz (100) a un accès en lecture, au moins temporairement, à un premier modèle (Mod) exploitable par ordinateur qui décrit une première dépendance de la concentration de gaz cible au moins par rapport à la grandeur de détection de détecteur (U10) et à la grandeur de détection de compensateur (U11) et éventuellement à au moins une condition ambiante pour un fonctionnement dans le mode de compensation de la pression, et

dans lequel l'unité d'évaluation (9.1) du second dispositif de mesure de gaz (100.1) a un accès en lecture, au moins temporairement, à un second modèle (Mod.1) exploitable par ordinateur qui décrit une seconde dépendance de la concentration de gaz cible au moins par rapport à la grandeur de détection de détecteur (U10) et à la grandeur de détection de compensateur (U11) et éventuellement à au moins une condition ambiante pour un fonctionnement dans le mode de compensation de l'humidité.

11. Dispositif d'étalonnage (110) pour l'étalonnage d'un dispositif de mesure de gaz (100) selon la revendication 9,

dans lequel le dispositif d'étalonnage (110) est configuré pour
détecter une consigne, dans lequel la consigne détectée spécifie au moins un mode dans lequel le dispositif de mesure de gaz (100) doit pouvoir fonctionner,
générer un modèle (Mod) exploitable par ordinateur de telle sorte que le modèle (Mod) généré

- comprend respectivement une relation fonctionnelle pour le ou chaque mode détecté et
- est applicable par l'unité d'évaluation (9) du dispositif de mesure de gaz (100),

dans lequel le dispositif d'étalonnage (110) est configuré pour utiliser un échantillon prédéfini et un ensemble de relations fonctionnelles possibles prédéfinies pour la génération du modèle,
dans lequel l'échantillon comprend plusieurs éléments d'échantillon,
dans lequel chaque élément d'échantillon de l'échantillon comprend

- une identification d'une combinaison de gaz cible et de conditions ambiantes,

à savoir une combinaison d'une température ambiante, d'une pression ambiante, d'une humidité ambiante et d'une concentration de gaz cible réelle, et

- une combinaison de valeurs de signal

qui présente, pour le signal respectif de chaque capteur de grandeur de détection (12.1, 12.2), respectivement une valeur mesurée pour ladite combinaison de gaz cible et de conditions ambiantes,
dans lequel le dispositif d'étalonnage (110) est configuré pour exécuter, pour chaque mode, pour chaque relation fonctionnelle possible prédéfinie et pour chaque élément d'échantillon, respectivement les étapes consistant à

- appliquer la relation fonctionnelle à la combinaison de valeurs de signal de l'élément d'échantillon et calculer ainsi une valeur pour la concentration de gaz cible ; et
- comparer la valeur calculée pour la concentration de gaz cible avec la valeur réelle de la concentration de gaz cible dans ledit élément d'échantillon, et

dans lequel le dispositif d'étalonnage (110) est configuré pour, pour chaque mode,

- sélectionner une relation fonctionnelle possible en utilisant les résultats de comparaison et
- amener la relation fonctionnelle sélectionnée à être utilisée par l'unité d'évaluation (9) en tant que relation fonctionnelle à appliquer dans ce mode.

12. Procédé de mesure de gaz pour la mesure de la concentration d'un gaz cible ($CH_4$) combustible dans une zone spatiale (B)

en utilisant un dispositif de mesure de gaz (100) qui comprend

- un détecteur (10),
- un compensateur (11),
- un capteur de grandeur de détection de détecteur (12.1) et
- un capteur de grandeur de détection de compensateur (12.2),

dans lequel le détecteur (10) présente une grandeur de détection de détecteur (U10) qui est corrélée à la concentration du gaz cible ($CH_4$) dans un échantillon de gaz (Gp),
dans lequel le compensateur (11) présente une grandeur de détection de compensateur (U11) qui est moins corrélée à la concentration de gaz cible que la grandeur de détection de détecteur (U10), ou même pas du tout corrélée,
dans lequel la grandeur de détection de détecteur (U10) et la grandeur de détection de compensateur (U11) sont influencées ou peuvent au moins être influencées par au moins une condition ambiante agissant sur l'échantillon de gaz (Gp),
dans lequel le dispositif de mesure de gaz comprend les étapes selon lesquelles

- un échantillon de gaz (Gp) provenant de la zone est amené à atteindre au moins temporairement le détecteur (10) et le compensateur (11),
- le capteur de grandeur de détection de détecteur (12.1) mesure la grandeur de détection de détecteur (U10),

- le capteur de grandeur de détection de compensateur (12.2) mesure la grandeur de détection de compensateur (U11) et
- en fonction de la grandeur de détection de détecteur (U10) mesurée et de la grandeur de détection de compensateur (U11) mesurée, la concentration du gaz cible ($CH_4$) dans l'échantillon de gaz (Gp) est déterminée,

**caractérisé en ce que**
le dispositif de mesure de gaz (100) fonctionne dans un mode de compensation de la pression et/ou dans un mode de compensation de l'humidité lors de l'exécution du procédé de mesure de gaz,
dans lequel, dans le mode de compensation de la pression, l'influence de la pression ambiante (P) sur un résultat de détermination est compensée de telle sorte que

- la condition limite est respectée, selon laquelle l'influence de l'humidité ambiante (Hum) sur le résultat de détermination reste en dessous d'une limite d'influence de l'humidité supérieure prédéfinie, et
- l'influence de la pression ambiante (P) sur le résultat de détermination est compensée selon ladite condition limite, et

dans lequel, dans le mode de compensation de l'humidité, l'influence de l'humidité ambiante (Hum) sur le résultat de détermination est compensée de telle sorte que

- la condition limite est respectée, selon laquelle l'influence de la pression ambiante (P) sur le résultat de détermination reste en dessous d'une limite d'influence de la pression supérieure prédéfinie, et
- l'influence de l'humidité ambiante (Hum) sur le résultat de détermination est compensée selon ladite condition limite, et

dans lequel, dans le mode de compensation de la pression, la dépendance de la concentration de gaz cible déterminée par rapport à la grandeur de détection de détecteur (U10) et/ou à la grandeur de détection de compensateur (U11) est différente de celle dans le mode de compensation de l'humidité.

13. Procédé d'étalonnage pour l'étalonnage d'un dispositif de mesure de gaz (100) selon la revendication 9,

dans lequel un échantillon et un ensemble de relations fonctionnelles possibles prédéfinies sont donnés,
dans lequel l'échantillon comprend plusieurs éléments d'échantillon,
dans lequel chaque élément d'échantillon de l'échantillon comprend

- une identification d'une combinaison de gaz cible et de conditions ambiantes,

à savoir une combinaison d'une température ambiante, d'une pression ambiante, d'une humidité ambiante et d'une concentration de gaz cible réelle, et

- une combinaison de valeurs de signal

à savoir, pour le signal respectif de chaque capteur de grandeur de détection (12.1, 12.2), respectivement une valeur mesurée pour ladite combinaison de gaz cible et de conditions ambiantes,
éventuellement en outre au moins une valeur d'un capteur (14, 17, 18) pour une condition ambiante, dans lequel le capteur (14, 17, 18) pour une condition ambiante a mesuré ladite valeur pour ladite combinaison de gaz cible et de conditions ambiantes,
dans lequel le procédé d'étalonnage comprend les étapes selon lesquelles

- une consigne est détectée,

dans lequel la consigne détectée spécifie au moins un mode dans lequel le dispositif de mesure de gaz (100) doit pouvoir fonctionner, et

- un modèle (Mod) exploitable par ordinateur est généré de telle sorte

que le modèle (Mod) généré pour le ou chaque mode détecté comprend respectivement une relation fonctionnelle et peut être appliqué par l'unité d'évaluation (9) du dispositif de mesure de gaz (100), et

dans lequel le procédé d'étalonnage comprend les étapes selon lesquelles

- pour chaque mode et pour chaque élément d'échantillon, respectivement chaque relation fonctionnelle possible prédéfinie est appliquée à la combinaison de valeurs de signal de l'élément d'échantillon,

une valeur pour la concentration de gaz cible est ainsi calculée et
la valeur calculée pour la concentration de gaz cible est comparée avec la valeur réelle de la concentration de gaz cible dans ledit élément d'échantillon,

- une relation fonctionnelle possible est sélectionnée pour chaque mode en utilisant les résultats de comparaison, et

la relation fonctionnelle sélectionnée est amenée à être utilisée par l'unité d'évaluation (9) en tant que relation fonctionnelle à appliquer dans ce mode.

FIG. 1

EP 4 560 302 B1

FIG. 2

$O_2$ $CH_4$ $CO_2$ $H_2O$

10  20  24  25  26  27

**FIG. 3**

EP 4 560 302 B1

FIG. 4

EP 4 560 302 B1

**FIG. 5**

FIG. 6

EP 4 560 302 B1

FIG. 7

FIG. 8

EP 4 560 302 B1

FIG. 9

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102022102969 A1